(19)

Europäisches
Patentamt
European
Patent Office
Office européen
des brevets

(11) **EP 3 995 577 A1**

(12) **EUROPEAN PATENT APPLICATION**

(43) Date of publication:
**11.05.2022   Bulletin 2022/19**

(21) Application number: **20206417.6**

(22) Date of filing: **09.11.2020**

(51) International Patent Classification (IPC):
**C12N 15/11** (2006.01)        **C12N 15/63** (2006.01)

(52) Cooperative Patent Classification (CPC):
**C12N 15/111; C12N 15/63; G16B 5/10;**
C12N 2310/141; C12N 2830/001; C12N 2830/002;
C12N 2830/005

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ETH Zurich
8092 Zurich (CH)**

(72) Inventors:
• **Frei, Timothy Thomas
  4058 Basel (CH)**

• **Chang, Ching-Hsiang
  4058 Basel (CH)**
• **Filo, Maurice
  4057 Basel (CH)**
• **Khammash, Mustafa
  8006 Zürich (CH)**

(74) Representative: **Schulz Junghans
Patentanwälte PartGmbB
Großbeerenstraße 71
10963 Berlin (DE)**

(54) **EXPRESSION SYSTEM AND METHOD FOR CONTROLLING A NETWORK IN A CELL AND CELL COMPRISING THE EXPRESSION SYSTEM**

(57)     The invention relates to an expression system for controlling a network in a cell, wherein the network comprises an actuator molecule and an output molecule, wherein the output molecule is positively or negatively regulated by the actuator molecule, wherein the expression system comprises a recombinant gene encoding a first controller molecule, wherein the first controller molecule positively or negatively regulates the actuator molecule.

The invention further relates to a cell comprising the expression system, to a cell for use as a medicament and to a method for controlling a network in a cell.

Fig. 23A

**Description**

The present invention relates to an expression system, and a method for controlling a regulatory network in a cell and a cell comprising the expression system as well as medical uses of the cell and the expression system.

The ability to maintain a steady internal environment in the presence of a changing and uncertain exterior world - called homeostasis - is a defining characteristic of living systems. Homeostasis is maintained by various regulatory mechanisms, often in the form of negative feedback loops. The concept of homeostasis is particularly relevant in physiology and medicine, where loss of homeostasis is often attributed to the development of a disease. In this regard, deepening the understanding of the molecular mechanisms that govern homeostasis will guide the development of treatments for such diseases.

In engineering, the ability of a system to maintain another system in a desired state when faced with perturbations to this state has been realized using various control mechanisms as well as their combinations, giving rise to integral, proportional integral, proportional derivative, and proportional integral derivative controllers, which are frequently used, e.g., in electronics.

In recent years, artificial genetic circuits have been introduced in the field of synthetic biology. These systems can be used to manipulate and artificially control networks, such as gene regulatory networks, in biological cells. Essentially, recombinant genes encoding cellular regulators are introduced into these cells using the tools of molecular biology. Such artificial genetic circuits offer promising new therapies for many kinds of diseases associated with the disregulation of cellular networks.

However, many of the known artificial genetic circuits according to the prior art lack robustness towards fluctuations of their environment, especially when very tight regulation of the desired setpoint is required.

In view of these disadvantages of the known artificial genetic circuits, the objective of the present invention is to provide means and methods for controlling a network in a cell in a robust and tightly-controlled manner. This objective is attained by the subject-matter of the independent claims of the present specification, with further advantageous embodiments described in the dependent claims, examples, figures and general description of this specification.

A first aspect of the invention relates to a recombinant expression system for controlling a network in a cell, wherein the network comprises an actuator molecule, particularly an actuator protein, and an output molecule, particularly an output protein, wherein the output molecule is positively or negatively regulated by the actuator molecule, and wherein the expression system comprises nucleic acids comprising a recombinant gene encoding a first controller molecule, wherein the first controller molecule positively or negatively regulates the actuator molecule.

In particular, the expression system comprises or consists of one or several nucleic acids carrying at least one recombinant gene capable of being expressed in the cell. Therein, expression particularly relates to transcription of the at least one recombinant gene into RNA, particularly messenger RNA (mRNA), and optionally subsequent translation of mRNA into a protein in the cell.

The cell may be a prokaryotic (particularly bacterial) or a eukaryotic (particularly fungus, plant or animal, more particularly mammalian) cell. Any suitable expression system known in the art may be used for a cell of interest. For example, the expression system may comprise one or several DNA vectors, such as plasmids, viruses or artificial chromosomes, known in the art of molecular biology.

As used herein, the term "network" describes at least two biological entities (e.g., genes or proteins) which are functionally linked in that one biological entity directly or indirectly influences the concentration and/or biological activity of any of the other entities of the network. For example, such networks may comprise at least one gene encoding a transcriptional regulator protein, which activates or represses the transcription of at least one other gene in the network. Furthermore, biological entities in the network could be proteins interacting with each other, wherein one protein of the network activates or inhibits a biological activity (e.g. an enzymatic activity) of another protein in the network.

In the network of the cell according to the present invention, an actuator molecule (e.g. a protein) directly or indirectly (i.e., via interactions with one or several further genes or proteins) regulates an output molecule (e.g., a protein or a small molecule, e.g. a metabolite) positively or negatively.

Therein, the term "regulate" means that the actuator directly or indirectly affects the concentration of the output molecule in the cell or its biological activity (e.g. enzymatic activity or binding to a target molecule) in the cell.

Such regulation may occur by several mechanisms. For example, in case the output molecule is a protein, regulation by the actuator molecule may occur by direct or indirect activation or repression of transcription of a gene encoding the output molecule, directly or indirectly mediating or inhibiting the degradation of mRNA encoding the output molecule, direct or indirect activation or inhibition of translation of the output molecule from mRNA, directly or indirectly mediating or inhibiting the degradation, post-translational modification, complex formation, secretion from the cell or intracellular transport of the output molecule, or activating or inhibiting the biological activity of the output molecule. Likewise, in case of the output molecule being a small molecule, positive or negative regulation may e.g. entail directly or indirectly affecting synthesis, degradation, transport or modification of the small molecule.

According to the present invention, the expression system is used to introduce nucleic acids encoding a recombinant molecular controller (at least the first controller molecule, and optionally also a feedback molecule, a first anti-controller molecule, a second controller molecule, and a second anti-controller molecule, see below) into the cell of interest to control the output molecule (controlled species) of the network by manipulating the actuator molecule (process input). In particular, the aim of this control is to achieve a desired setpoint, i.e. a desired concentration and/or activity of the output molecule in spite of fluctuations and external perturbations of the network equilibrium.

In certain embodiments, the expression system further comprises nucleic acids comprising a recombinant gene encoding a feedback molecule, wherein the feedback molecule is positively regulated by the output molecule, and wherein in case the actuator molecule positively regulates the output molecule, the feedback molecule negatively regulates the actuator molecule, and in case the actuator molecule negatively regulates the output molecule, the feedback molecule positively regulates the actuator molecule.

The case where the actuator molecule positively regulates the output molecule is also referred to herein as a "positive gain process", and the case where the actuator molecule negatively regulates the output molecule is also referred to herein as a "negative gain process".

Advantageously, the feedback molecule artificially introduces molecular feedback into the network and thereby improves the stability of the concentration and/or activity of the output molecule against perturbations to the network. In terms of control theory, the feedback molecule introduces proportional control to the network, in other words, the correction applied to the controlled species (output molecule) is proportional to the measured value.

As an alternative or in addition to introducing the feedback molecule into the cell to achieve artificial feedback regulation of the network, a naturally occurring (i.e., non-recombinant) feedback of the network may also be utilized to achieve stability of regulation. That is, if the network itself is naturally feedback-regulated, it is possible, e.g., to implement a proportional integral controller just by introducing a first controller molecule and a first anti-controller molecule (antithetic motif resulting in integral control, see below), but without introducing a recombinant feedback molecule. In this case, e.g., proportional control would be achieved by the naturally occurring (i.e., non-recombinant) feedback mechanism.

In certain embodiments, in case the actuator molecule positively regulates the output molecule (in other words in case of a positive gain process), the feedback molecule is a microRNA which negatively regulates production of the actuator molecule, particularly by inhibiting translation of an mRNA encoding the actuator molecule and/or promoting degradation of an mRNA encoding the actuator molecule.

In certain embodiments, in case the actuator molecule positively regulates the output molecule (in other words in case of a positive gain process), the feedback molecule is an RNA binding protein which negatively regulates production of the actuator molecule, particularly by binding to an untranslated region of an mRNA encoding the actuator molecule and inhibiting translation of the mRNA.

In certain embodiments, in case the actuator molecule negatively regulates the output molecule (in other words in case of a negative gain process), the feedback molecule is an additional mRNA encoding the actuator molecule. Therein the term "additional mRNA" means the transcript of an additional recombinant gene introduced into the cell in addition to the transcript of a naturally occurring (i.e., non-recombinant) gene encoding the actuator molecule.

In certain embodiments, the first controller molecule positively regulates the actuator molecule, wherein the expression system further comprises nucleic acids comprising a recombinant gene encoding a first anti-controller molecule, wherein the first anti-controller molecule negatively regulates the first controller molecule, and wherein the first controller molecule negatively regulates the first anti-controller molecule. In particular, the first anti-controller molecule inactivates, sequesters and/or annihilates the first controller molecule, and the first controller molecule inactivates, sequesters and/or annihilates the first anti-controller molecule.

In case the actuator molecule positively regulates the output molecule (in other words in case of a positive gain process), the first anti-controller molecule is positively regulated by the output molecule. Alternatively, in case the actuator molecule negatively regulates the output molecule (in other words in case of a negative gain process), the first controller molecule is positively regulated by the output molecule. In this manner, a closed control loop between the actuator molecule and the output molecule is formed via the first controller molecule and the first anti-controller molecule.

This type of control, which may also be designated "antithetic motif" herein, implements integral control of the network, in other words correction applied to the controlled species (output molecule) depends on an integral over the difference between the setpoint and the measured value. In this implementation, in particular, the setpoint may be controlled by controlling a ratio between the production rate of the controller molecule and the production rate of the anti-controller molecule in the cell.

In certain embodiments, the first anti-controller molecule inactivates, particularly completely inactivates, the first controller molecule, and the first controller molecule inactivates, particularly completely inactivates, the first anti-controller molecule. In particular, the inactivation reaction between the first controller molecule and the first anti-controller molecule is stoichiometrically fixed, in other words a given number of first anti-controller molecules inactivates a fixed number of first controller molecules and/or a given number of first controller molecules inactivates a fixed number of first anti-controller molecules. Therein, "stoichiometrically fixed" means that the ratio of numbers of first controller molecules and first anti-controller molecules does not change in time.

In the context of the present specification, a first molecule "inactivating" a second molecule means that the first molecule abolishes a biological function of the second molecule. Such a biological function may be, e.g., binding of a transcriptional regulator to a target DNA, binding of a translational regulator to a target mRNA, binding of a protein to a target molecule or an enzymatic activity of an enzyme.

In certain embodiments, the first anti-controller molecule and the first controller molecule physically interact, particularly bind to each other (e.g., in case of proteins) or hybridize (e.g., in case of nucleic acids) to negatively regulate, particularly inactivate, each other.

In certain embodiments, the first anti-controller molecule and the first controller molecule physically interact to inactivate each other, wherein the first anti-controller molecule abolishes a biological function of the first controller molecule, particularly a binding activity of the first controller molecule to a target molecule (e.g., target DNA, RNA or protein), wherein the first controller molecule sequesters the first anti-controller molecule.

In the context of the present specification, the term "sequester" describes binding of a first molecule to a second molecule, such that physical interactions of the second molecules with further molecules are abolished (e.g., a single first controller molecule binds to a single first anti-controller molecule to abolish binding of the first anti-controller molecule to other first controller molecules).

In certain embodiments, the first anti-controller molecule and the first controller molecule annihilate each other to negatively regulate, particularly inactivate, each other.

In the context of the present specification, the term "annihilate" describes an interaction between a first molecule and a second molecule which leads to degradation of the first molecule and the second molecule.

In certain embodiments, the first controller molecule comprises or is a sense mRNA encoding the actuator molecule or a sense mRNA coding for an activator, e.g., a transcriptional activator of a gene encoding the actuator molecule, which positively regulates the actuator molecule, and wherein the first anti-controller molecule comprises or is an anti-sense RNA comprising a sequence which is complementary to a sequence of the sense mRNA. The sense mRNA and the anti-sense RNA hybridize which results in an inhibition of translation of the sense mRNA (leading to inactivation). At the same time, the hybridization prevents the antisense RNA from interacting with other sense mRNA molecules (i.e., sequestration).

In certain embodiments, the first controller molecule is an activator protein which positively regulates production of the actuator molecule, e.g., by activating transcription of a gene encoding the actuator molecule, activating translation of an mRNA encoding the actuator molecule or inhibiting degradation of an mRNA encoding the actuator molecule or inhibiting degradation of the actuator molecule or by negatively regulating an inhibitor of the function of the actuator molecule, and wherein the first anti-controller molecule is an anti-activator protein, wherein the activator protein and the anti-activator protein form a protein-protein complex, wherein the positive regulation of the actuator molecule by the activator protein is inhibited by formation of the complex (resulting in inactivation). At the same time, the complex formation prevents the anti-activator protein from interacting with other activator protein molecules (i.e., sequestration).

In particular, this antithetic motif may be combined with the feedback mechanism of the feedback molecule to achieve a molecular proportional integral controller (PI controller).

In certain embodiments, to provide a molecular PI controller, the expression system comprises nucleic acids comprising at least one recombinant gene encoding a first controller molecule, a first anti-controller molecule and, particularly a feedback molecule, wherein in case the actuator molecule positively regulates the output molecule, i.e., in case of a positive gain process (N-type PI controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first anti-controller molecule (resulting in integral control), and
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control).

Alternatively, in case the actuator molecule negatively regulates the output molecule, i.e., in case of a negative gain process (P-type PI controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control), and
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control).

In certain embodiments, the actuator molecule positively regulates the output molecule (in other words, the network between the actuator molecule and the output molecule represents a positive gain process), wherein the first controller molecule is positively regulated by the output molecule.

In certain embodiments, the actuator molecule negatively regulates the output molecule (in other words, the network between the actuator molecule and the output molecule represents a negative gain process), wherein the first anti-controller molecule is positively regulated by the output molecule.

By this additional link between the output molecule and the first controller or anti-controller molecule, derivative control can be implemented in addition to proportional integral control by the antithetic motif. Derivative control as used herein, is a control mechanism, in which correction applied to the controlled species (output molecule) depends on a derivative of the measured value (output). In combination with a feedback loop to implement proportional control, this can be used to implement a molecular second-order proportional-integral-derivative (PID) controller (second order due to the presence of two controller species, the first controller molecule and the first anti-controller-molecule).

In certain embodiments, to implement a second order PID controller, the expression system comprises nucleic acids comprising at least one recombinant gene encoding a first controller molecule, a second controller molecule and particularly a feedback molecule, wherein in case the actuator molecule positively regulates the output molecule, i.e., in case of a positive gain process (N-type second order PID controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first anti-controller molecule (resulting in integral control),
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the output molecule positively regulates the first controller molecule (this component combined with the Proportional component results in a filtered PD control).

Alternatively, in case the actuator molecule negatively regulates the output molecule, i.e., in case of a negative gain process (P-type second order PID controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the output molecule positively regulates the first anti-controller molecule (this component combined with the Proportional component results in a filtered PD control).

In certain embodiments, the expression system further comprises nucleic acids comprising a recombinant gene encoding a second controller molecule.

In certain embodiments, in case the actuator molecule positively regulates the output molecule (positive gain process), the second controller molecule is positively or negatively regulated by the output molecule and the second controller-molecule negatively regulates the actuator molecule.

In certain embodiments, in case the actuator molecule positively regulates the output molecule (positive gain process), the second controller molecule is negatively regulated by the output molecule and the second controller-molecule positively or negatively regulates the actuator molecule.

In certain embodiments, in case the actuator molecule negatively regulates the output molecule (negative gain process), the second controller molecule is positively or negatively regulated by the output molecule and the second controller-molecule positively regulates the actuator molecule.

In certain embodiments, in case the actuator molecule negatively regulates the output molecule (negative gain process), the second controller molecule is positively regulated by the output molecule and the second controller-molecule positively or negatively regulates the actuator molecule.

By the additional second controller molecule, derivative control is implemented in the network. In combination with integral control (e.g., via an antithetic motif) and proportional control (e.g., using an artificial feedback loop), a molecular third-order proportional-integral-derivative (PID) controller may be implemented. This controller is a third-order controller due to the three involved species: first controller molecule, first anti-controller molecule, second controller molecule.

In certain embodiments, to implement a molecular third-order PID controller, the expression system comprises nucleic acids comprising at least one recombinant gene encoding a first controller molecule, a first anti-controller molecule, a second controller molecule and particularly a feedback molecule, wherein in case the actuator molecule positively regulates the output molecule, i.e., in case of a positive gain process (N-type third-order PID controller):

-   the first controller molecule positively regulates the actuator molecule, the first anti-controller molecule negatively regulates the first controller molecule, the first controller molecule negatively regulates the first anti-controller molecule (antithetic motif), and the output molecule positively regulates the anti-controller molecule (resulting in integral control),
-   the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
-   the second controller molecule is positively or negatively regulated by the output molecule, and the second controller molecule negatively regulates the actuator molecule (together with the proportional controller this results in a filtered PD controller), or the second controller molecule is negatively regulated by the output molecule and the second controller molecule positively or negatively regulates the actuator molecule (together with the proportional controller this results in a filtered PD controller). In both cases, when the regulation of the second controller molecule and the regulation of the actuator molecule have opposite signs (one positive, the other negative), the filtered PD controller approximates a pure PD controller. When the signs are the same, the filtered PD controller approximates a so-called LAG controller.

Alternatively, in case the actuator molecule negatively regulates the output molecule, i.e., in case of a negative gain process (P-type third-order PID controller),

-   the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
-   the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
-   the second controller molecule is positively or negatively regulated by the output molecule, and the second controller molecule positively regulates the actuator molecule (together with the proportional controller this results in a filtered PD controller), or the second controller molecule is positively regulated by the output molecule and the second controller molecule positively or negatively regulates the actuator molecule (together with the proportional controller this results in a filtered PD controller). In both cases, when the regulation of the second controller molecule and the regulation of the actuator molecule have opposite signs (one positive, the other negative), the filtered PD controller approximates a pure PD controller. When the signs are the same, the filtered PD controller approximates a so-called LAG controller.

In certain embodiments, the expression system further comprises nucleic acids comprising at least one recombinant gene encoding a second anti-controller molecule, wherein the second anti- controller molecule negatively regulates, particularly inactivates, sequesters and/or annihilates, the second controller molecule, and wherein the second controller molecule negatively regulates, particularly inactivates, sequesters and/or annihilates, the second anti-controller molecule, wherein in case the actuator molecule positively regulates the output molecule, i.e., in case of a positive gain process, the second controller molecule is negatively regulated by the output molecule, and in case the actuator molecule negatively regulates the output molecule, i.e., in case of a negative gain process, the second controller molecule is positively regulated by the output molecule.

According to this embodiment, the second controller molecule and the second anti-controller molecule form a second antithetic motif which, in particular, can be used to implement a molecular fourth order proportional-integral-derivative (PID) controller to control the network in the cell.

In certain embodiments, the second controller molecule negatively regulates itself.

In certain embodiments, to implement a fourth order PID controller, the expression system comprises nucleic acids comprising at least one recombinant gene encoding a first controller molecule, a first anti-controller molecule, a second controller molecule, a second anti-controller molecule and a feedback molecule, wherein in case the actuator molecule positively regulates the output molecule, i.e., in case of a positive gain process (N-type fourth order PID controller),

- the first controller molecule positively regulates the actuator molecule, the first anti-controller molecule negatively regulates the first controller molecule, the first controller molecule negatively regulates the first anti-controller molecule (first antithetic motif), the output molecule positively regulates the first anti-controller molecule, (resulting in integral control),
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the second controller molecule negatively regulates the actuator molecule, the second anti-controller molecule negatively regulates the second controller molecule, the second controller molecule negatively regulates the second anti-controller molecule (second antithetic motif), the output molecule negatively regulates the second controller molecule, and the second controller molecule negatively regulates itself (resulting in derivative control).

Alternatively, in case the actuator molecule negatively regulates the output molecule, i.e., in case of a negative gain process (P-type fourth order PID controller),

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the second controller molecule positively regulates the actuator molecule, the second anti-controller molecule negatively regulates the second controller molecule, the second controller molecule negatively regulates the second anti-controller molecule (second antithetic motif), the output molecule positively regulates the second controller molecule, and the second controller molecule negatively regulates itself (resulting in derivative control).

In certain embodiments (particularly in case of any one of the above-described N-type or P-type PI controllers, N-type or P-type second order, third order or fourth order PID controllers), the first anti-controller molecule inactivates, particularly completely inactivates, the first controller molecule, and the first controller molecule inactivates, particularly completely inactivates, the first anti-controller molecule. In particular, the inactivation reaction between the first controller molecule and the first anti-controller molecule is stoichiometrically fixed.

In certain embodiments (particularly in case of any one of the above-described N-type or P-type PI controllers, N-type or P-type second order, third order or fourth order PID controllers), the first anti-controller molecule and the first controller molecule physically interact, particularly bind to each other (e.g., in case of proteins) or hybridize (e.g., in case of nucleic acids) to negatively regulate, particularly inactivate, each other.

In certain embodiments, (particularly in case of any one of the above-described N-type or P-type PI controllers, N-type or P-type second order, third order or fourth order PID controllers) the first anti-controller molecule and the first controller molecule physically interact to inactivate each other, wherein the first anti-controller molecule abolishes a biological function of the first controller molecule, particularly a binding activity of the first controller molecule to a target molecule (e.g., target DNA, RNA or protein), wherein the first controller molecule sequesters the first anti-controller molecule.

In certain embodiments (particularly in case of any one of the above-described N-type or P-type PI controllers, N-type or P-type second order, third order or fourth order PID controllers), the first anti-controller molecule and the first controller molecule annihilate each other to negatively regulate, particularly inactivate, each other.

In certain embodiments, the second controller molecule is a sense mRNA encoding a regulator protein, particularly a transcriptional activator or transcriptional repressor, which regulates expression of the actuator molecule, wherein the second anti-controller molecule is an antisense RNA comprising a complementary sequence to a sequence of the sense mRNA encoding the regulator protein, wherein particularly in case the feedback molecule is an additional mRNA encoding the actuator molecule (e.g., for a P-type controller in case of negative gain process), the sense mRNA may encode a regulator protein which negatively regulates the expression of the additional mRNA encoding the actuator molecule.

In certain embodiments, the second controller molecule is an RNA binding protein binding to an untranslated region of an mRNA encoding the actuator molecule, thereby negatively or positively regulating the actuator molecule, e.g., by inhibiting or activating translation or promoting or inhibiting degradation of the mRNA, and wherein the second anti-controller molecule is an anti-RNA-binding protein, wherein the RNA binding protein and the anti-RNA-binding protein form a complex, wherein the negative or positive regulation of the actuator molecule by the RNA binding protein is inhibited by formation of the complex.

In certain embodiments, in case the actuator molecule positively regulates the output molecule (positive gain process), the first controller molecule is positively or negatively regulated by the output molecule, and the first controller molecule negatively regulates the actuator molecule.

In certain embodiments, in case the actuator molecule positively regulates the output molecule (positive gain process), the first controller molecule is negatively regulated by the output molecule and the first controller molecule positively or negatively regulates the actuator molecule.

In certain embodiments, in case the actuator molecule negatively regulates the output molecule (negative gain process), the first controller molecule is positively or negatively regulated by the output molecule, and the first controller molecule positively regulates the actuator molecule.

In certain embodiments, in case the actuator molecule negatively regulates the output molecule (negative gain process), the first controller molecule is positively regulated by the output molecule and the first controller molecule positively or negatively regulates the actuator molecule.

In this manner, a molecular derivative controller may be implemented using only one controller species (the first controller molecule). Whether the output molecule positively or negatively regulates the first controller molecule is determined by the parameters of the network. In particular, this type of derivative control may be combined with proportional control by an artificial feedback loop to implement a molecular PD controller.

In certain embodiments, to implement a proportional-derivative (PD) controller, the expression system comprises nucleic acids comprising at least one recombinant gene encoding a first controller molecule and particularly a feedback molecule, wherein in case the actuator molecule positively regulates the output molecule, i.e., in case of a positive gain process (N-type PD controller),

- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the first controller molecule is positively or negatively regulated by the output molecule and the first controller molecule negatively regulates the actuator molecule (together with the proportional controller this results in a filtered PD controller), or the first controller molecule is negatively regulated by the output molecule and the first controller molecule positively or negatively regulates the actuator molecule (together with the proportional controller this results in a filtered PD controller). In both cases, when the regulation of the first controller molecule and the regulation of the actuator molecule have opposite signs (one positive, the other negative), the filtered PD controller approximates a pure PD controller. When the signs are the same, the filtered PD controller approximates a so-called LAG controller.

Alternatively, in case the actuator molecule negatively regulates the output molecule, i.e., in case of a negative gain process (P-type PD controller),

- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the first controller molecule is positively or negatively regulated by the output molecule and the first controller molecule positively regulates the actuator molecule (together with the proportional controller this results in a filtered PD controller), or the first controller molecule is positively regulated by the output molecule and the first controller molecule positively or negatively regulates the actuator molecule (together with the proportional controller this results in a filtered PD controller). In both cases, when the regulation of the first controller molecule and the regulation of the actuator molecule have opposite signs (one positive, the other negative), the filtered PD controller approximates a pure PD controller. When the signs are the same, the filtered PD controller approximates a so-called LAG controller.

A second aspect of the invention relates to a cell comprising the expression system according to the first aspect of the invention.

In certain embodiments, the cell is a mammalian cell, particularly a human cell.

In certain embodiments, the cell is a T cell, particularly expressing a chimeric antigen receptor (CAR).

CAR T-cells are frequently used in cancer therapy, wherein the engineered chimeric antigen receptor interacts with an antigen expressed by cancer cells of interest, which are then specifically targeted by the CAR T-cells.

In certain embodiments, a concentration of the output molecule in the cell is indicative of a concentration of at least one inflammatory cytokine in the cell, wherein the actuator molecule positively regulates production or release of at least one immunosuppressive agent in the cell. During CAR-T-cell therapy, a condition termed Cytokine Release Syndrome (CRS) frequently occurs. CRS is a form of systemic inflammatory response syndrome which can be life-threatening due to hyper-inflammation, hypotensive shock, and multi-organ failure. During CRS, positive feedback activates T-cells and other immune cells leading to a cytokine storm.

In particular, the expression system and the cell according to the invention may be used to counteract CRS during CAR T-cell therapy by controlling and stabilizing a network which is responsible for the immune reaction during CRS:

To this end, in particular, a molecule, the presence or concentration or activity of which is indicative of a concentration of at least one inflammatory cytokine in the cell can be chosen as an output molecule, the output is sensed by the controller molecules according to the invention. Furthermore, a molecule which is part of the same network as the output molecule, and which positively regulates production or release of at least one immunosuppressive agent in the cell, can be chosen as an actuator molecule to stabilize the immune response and alleviate CRS. For instance, the actuator molecule may function as an antagonist of IL-6 or an antagonist of the IL-1 receptor which have been shown to be effective against CRS.

By means of the control mechanism according to the invention, a desired setpoint of this antagonistic function may be achieved to avoid both a too small immunosuppressive effect which would be ineffective for immunosuppression and a too large immunosuppressive effect which would inhibit anti-tumor response efficacy. In addition, adaptation to patient-specific dosage can be achieved using the control mechanism according to the invention.

A third aspect of the invention relates to a cell comprising a network, wherein the network comprises an actuator molecule and an output molecule, wherein the output molecule is positively or negatively regulated by the actuator molecule, and wherein the cell expresses a recombinant gene encoding a first controller molecule, wherein the first controller molecule positively or negatively regulates the actuator molecule.

In certain embodiments, the cell is a prokaryotic (particularly bacterial) or a eukaryotic (particularly fungus, plant or animal, more particularly mammalian) cell.

In certain embodiments, the cell expresses a recombinant gene encoding a feedback molecule, wherein the feedback molecule is positively regulated by the output molecule, and wherein in case the actuator molecule positively regulates the output molecule, the feedback molecule negatively regulates the actuator molecule, and in case the actuator molecule negatively regulates the output molecule, the feedback molecule positively regulates the actuator molecule.

In certain embodiments, in case the actuator molecule positively regulates the output molecule (in other words in case of a positive gain process), the feedback molecule is a microRNA which negatively regulates production of the actuator molecule, particularly by inhibiting translation of an mRNA encoding the actuator molecule or promoting degradation of an mRNA encoding the actuator molecule.

In certain embodiments, in case the actuator molecule positively regulates the output molecule (in other words in case of a positive gain process), the feedback molecule is an RNA binding protein which negatively regulates production of the actuator molecule, particularly by binding to an untranslated region of an mRNA encoding the actuator molecule and inhibiting translation of the mRNA.

In certain embodiments, in case the actuator molecule negatively regulates the output molecule (in other words in case of a negative gain process), the feedback molecule is an additional mRNA encoding the actuator molecule. Therein the term "additional mRNA" means the transcript of an additional recombinant gene introduced into the cell in addition to the transcript of a naturally occurring (i.e., non-recombinant) gene encoding the actuator molecule.

In certain embodiments, the first controller molecule positively regulates the actuator molecule, wherein the cell expresses a recombinant gene encoding a first anti-controller molecule, wherein the first anti-controller molecule negatively regulates the first controller molecule, and wherein the first controller molecule negatively regulates the first anti-controller molecule. In particular, the first anti-controller molecule inactivates, sequesters and/or annihilates the first controller molecule, and the first controller molecule inactivates, sequesters and/or annihilates the first anti-controller molecule. In particular, the first anti-controller molecule inactivates, sequesters and/or annihilates the first controller molecule, and the first controller molecule inactivates, sequesters and/or annihilates the first anti-controller molecule. In case the actuator molecule positively regulates the output molecule (in other words in case of a positive gain process), the first anti-controller molecule is positively regulated by the output molecule. Alternatively, in case the actuator molecule negatively regulates the output molecule (in other words in case of a negative gain process), the first controller molecule is positively regulated by the output molecule. In this manner, a closed control loop between the actuator molecule and the output molecule is formed via the first controller molecule and the first anti-controller molecule.

In certain embodiments, the first anti-controller molecule inactivates, particularly completely inactivates, the first controller molecule, and the first controller molecule inactivates, particularly completely inactivates, the first anti-controller molecule. In particular, the inactivation reaction between the first controller molecule and the first anti-controller molecule is stoichiometrically fixed.

In certain embodiments, the first anti-controller molecule and the first controller molecule physically interact, particularly bind to each other (e.g., in case of proteins) or hybridize (e.g., in case of nucleic acids) to negatively regulate, particularly inactivate, each other.

In certain embodiments, the first anti-controller molecule and the first controller molecule physically interact to inactivate each other, wherein the first anti-controller molecule abolishes a biological function of the first controller molecule, particularly a binding activity of the first controller molecule to a target molecule (e.g., target DNA, RNA or protein), wherein the first controller molecule sequesters the first anti-controller molecule.

In certain embodiments, the first anti-controller molecule and the first controller molecule annihilate each other to negatively regulate, particularly inactivate, each other.

In certain embodiments, the first controller molecule comprises or is a sense mRNA encoding the actuator molecule or a sense mRNA coding for an activator, e.g., a transcriptional activator of a gene encoding the actuator molecule, which positively regulates the actuator molecule, and wherein the first anti-controller molecule comprises or is an anti-sense RNA comprising a sequence which is complementary to a sequence of the sense mRNA. The sense mRNA and the anti-sense RNA hybridize which results in an inhibition of translation of the sense mRNA. At the same time, the hybridization prevents the antisense RNA from interacting with other sense mRNA molecules.

In certain embodiments, the first controller molecule is an activator protein which positively regulates production of the actuator molecule, e.g., by activating transcription of a gene encoding the actuator molecule, activating translation of an mRNA encoding the actuator molecule or inhibiting degradation of an mRNA encoding the actuator molecule or inhibiting degradation of the actuator molecule or by negatively regulating an inhibitor of the function of the actuator molecule, and wherein the first anti-controller molecule is an anti-activator protein, wherein the activator protein and the anti-activator protein form a complex, wherein the positive regulation of the actuator molecule by the activator protein is inhibited by formation of the complex. At the same time, the complex formation prevents the anti-activator protein from interacting with other activator protein molecules.

In particular, this antithetic motif may be combined with the feedback mechanism of the feedback molecule to achieve a molecular proportional integral controller (PI controller).

In certain embodiments, to provide a molecular PI controller, the cell expresses at least one recombinant gene encoding a first controller molecule, a first anti-controller molecule and particularly a feedback molecule, wherein in case the actuator molecule positively regulates the output molecule, i.e., in case of a positive gain process (N-type PI controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first anti-controller molecule (resulting in integral control), and
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control).

Alternatively, in case the actuator molecule negatively regulates the output molecule, i.e., in case of a negative gain process (P-type PI controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control), and
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control).

In certain embodiments, the actuator molecule positively regulates the output molecule (in other words, the network between the actuator molecule and the output molecule represents a positive gain process), wherein the first controller molecule is positively regulated by the output molecule.

In certain embodiments, the actuator molecule negatively regulates the output molecule (in other words, the network between the actuator molecule and the output molecule represents a negative gain process), wherein the first anti-controller molecule is positively regulated by the output molecule.

In certain embodiments, to implement a second order PID controller, the cell expresses at least one recombinant gene encoding a first controller molecule, a second controller molecule and particularly a feedback molecule, wherein in case the actuator molecule positively regulates the output molecule, i.e., in case of a positive gain process (N-type second order PID controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first anti-controller molecule (resulting in integral control),

- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the output molecule positively regulates the first controller molecule (this component combined with the Proportional component results in a filtered PD control).

Alternatively, in case the actuator molecule negatively regulates the output molecule, i.e., in case of a negative gain process (P-type second order PID controller)

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the output molecule positively regulates the first anti-controller molecule (this component combined with the Proportional component results in a filtered PD control)

In certain embodiments, the cell further expresses a recombinant gene encoding a second controller molecule.

In certain embodiments, in case the actuator molecule positively regulates the output molecule (positive gain process), the second controller molecule is positively or negatively regulated by the output molecule and the second controller-molecule negatively regulates the actuator molecule.

In certain embodiments, in case the actuator molecule positively regulates the output molecule (positive gain process), the second controller molecule is negatively regulated by the output molecule and the second controller-molecule positively or negatively regulates the actuator molecule.

In certain embodiments, in case the actuator molecule negatively regulates the output molecule (negative gain process), the second controller molecule is positively or negatively regulated by the output molecule and the second controller-molecule positively regulates the actuator molecule.

In certain embodiments, in case the actuator molecule negatively regulates the output molecule (negative gain process), the second controller molecule is positively regulated by the output molecule and the second controller-molecule positively or negatively regulates the actuator molecule.

By the additional second controller molecule, derivative control is implemented in the network. In combination with integral control (e.g., via an antithetic motif) and proportional control (e.g., using an artificial feedback loop), a molecular third-order proportional-integral-derivative (PID) controller may be implemented. This controller is a third-order controller due to the three involved species: first controller molecule, first anti-controller molecule, second controller molecule.

In certain embodiments, to implement a molecular third-order PID controller, the cell expresses at least one recombinant gene encoding a first controller molecule, a first anti-controller molecule, a second controller molecule and particularly a feedback molecule, wherein in case the actuator molecule positively regulates the output molecule, i.e., in case of a positive gain process (N-type third-order PID controller):

- the first controller molecule positively regulates the actuator molecule, the first anti-controller molecule negatively regulates the first controller molecule, the first controller molecule negatively regulates the first anti-controller molecule (antithetic motif), and the output molecule positively regulates the anti-controller molecule (resulting in integral control),
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the second controller molecule is positively or negatively regulated by the output molecule, and the second controller molecule negatively regulates the actuator molecule (together with the proportional controller this results in a filtered PD controller), or the second controller molecule is negatively regulated by the output molecule and the second controller molecule positively or negatively regulates the actuator molecule (together with the proportional controller this results in a filtered PD controller). In both cases, when the regulation of the second controller molecule and the regulation of the actuator molecule have opposite signs (one positive, the other negative), the filtered PD controller approximates a pure PD controller. When the signs are the same, the filtered PD controller approximates a so-called

EP 3 995 577 A1

LAG controller.

Alternatively, in case the actuator molecule negatively regulates the output molecule, i.e., in case of a negative gain process (P-type third-order PID controller),

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the second controller molecule is positively or negatively regulated by the output molecule, and the second controller molecule positively regulates the actuator molecule (together with the proportional controller this results in a filtered PD controller), or the second controller molecule is positively regulated by the output molecule and the second controller molecule positively or negatively regulates the actuator molecule (together with the proportional controller this results in a filtered PD controller). In both cases, when the regulation of the second controller molecule and the regulation of the actuator molecule have opposite signs (one positive, the other negative), the filtered PD controller approximates a pure PD controller. When the signs are the same, the filtered PD controller approximates a so-called LAG controller.

In certain embodiments, the cell further expresses at least one recombinant gene encoding a second anti-controller molecule, wherein the second anti- controller molecule negatively regulates the second controller molecule, and wherein the second controller molecule negatively regulates the second anti-controller molecule, wherein in case the actuator molecule positively regulates the output molecule, i.e., in case of a positive gain process, the second controller molecule is negatively regulated by the output molecule, and in case the actuator molecule negatively regulates the output molecule, i.e., in case of a negative gain process, the second controller molecule is positively regulated by the output molecule.

According to this embodiment, the second controller molecule and the second anti-controller molecule form a second antithetic motif which, in particular, can be used to implement a molecular fourth order proportional-integral-derivative (PID) controller to control the network in the cell.

In certain embodiments, the second controller molecule negatively regulates itself.

In certain embodiments, to implement a fourth order PID controller, the cell expresses at least one recombinant gene encoding a first controller molecule, a first anti-controller molecule, a second controller molecule, a second anti-controller molecule and particularly a feedback molecule, wherein in case the actuator molecule positively regulates the output molecule, i.e., in case of a positive gain process (N-type fourth order PID controller),

- the first controller molecule positively regulates the actuator molecule, the first anti-controller molecule negatively regulates the first controller molecule, the first controller molecule negatively regulates the first anti-controller molecule (first antithetic motif), the output molecule positively regulates the first anti-controller molecule, (resulting in integral control),
- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the second controller molecule negatively regulates the actuator molecule, the second anti-controller molecule negatively regulates the second controller molecule, the second controller molecule negatively regulates the second anti-controller molecule (second antithetic motif), the output molecule negatively regulates the second controller molecule, and the second controller molecule negatively regulates itself (resulting in derivative control).

Alternatively, in case the actuator molecule negatively regulates the output molecule, i.e., in case of a negative gain process (P-type fourth order PID controller),

- the first controller molecule positively regulates the actuator molecule, the first anti-controller molecule negatively regulates the first controller molecule, the first controller molecule negatively regulates the first anti-controller molecule (antithetic motif), and the output molecule positively regulates the first controller molecule (resulting in integral control),
- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and

- the second controller molecule positively regulates the actuator molecule, the second anti-controller molecule negatively regulates the second controller molecule, the second controller molecule negatively regulates the second anti-controller molecule (second antithetic motif), the second controller molecule negatively regulates itself, and the output molecule positively regulates the second controller molecule (resulting in derivative control).

In certain embodiments, the second controller molecule negatively regulates itself.

In certain embodiments (particularly in case of any one of the above-described N-type or P-type PI controllers, N-type or P-type second order, third order or fourth order PID controllers), the first anti-controller molecule inactivates, particularly completely inactivates, the first controller molecule, and the first controller molecule inactivates, particularly completely inactivates, the first anti-controller molecule. In particular, the inactivation reaction between the first controller molecule and the first anti-controller molecule is stoichiometrically fixed.

In certain embodiments (particularly in case of any one of the above-described N-type or P-type PI controllers, N-type or P-type second order, third order or fourth order PID controllers), the first anti-controller molecule and the first controller molecule physically interact, particularly bind to each other (e.g., in case of proteins) or hybridize (e.g., in case of nucleic acids) to negatively regulate, particularly inactivate, each other.

In certain embodiments, (particularly in case of any one of the above-described N-type or P-type PI controllers, N-type or P-type second order, third order or fourth order PID controllers) the first anti-controller molecule and the first controller molecule physically interact to inactivate each other, wherein the first anti-controller molecule abolishes a biological function of the first controller molecule, particularly a binding activity of the first controller molecule to a target molecule (e.g., target DNA, RNA or protein), wherein the first controller molecule sequesters the first anti-controller molecule.

In certain embodiments (particularly in case of any one of the above-described N-type or P-type PI controllers, N-type or P-type second order, third order or fourth order PID controllers), the first anti-controller molecule and the first controller molecule annihilate each other to negatively regulate, particularly inactivate, each other.

In certain embodiments, the second controller molecule is a sense mRNA encoding a regulator protein, particularly a transcriptional activator or transcriptional repressor, which regulates expression of the actuator molecule, wherein the second anti-controller molecule is an antisense RNA comprising a complementary sequence to a sequence of the sense mRNA encoding the regulator protein, wherein particularly in case the feedback molecule is an additional mRNA encoding the actuator molecule (e.g., for a P-type controller in case of negative gain process), the sense mRNA may encode a regulator protein which negatively regulates the expression of the additional mRNA encoding the actuator molecule.

In certain embodiments, the second controller molecule is an RNA binding protein binding to an untranslated region of an mRNA encoding the actuator molecule, thereby negatively or positively regulating the actuator molecule, e.g., by inhibiting or activating translation or promoting or inhibiting degradation of the mRNA, and wherein the second anti-controller molecule is an anti-RNA-binding protein, wherein the RNA binding protein and the anti-RNA-binding protein form a complex, wherein the negative or positive regulation of the actuator molecule by the RNA binding protein is inhibited by formation of the complex.

In certain embodiments, in case the actuator molecule positively regulates the output molecule (positive gain process), the first controller molecule is positively or negatively regulated by the output molecule, and the first controller molecule negatively regulates the actuator molecule.

In certain embodiments, in case the actuator molecule positively regulates the output molecule (positive gain process), the first controller molecule is negatively regulated by the output molecule and the first controller molecule positively or negatively regulates the actuator molecule.

In certain embodiments, in case the actuator molecule negatively regulates the output molecule (negative gain process), the first controller molecule is positively or negatively regulated by the output molecule, and the first controller molecule positively regulates the actuator molecule.

In certain embodiments, in case the actuator molecule negatively regulates the output molecule (negative gain process), the first controller molecule is positively regulated by the output molecule and the first controller molecule positively or negatively regulates the actuator molecule.

In this manner, a molecular derivative controller may be implemented using only one controller species (the first controller molecule). Whether the output molecule positively or negatively regulates the first controller molecule is determined by the parameters of the network. In particular, this type of derivative control may be combined with proportional control by an artificial feedback loop to implement a molecular PD controller.

In certain embodiments, to implement a proportional-derivative (PD) controller, the cell expresses at least one recombinant gene encoding a first controller molecule and particularly a feedback molecule, wherein in case the actuator molecule positively regulates the output molecule, i.e., in case of a positive gain process (N-type PD controller),

- the feedback molecule is positively regulated by the output molecule, and the feedback molecule negatively regulates the actuator molecule, or (in case no feedback molecule is provided), the output molecule negatively regulates the actuator molecule, particularly directly (resulting in proportional control), and
- the first controller molecule is positively or negatively regulated by the output molecule and the first controller molecule negatively regulates the actuator molecule (together with the proportional controller this results in a filtered PD controller), or the first controller molecule is negatively regulated by the output molecule and the first controller molecule positively or negatively regulates the actuator molecule (together with the proportional controller this results in a filtered PD controller). In both cases, when the regulation of the first controller molecule and the regulation of the actuator molecule have opposite signs (one positive, the other negative), the filtered PD controller approximates a pure PD controller. When the signs are the same, the filtered PD controller approximates a so-called LAG controller.

Alternatively, in case the actuator molecule negatively regulates the output molecule, i.e., in case of a negative gain process (P-type PD controller),

- the feedback molecule is positively regulated by the output molecule, and the feedback molecule positively regulates the actuator molecule, or (in case no feedback molecule is provided), the output molecule positively regulates the actuator molecule, particularly directly (resulting in proportional control), and
- the first controller molecule is positively or negatively regulated by the output molecule and the first controller molecule positively regulates the actuator molecule (together with the proportional controller this results in a filtered PD controller), or the first controller molecule is positively regulated by the output molecule and the first controller molecule positively or negatively regulates the actuator molecule (together with the proportional controller this results in a filtered PD controller). In both cases, when the regulation of the first controller molecule and the regulation of the actuator molecule have opposite signs (one positive, the other negative), the filtered PD controller approximates a pure PD controller. When the signs are the same, the filtered PD controller approximates a so-called LAG controller.

A fourth aspect of the invention relates to the cell according to the second or third aspect of the invention or the expression system according to the first aspect of the invention for use as a medicament.

A fifth aspect of the invention relates to the cell according to the second or third aspect or the expression system according to the first aspect of the invention for use in a method for the treatment or prevention of an immunological condition, particularly cytokine release syndrome or rheumatoid arthritis.

A sixth aspect of the invention relates to the cell according to the second or third aspect or the expression system according to the first aspect of the invention for use in a method for the treatment or prevention of a metabolic or endocrine condition, particularly diabetes.

A seventh aspect of the invention relates to a method for controlling a network in a cell, particularly the cell according to the second or third aspect, wherein the method comprises expressing the at least one recombinant gene of the expression system according to the first aspect of the invention in the cell.

An eighth aspect of the invention relates to the use of a cell according to the second or third aspect or the expression system according to the first aspect in the manufacture of a medicament.

A ninth aspect of the invention relates to the use of a cell according to the second or third aspect or the expression system according to the first aspect in the manufacture of a medicament for the treatment or prevention of an immunological condition, particularly cytokine release syndrome or rheumatoid arthritis.

A tenth aspect of the invention relates to the use of a cell according to the second or third aspect or the expression system according to the first aspect in the manufacture of a medicament for the treatment or prevention of a metabolic or endocrine condition, particularly diabetes.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

*Short Description of the Figures*

Fig. 1    shows an example of a molecular N-type integral controller according to the invention;

Fig. 2    shows an example of a molecular N-type PI controller according to the invention;

Fig. 3    shows an example of a molecular N-type second order PID controller according to the invention;

Fig. 4    shows an example of a molecular N-type third order PID controller according to the invention;

Fig. 5    shows an example of a molecular N-type fourth order PID controller according to the invention;

Fig. 6    shows an example of a molecular P-type integral controller according to the invention;

Fig. 7    shows an example of a molecular P-type PI controller according to the invention;

Fig. 8    shows an example of a molecular P-type second order PID controller according to the invention;

Fig. 9    shows an example of a molecular P-type third order PID controller according to the invention;

Fig. 10    shows an example of a molecular P-type fourth order PID controller according to the invention;

Fig. 11    shows a further example of a molecular N-type integral controller according to the invention;

Fig. 12    shows a further example of a molecular N-type PI controller according to the invention;

Fig. 13    shows a further example of a molecular N-type second order PID controller according to the invention;

Fig. 14    shows a further example of a molecular N-type third order PID controller according to the invention;

Fig. 15    shows a further example of a molecular N-type fourth order PID controller according to the invention;

Fig. 16    shows a further example of a molecular P-type integral controller according to the invention;

Fig. 17    shows a further example of a molecular P-type PI controller according to the invention;

Fig. 18    shows a further example of a molecular P-type second order PID controller according to the invention;

Fig. 19    shows a further example of a molecular P-type third order PID controller according to the invention;

Fig. 20    shows a further example of a molecular P-type fourth order PID controller according to the invention;

Fig. 21    shows the network topology of an arbitrary molecular network with an embedded antithetic integral feedback motif for a positive gain process (N-type controller, left) and a negative gain process (P-type controller, right).

Fig. 22    shows a comparison of open- and closed-loop dynamics (A) and the dynamics of the antithetic motif is given by the system of ordinary differential equations (B);

Fig. 23    shows data illustrating perfect adaptation of a synthetic antithetic integral feedback circuit in mammalian cells.

Fig. 24    shows data illustrating responses to a perturbation to the regulated network.

Fig. 25    shows an implementation of a Proportional-Integral Controller according to the invention.

Fig. 26    shows a mathematical model describing closed and open loop integral control and corresponding fitting results.

Fig. 27    shows a list of biochemical species used in a mathematical model;

Fig. 28    shows a detailed biochemical reaction network used in a mathematical model describing the controller according to the invention;

Fig. 29    shows a schematic representation of a mathematical model describing a molecular PI controller according to the invention for a positive gain process (left, N-type controller) and a negative gain process (right, P-type controller);

Fig. 30    shows a schematic representation of a mathematical model describing a molecular PD controller according to the invention for a positive gain process (left, N-type controller) and a negative gain process (right, P-type controller);

Fig. 31 shows a schematic representation of a mathematical model describing a molecular second order PID controller according to the invention for a positive gain process (left, N-type controller) and a negative gain process (right, P-type controller);

Fig. 32 shows a schematic representation of a mathematical model describing a molecular third order PID controller according to the invention for a positive gain process (left, N-type controller) and a negative gain process (right, P-type controller);

Fig. 33 shows a schematic representation of a mathematical model describing a molecular fourth order PID controller according to the invention for a positive gain process (left, N-type controller) and a negative gain process (right, P-type controller).

## Detailed Description of the Figures

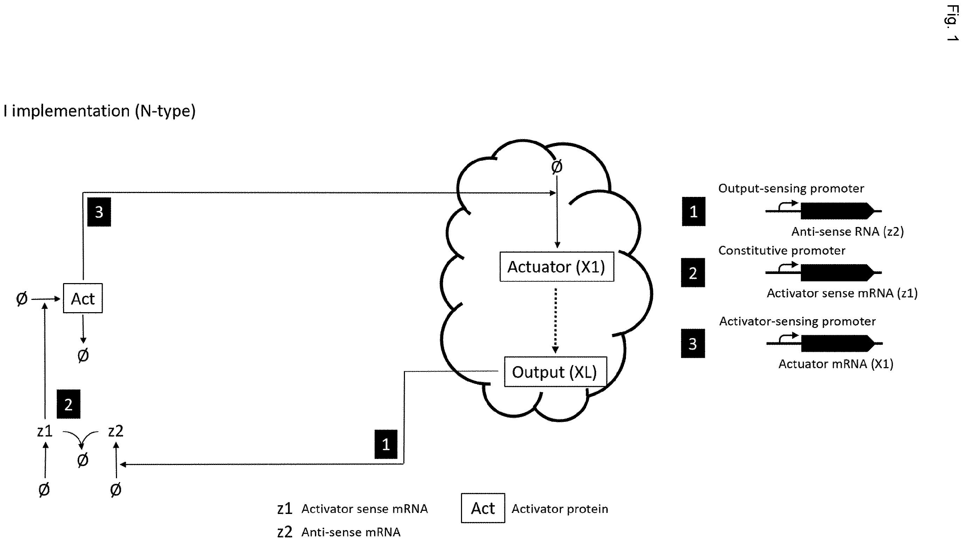

Fig. 1 shows an example of a molecular N-type integral controller according to the invention based on an antithetic integral feedback motif formed by an activator sense mRNA z1 (first controller molecule) and an anti-sense RNA z2 (first anti-controller molecule). The cloud on the right side of Fig. 1 symbolizes the regulated network in a biological cell comprising the actuator X1 and the output XL, wherein the actuator X1 positively regulates the output XL (positive gain process), particularly indirectly, i.e. by a plurality of further molecules of the network. The activator sense mRNA z1 is the product of a first recombinant gene (construct and branch labelled "2") expressed in the cell under a constitutive promoter. In the depicted example, the activator sense mRNA z1 is translated yielding the Activator protein Act which is a positive transcriptional regulator of the of a recombinant gene (construct and branch labelled "3") encoding the actuator mRNA X1 (actuator molecule), i.e. the gene encoding X1 has an activator-sensing promotor. A second gene encoding the anti-sense RNA z2 (construct and branch labelled "1") is recombinantly expressed in the cell under a promotor which is positively regulated by the output molecule XL. The anti-sense RNA has a complementary sequence to the activator sense RNA z1 and thus hybridizes to z1 resulting in an inactive complex z1-z2, blocking translation of z1 and ultimately leading to degradation of z1 and z2 (antithetic motif).

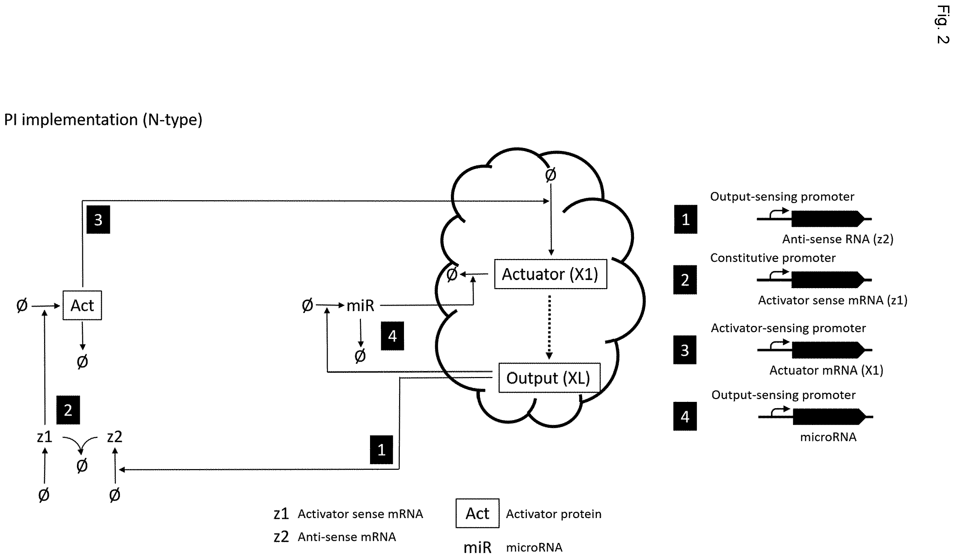

Fig. 2 shows an example of a molecular N-type proportional integral controller according to the invention. Integral control is implemented by the same RNA-based antithetic motif as shown in Fig. 1 and described above (constructs and branches 1 to 3). In addition, a further recombinant gene (construct and branch labelled "4") encoding a microRNA (feedback molecule) is expressed in the cell under a promotor which is positively regulated by the output molecule XL. The microRNA binds to untranslated regions of the actuator mRNA X1, thereby blocking translation and initiating degradation of the actuator mRNA. Thereby, a negative feedback between XL and X1 is implemented resulting in proportional control in addition to the integral control by the antithetic motif.

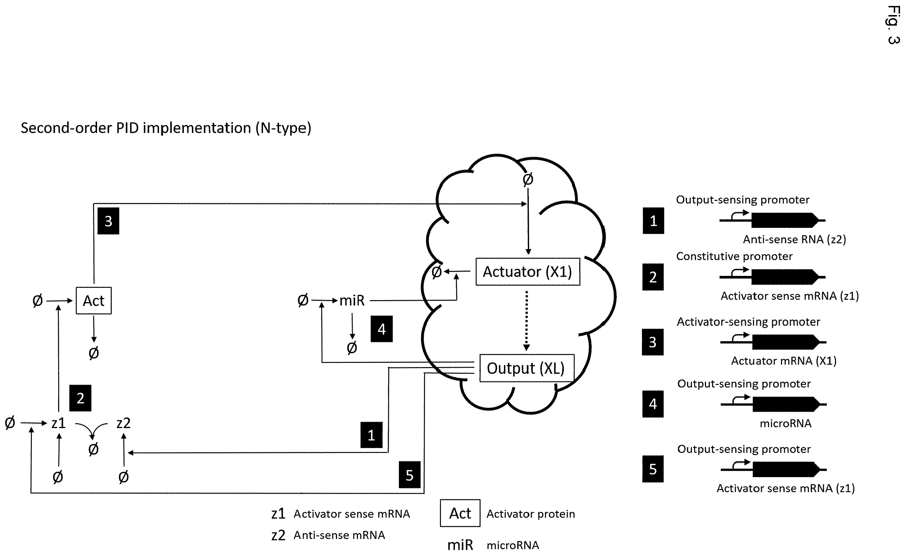

Fig. 3 shows an example of a molecular N-type second order PID controller according to the invention. The RNA-based antithetic motif and negative feedback mechanism are implemented as shown in Fig. 1 and 2 and described above (constructs and branches 1 to 4). In addition, to obtain derivative control, a further recombinant gene (construct and branch labelled "5") encoding a further copy of the activator sense mRNA z1 (first controller molecule) is expressed in the cell under the control of a promotor which is positively regulated by the output molecule XL.

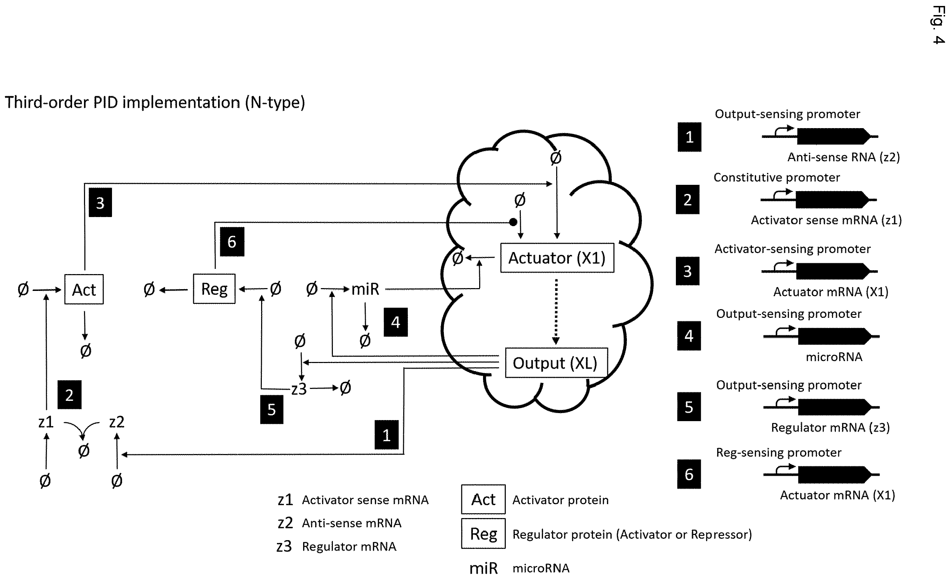

Fig. 4 shows an example of a molecular N-type third order PID controller according to the invention. The RNA-based antithetic motif and negative feedback mechanism are implemented as shown in Fig. 1 and 2 and described above (constructs and branches 1 to 4). In addition, a further recombinant gene (construct and branch labelled "5") encoding a regulator mRNA z3 (second controller molecule) is expressed in the cell under the control of a promotor which is positively regulated by the output molecule XL. The regulator mRNA encodes a Regulator protein, which is a transcriptional activator or repressor of a further recombinant gene (construct "6" with Regulator protein sensing promoter and branch "6") encoding a further copy of the actuator mRNA X1.

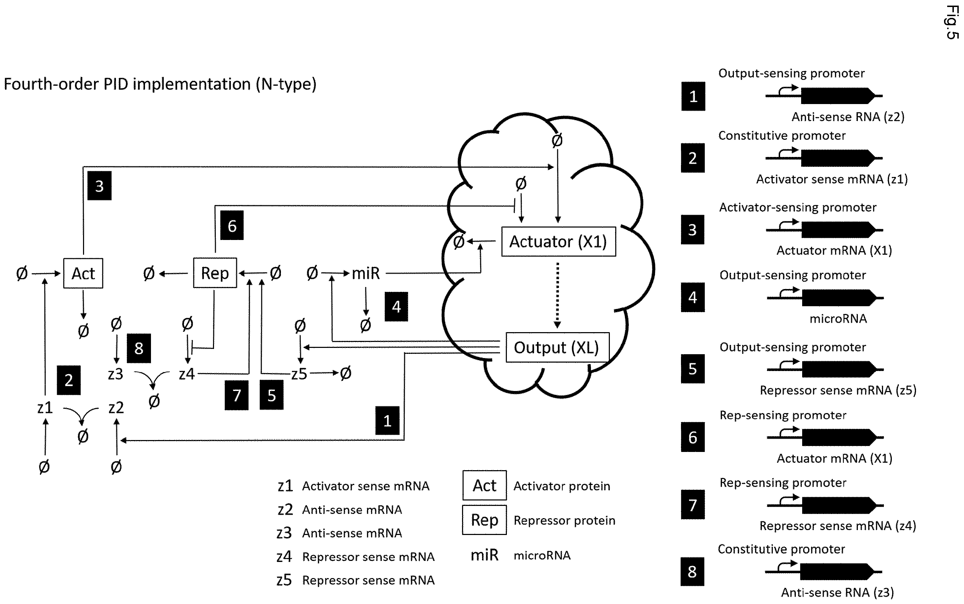

Fig. 5 shows an example of a molecular N-type fourth order PID controller according to the invention. The RNA-based antithetic motif and negative feedback mechanism are implemented as shown in Fig. 1 and 2 and described above (constructs and branches 1 to 4). Additionally, a recombinant gene (construct "5") encoding a repressor sense mRNA z5 is expressed in the cell under a promoter which is positively regulated by the output molecule XL. The repressor sense mRNA z5 is translated to a Repressor protein Rep which represses the transcription of a recombinant gene (construct "6" with Rep-sensing promoter) encoding a further copy of the actuator mRNA X1. Furthermore, a recombinant gene (construct "7") encoding a repressor sense mRNA z4 (second control molecule) is expressed under the negative control of the Repressor protein Rep (Rep-sensing promoter), and a recombinant gene (construct "8") encoding, an anti-sense RNA z3 (second anti-controller molecule) which is complementary to the mRNA z4 is expressed under a constitutive promoter. The mRNA z4 and the anti-sense RNA z3 hybridize and form an inactive complex blocking translation of the mRNA z4 and resulting in degradation. Hence, z3 and z4 form a further antithetic motif involved in derivative control of the network.

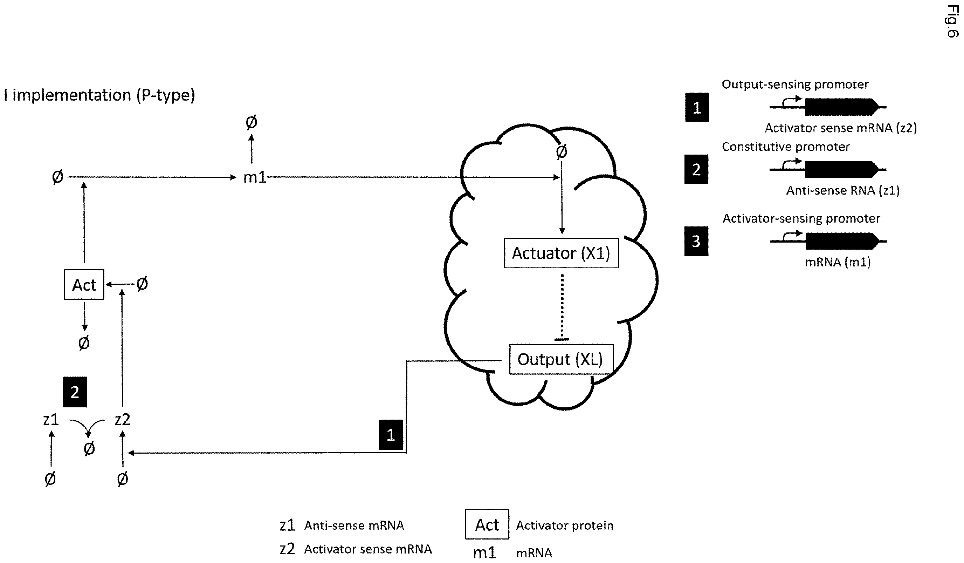

Fig. 6 shows an example of a molecular P-type integral controller according to the invention based on an antithetic integral feedback motif formed by an activator sense mRNA z2 and an anti-sense RNA z1. In this example, the actuator molecule X1 negatively regulates the output molecule XL (negative gain process). The activator sense mRNA z2 (first controller molecule) is recombinantly expressed (construct "1") under a promoter which is positively regulated by the output molecule XL. The activator sense RNA z2 is translated to yield an activator protein Act, which is a positive transcriptional regulator of the mRNA m1 which is recombinantly expressed (construct "3") in the cell under an activator Act-sensing promoter. The gene product of the mRNA m1 positively regulates the production of the actuator molecule X1 (directly or indirectly). The anti-sense RNA z1 (first anti-controller molecule) is expressed under a constitutive promoter (see construct "2") and has a complementary sequence to z2, such that z1 and z2 form an inactive complex interfering with translation of z2 (and thus a reduction in Act protein production) and leading to RNA degradation of the complex. Thus, z1 and z2 form an antithetic motif resulting in integral control of the network.

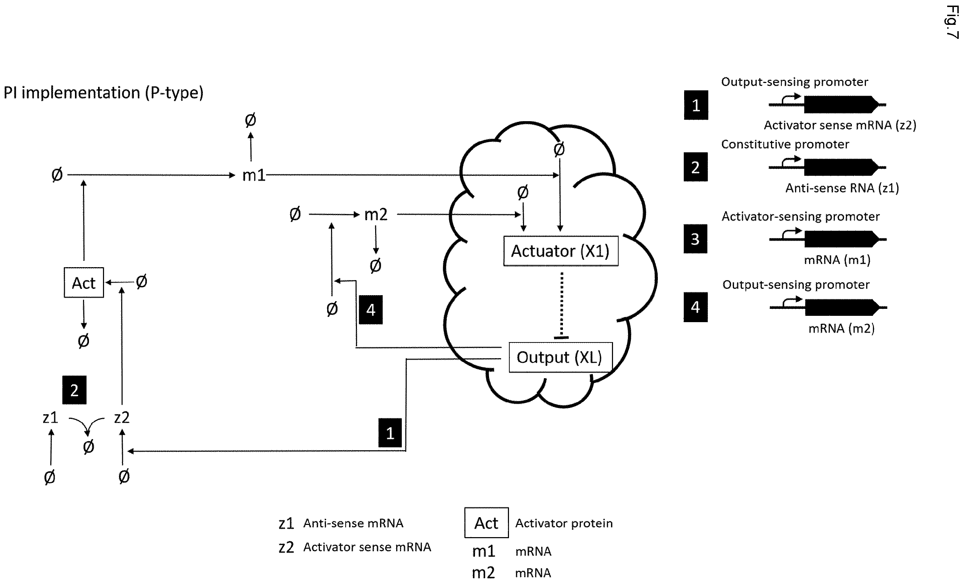

Fig. 7 shows an example of a molecular P-type PI controller (controlling a negative gain process) according to the invention comprising all components shown in Fig. 6 and described above. In addition, a further mRNA m2 (feedback molecule) is recombinantly expressed in the cell from the construct labelled "4" under a promoter which is positively regulated by the output molecule XL (output-sensing promoter). The mRNA m2 encodes a protein which positively regulates (directly or indirectly) the production of the actuator molecule X1 (either from its natural gene or from a further recombinant gene copy). This results in a feedback loop between the output molecule XL and the actuator molecule X1 resulting in proportional control of the network in addition to the integral control mediated by the antithetic motif.

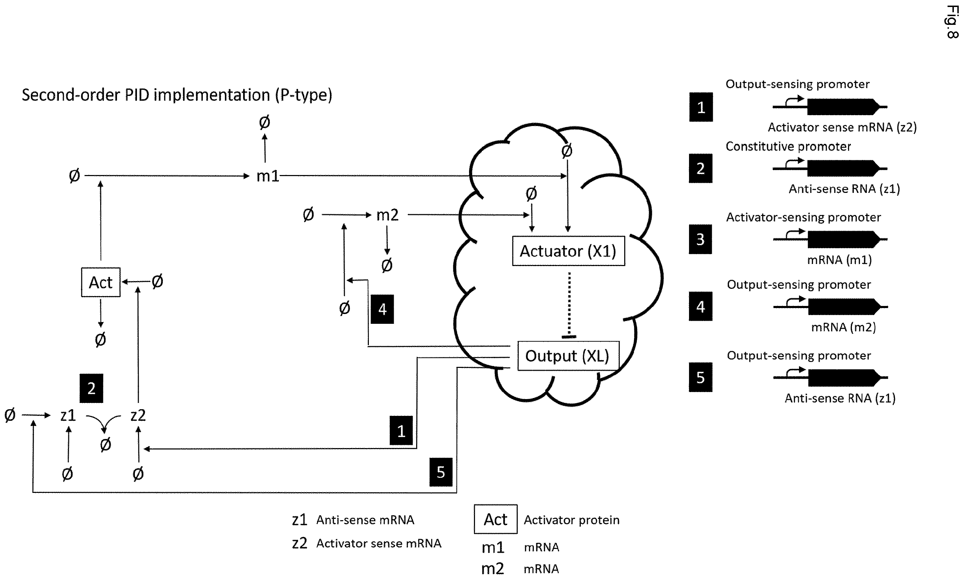

Fig. 8 shows an example of a molecular P-type second-order PID controller according to the invention comprising all components (constructs 1 to 4) shown in Fig. 6 and 7. In addition, a further copy of the anti-sense RNA z1 (first controller molecule) is expressed in the cell (construct "5") under a promoter which is positively regulated by the output molecule XL to implement derivative control of the network.

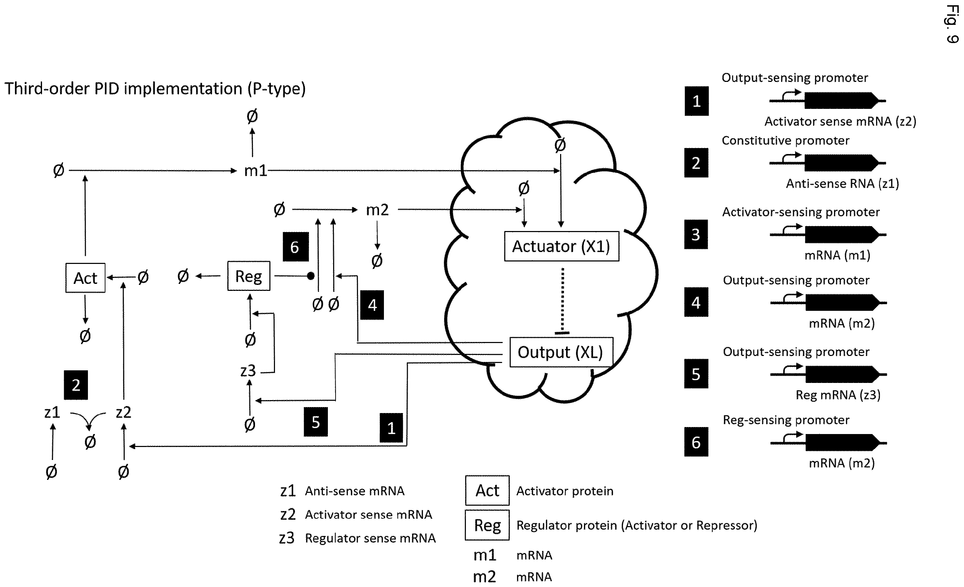

Fig. 9 shows an example of a molecular P-type third-order PID controller according to the invention comprising all components (constructs 1 to 4) shown in Fig. 6 and 7. Additionally, a regulator sense mRNA z3 (second controller molecule) is recombinantly expressed in the cell (construct "5") under a promoter which is positively regulated by the output molecule XL. The regulator sense mRNA z3 yields a Regulator protein Reg (transcriptional activator or repressor). Furthermore, the mRNA m2 (positive regulator of the actuator molecule X1) is recombinantly expressed in the cell (construct "6") under the control of a promoter which is positively or negatively regulated by the Regulator protein Reg

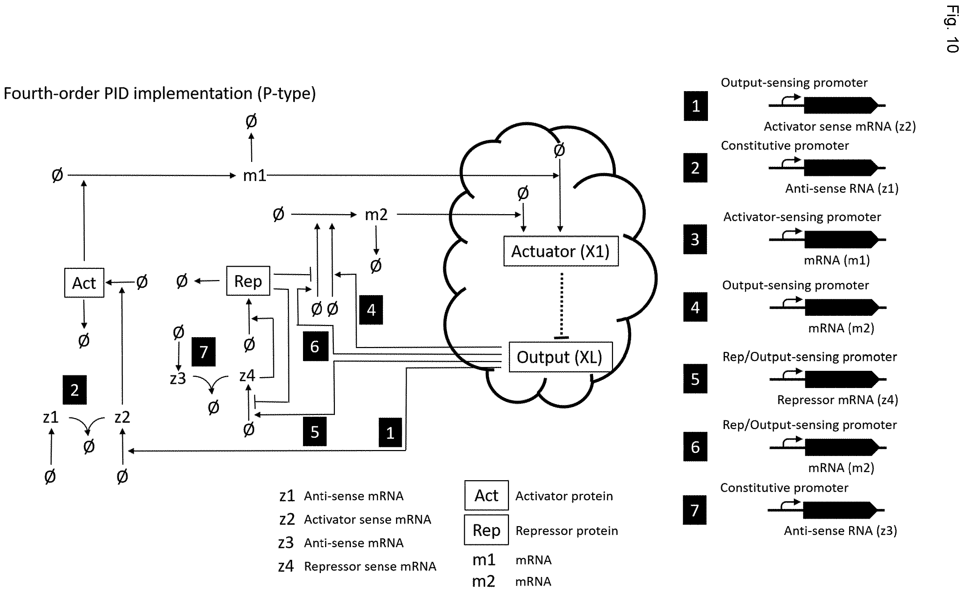

Fig. 10 shows an example of a molecular P-type fourth-order PID controller according to the invention comprising all components (constructs 1 to 4) shown in Fig. 6 and 7. Additionally, a repressor mRNA z4 (second controller molecule) yielding a Repressor protein Rep is recombinantly expressed in the cell from construct "5" under the control of a promoter which is positively regulated by the output molecule XL and negatively regulated by the Repressor protein. A further construct "6" encoding an mRNA m2 is recombinantly expressed in the cell under a promoter which is positively regulated by the output molecule XL and negatively regulated by the Repressor protein. The mRNA m2 positively regulates the actuator molecule X1, e.g. by activating transcription from a further copy of the gene encoding the actuator molecule. Moreover, an anti-sense RNA z3 (second anti-controller molecule) which has a complementary sequence to the repressor mRNA z4 is expressed from a constitutive promoter (construct "7"). As described above for z1 and z2, z3 and z4 form a complex interfering with translation of z4 and ultimately leading to degradation of the mRNAs z3 and z4. Thereby, z3 and z4 form a further antithetic motif contributing to derivative control of the network.

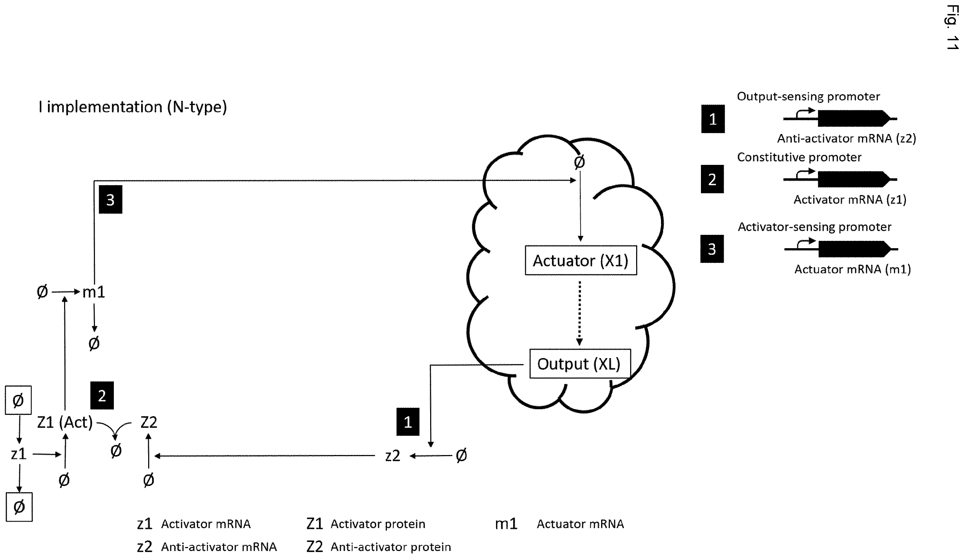

Fig. 11 depicts a further example of a molecular N-type integral controller according to the invention. In contrast to the controller shown in Fig. 1, the antithetic motif is implemented by protein-protein interaction. In the cellular network symbolized by the cloud on the right hand side of Fig. 11, the actuator molecule X1 positively regulates the output molecule XL, in other words a positive gain process is controlled. An activator mRNA z1 is recombinantly expressed in the cell from a constitutive promoter (see construct "2"). The mRNA z1 is translated to yield an activator protein Z1 (first controller molecule, also termed Z1 (Act)). An actuator mRNA m1 which positively regulates the actuator molecule X1, is recombinantly expressed (see construct "3") from a promoter which is positively regulated by the Activator protein Z1. In addition, an anti-activator mRNA z2 is recombinantly expressed under the control of a promoter which is positively regulated by the output molecule XL (see construct "1"). The anti-activator mRNA z2 is translated to the Anti-activator protein Z2 (first anti-controller molecule) which specifically interacts with the activator protein Z1 to sequester and inactivate Z1, resulting in reduction or loss of transcriptional activation of m1 by Z1. The proteins Z1 and Z2 implement a protein-based antithetic motif resulting in integral control of the network.

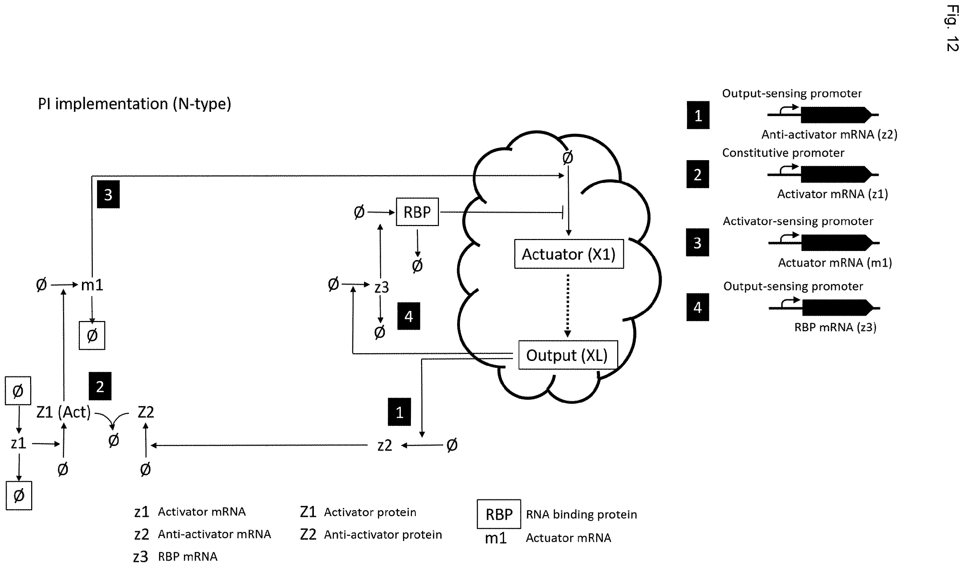

Fig. 12 shows a further example of a molecular N-type PI controller according to the invention. The controller comprises all components shown in Fig. 11 (constructs 1 to 3). In addition, an mRNA z3 encoding an RNA-binding protein RBP (feedback molecule) is recombinantly expressed in the cell (from construct "4") under the control of a promoter which is positively regulated by the output molecule XL. The mRNA is translated to yield the RNA-binding protein RBP which binds to an untranslated region of the mRNA encoding the actuator molecule X1 and inhibits translation of the X1 mRNA, thereby negatively regulating X1. In this manner, a negative feedback loop between XL and X1 is implemented resulting in proportional control.

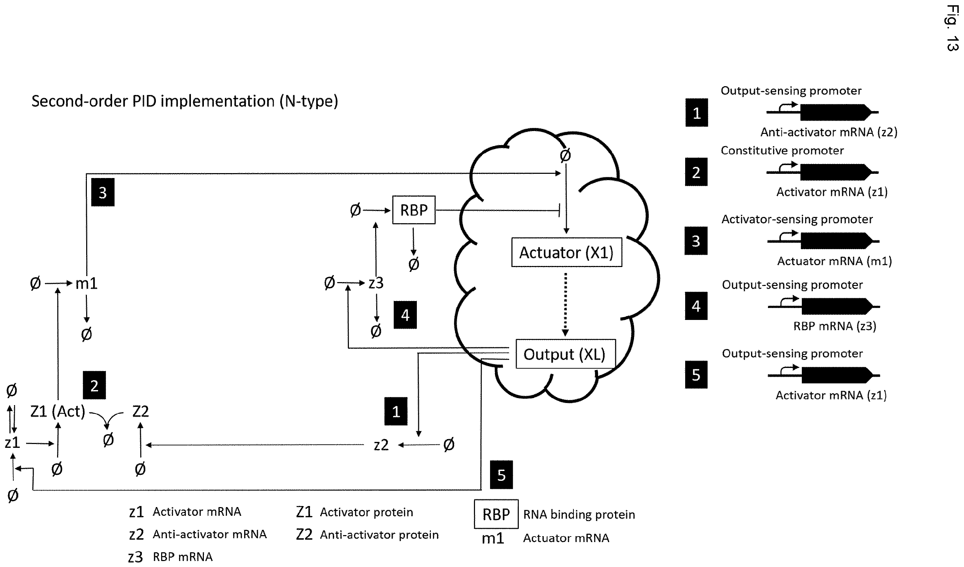

Fig. 13 shows a further example of a molecular N-type second order PID controller according to the invention. The controller comprises all components shown in Fig. 11 and 12 (constructs 1 to 4). Additionally, a second copy of the activator mRNA z1 described above is recombinantly expressed from a promoter which is positively regulated by the output molecule XL (see construct "5", this component combined with the Proportional component results in a filtered PD control).

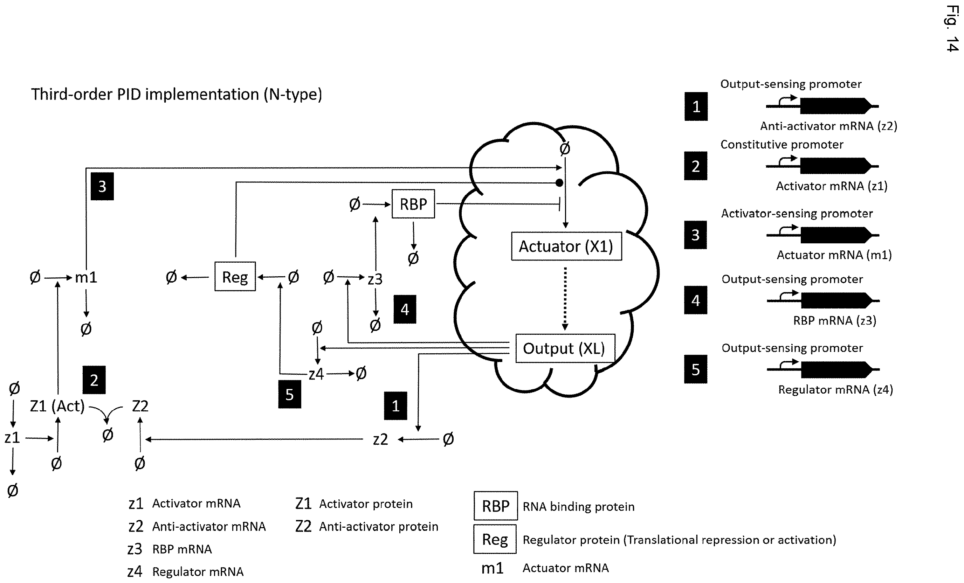

Fig. 14 shows a further example of a molecular N-type third order PID controller according to the invention. The controller comprises all components shown in Fig. 11 and 12 (constructs 1 to 4). Additionally, a regulator mRNA z4 is recombinantly expressed in the cell from a promoter which is positively regulated by the output molecule XL. The mRNA z4 is translated into a regulator protein Reg (second controller molecule) which may be a translational repressor or activator. The regulator protein Reg negatively or positively regulates translation of the mRNA encoding the actuator molecule X1 (this component combined with the Proportional component results in a filtered PD control).

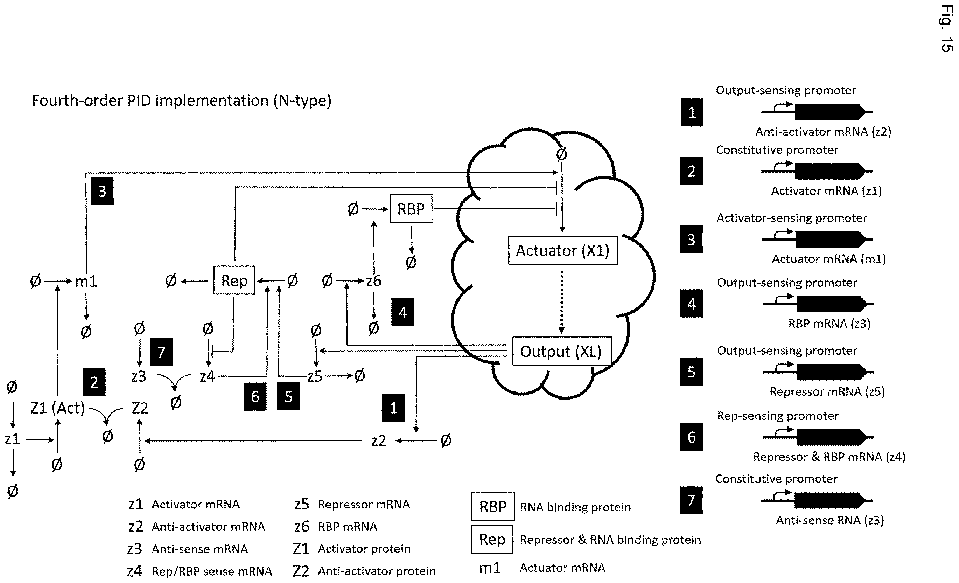

Fig. 15 shows a further example of a molecular N-type fourth order PID controller according to the invention. The controller comprises all components shown in Fig. 11 and 12 (constructs 1 to 4). Additionally, a repressor mRNA z5 is recombinantly expressed in the cell under the control of a promoter which is positively regulated by the output molecule XL (construct "5"). Furthermore, a repressor/RBP sense mRNA z4 (second controller molecule) is recombinantly expressed in the cell (construct "6"). The translation product of z4 is a protein Rep (Repressor and RNA binding protein) with a dual function as a transcriptional repressor of z4 itself and as a further RNA binding protein (in addition to RBP expressed from construct 4) which binds to an untranslated region of the mRNA encoding the actuator molecule X1, thereby inhibiting translation of X1. The mRNA z4 is expressed from construct 5 under a Rep-sensitive promoter which is repressed by the Rep protein. Finally, an anti-sense RNA z3 (second anti-controller molecule) with a complementary sequence to z4 is recombinantly expressed in the cell (construct "7") under a constitutive promoter. The anti-sense RNA z3 forms a complex with the mRNA z4 which interferes with translation of z4 (and thus reduction of Rep protein concentration) and ultimately leads to degradation of z3 and z4. Thereby, a second (RNA-based) antithetic motif is formed by z3 and z4, contributing to derivative control of the network.

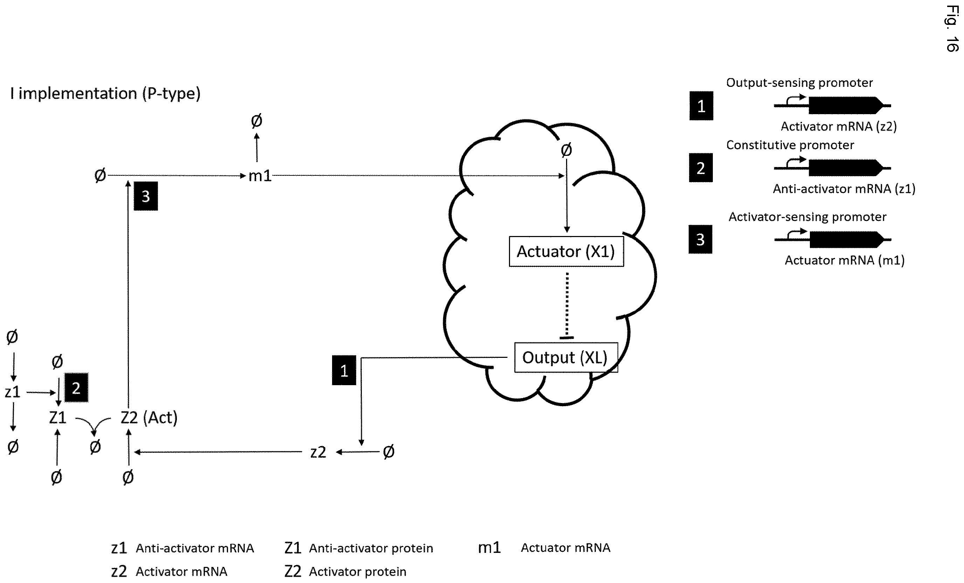

Fig. 16 shows a further example of a molecular P-type integral controller according to the invention. Here, a negative gain process is regulated, i.e., the actuator molecule X1 negatively regulates the output molecule XL. An activator mRNA z2 is recombinantly expressed in the cell under the control of a promoter which is positively regulated by the output molecule XL (construct "1"). The translation product of z2 is an activator protein Z2 (first controller molecule). An anti-activator mRNA z1 is recombinantly expressed in the cell under a constitutive promoter (construct "2"). The mRNA z1 is translated into an Anti-activator protein Z1 (first anti-controller molecule) which specifically binds to the activator protein Z2, thereby sequestering and inactivating the activator protein (antithetic motif based on protein-protein interaction). An actuator mRNA m1 is further recombinantly expressed in the cell under a promoter which is positively regulated by the activator protein Z2 (construct "3"). The mRNA m1 positively regulates (directly or indirectly) the production of the actuator molecule X1.

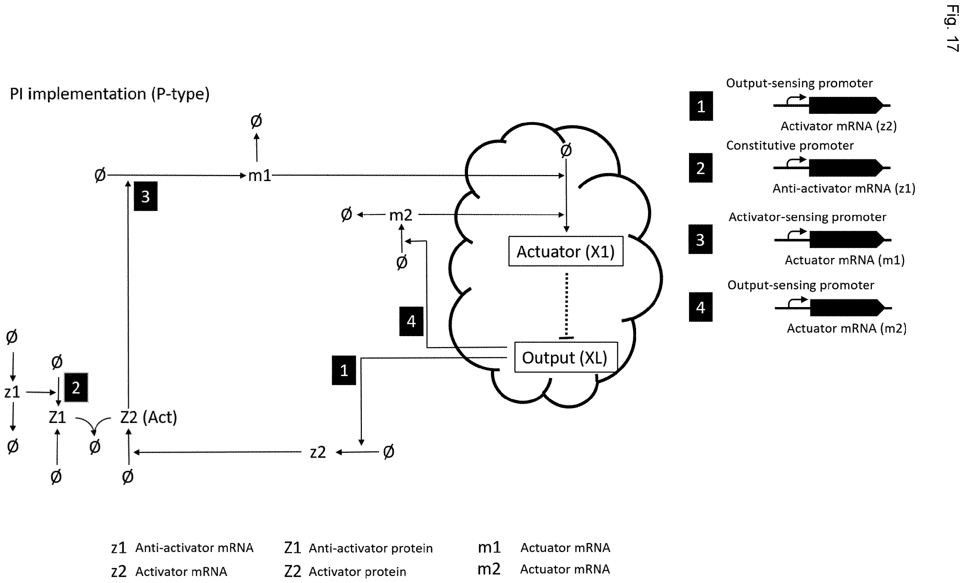

Fig. 17 shows a further example of a molecular P-type PI controller according to the invention. In addition to the components shown in Fig. 16 and described above (constructs 1 to 3), the controller includes a further construct (labelled "4") for recombinant expression of an actuator mRNA m2 (feedback molecule) encoding the actuator molecule X1 (further copy of X1 gene) in the cell under a promoter which is positively regulated by the output molecule XL to implement a negative feedback loop between XL and X1 resulting in proportional control of the network.

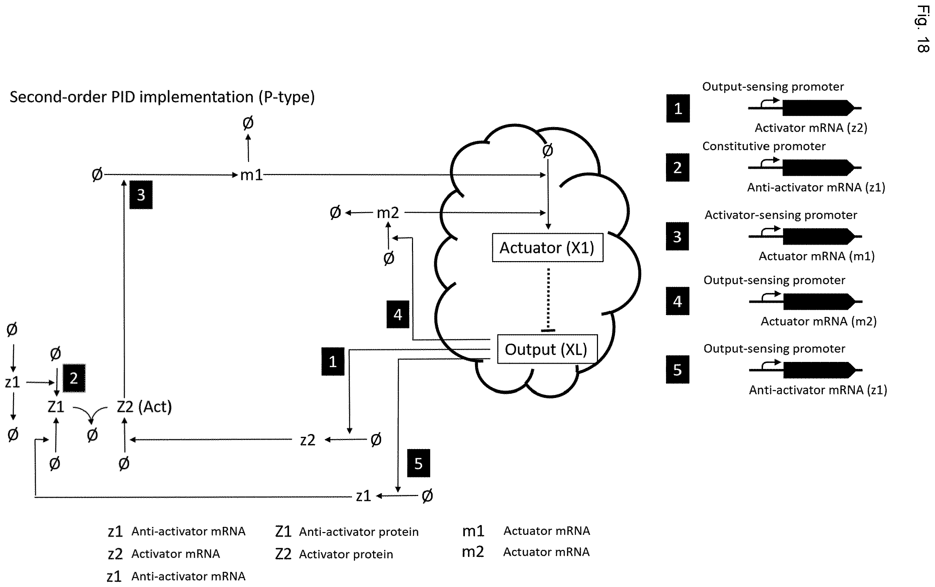

Fig. 18 shows a further example of a molecular P-type second order PID controller according to the invention. The controller comprises all components shown in Fig. 16 and 17 and described above (constructs 1 to 4). In addition, a further copy of the gene encoding the anti-activator mRNA z1 is introduced into the cell via construct "5". Thereby, the anti-activator mRNA z1 is recombinantly expressed under the control of a promoter which is positively regulated by the output molecule XL to achieve derivative control.

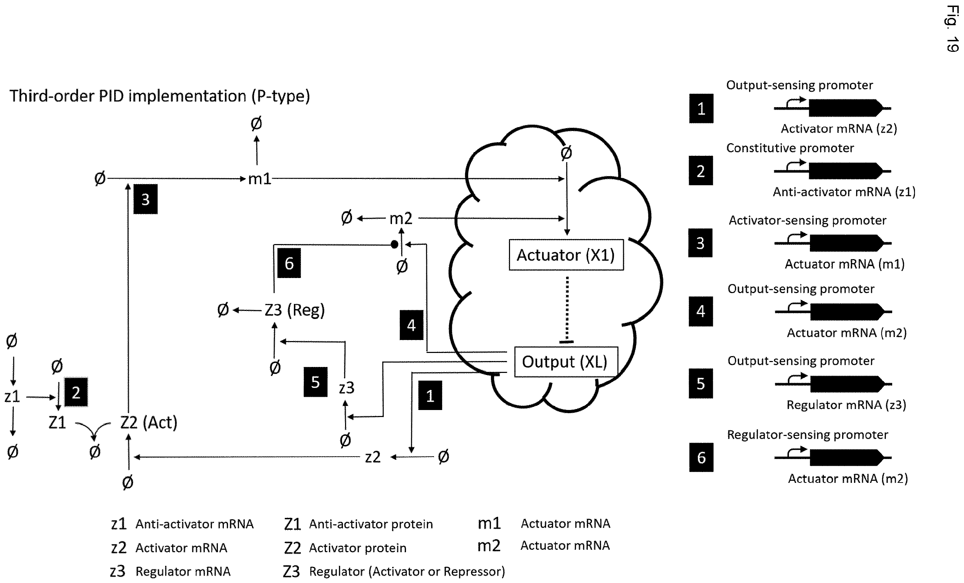

Fig. 19 shows a further example of a molecular P-type third order PID controller according to the invention. The controller comprises all components shown in Fig. 16 and 17 and described above (constructs 1 to 4). In addition, a regulator mRNA z3 is recombinantly expressed in the cell from construct "5" under the control of a promoter which is positively regulated by the output molecule XL. The regulator mRNA is translated into a Regulator protein Z3 (second controller molecule, also designated Z3(Reg) in Fig. 19) which may be a transcriptional activator or a repressor. Furthermore, a further copy of the actuator mRNA m2 is recombinantly expressed from construct "6" under the control of a promoter which is activated or repressed by the Regulator protein Z3. This results in derivative control of the network.

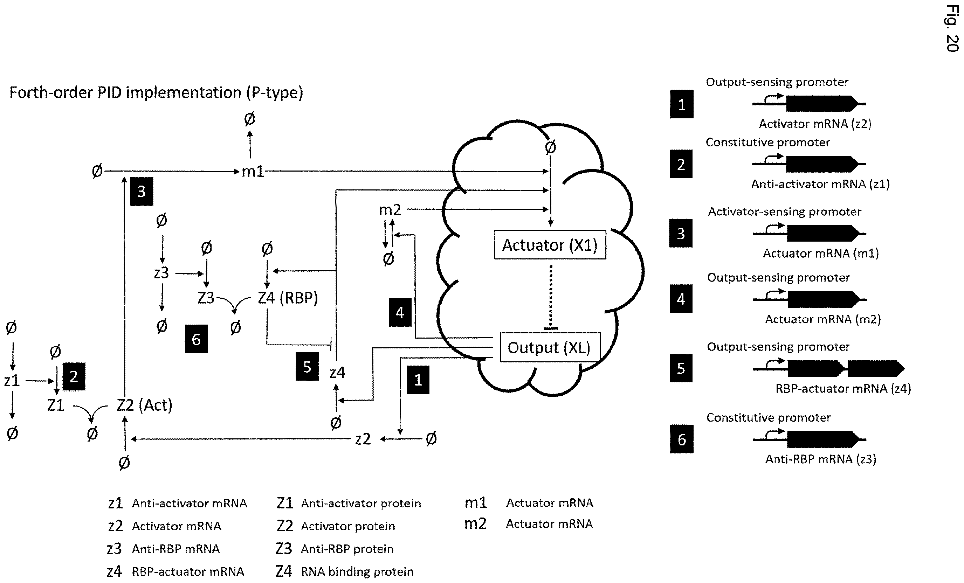

Fig. 20 shows a further example of a molecular P-type fourth order PID controller according to the invention. The controller comprises all components shown in Fig. 16 and 17 and described above (constructs 1 to 4). In addition, a construct "5" is introduced which encodes an RBP-actuator mRNA z4 encoding in tandem an RNA binding protein Z4 (second controller molecule) and the actuator molecule X1, such that they are co-expressed in the cell under the control of a promoter which is positively regulated by the output molecule XL. The RNA binding protein Z4 binds to an untranslated region of the RBP-actuator mRNA z4 and inhibits its translation into Z4 and X1. Furthermore, an anti-RBP mRNA z3 is recombinantly expressed from a constitutive promoter in the cell (see construct "6"). The translation product of z3 is the Anti-RBP protein Z3 (second anti-controller molecule) which forms a complex with the RNA binding protein Z4 leading to inhibition of the RNA-binding function of Z4. Thereby, a second protein-based antithetic motif is implemented by Z3 and Z4, which contributes to derivative control of the network.

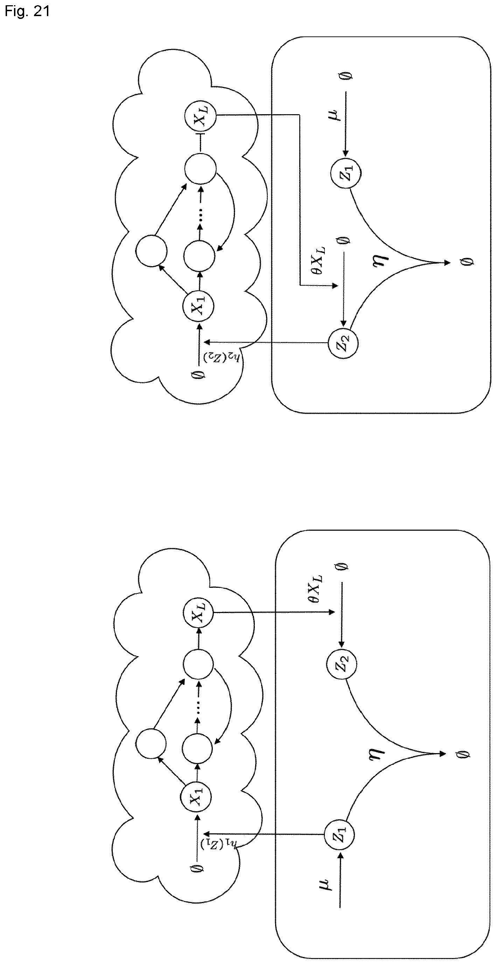

Fig. 21 shows the network topology of an arbitrary molecular network with an embedded antithetic integral feedback motif for a positive gain process (N-type controller, left) and a negative gain process (P-type controller, right). The nodes labelled with $Z_1$ and $Z_2$ (first controller molecule and fist anti-controller molecule) together form the antithetic motif. Species $Z_1$ is created with rate $\mu$ and is functionally annihilated when it interacts with species $Z_2$ with a rate $\eta$. Furthermore, it interacts with the controlled network by promoting the creation of species $X_1$ (actuator molecule). To close the feedback loop, species $Z_2$ is created with a reaction rate that is proportional to $\theta$ and the output species $X_L$ (output molecule).

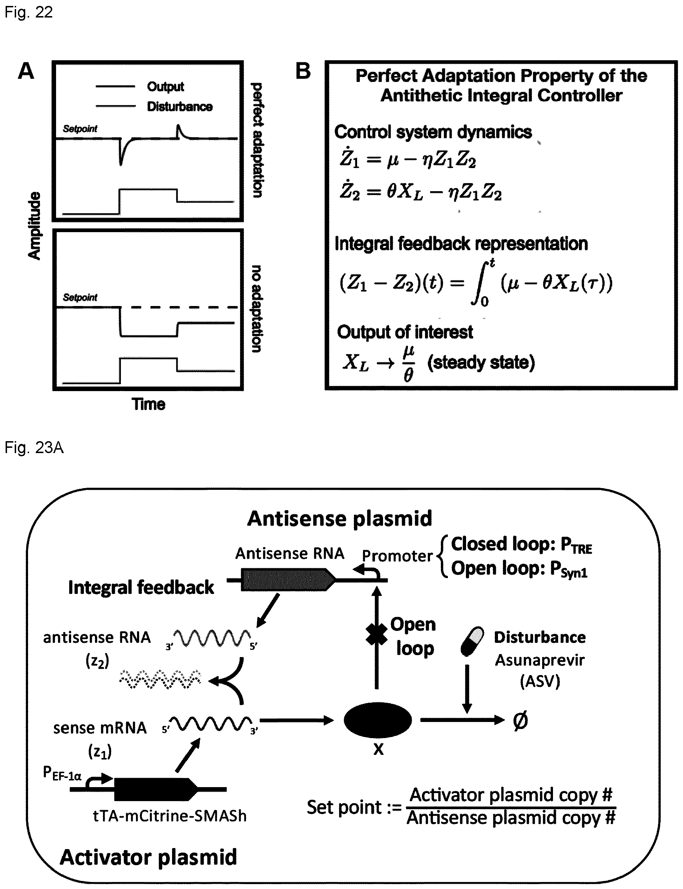

Fig. 22a shows a comparison of open- and closed-loop dynamics. In the absence of any disturbance to the controlled network, both the open- (bottom) and closed-loop (top) systems track the desired setpoint. However, when a disturbance occurs and persists, the open-loop circuit deviates from the desired setpoint while the closed-loop system returns after some transient deviation. This is also the case when after some time the disturbance weakens but still persists. The dynamics of the antithetic motif is given by the system of ordinary differential equations shown in Fig. 22b. Subtracting the ordinary differential equation for species $Z_2$ from the one for species $Z_1$ and integrating, reveals the hidden integral action of the controller that ensures that the steady state of the output converges to a value that is independent of the plant parameters. The long-term behavior of the output is given by the ratio of the two reaction rates $\mu$ and $\theta$. Importantly, this steady state is independent of any rate in the controlled network and is therefore robust to any disturbance in these rates.

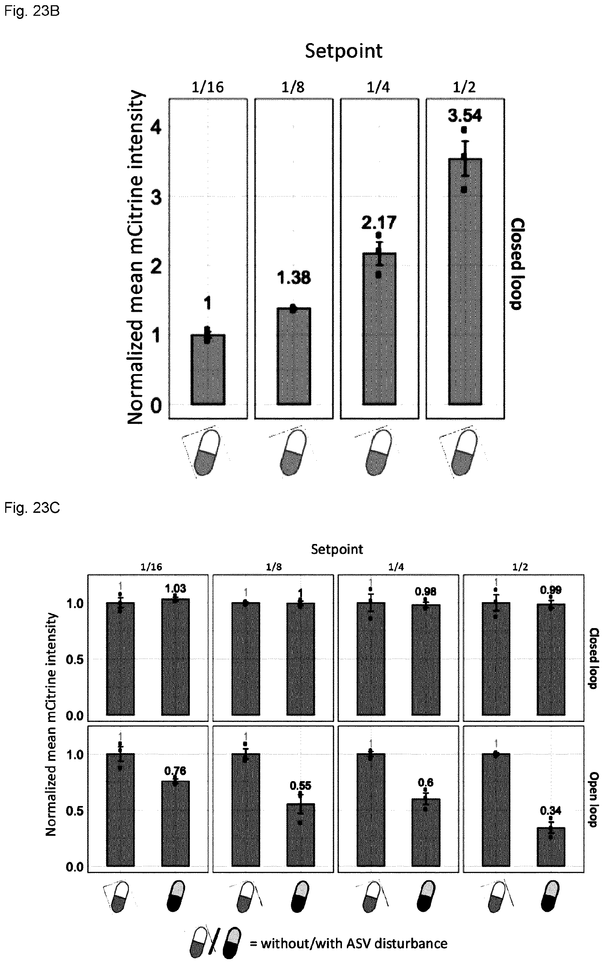

Fig. 23 shows data illustrating perfect adaptation of a synthetic antithetic integral feedback circuit in mammalian cells. Fig. 23a shows a genetic implementation of open- and closed-loop circuits. Both circuits consist of two genes, realized on separate plasmids. The gene in the activator plasmid (first controller molecule) encodes the synthetic transcription factor tTA (tetracycline transactivator) tagged with the fluorescent protein mCitrine and a chemically inducible degradation tag (SMASh). Its expression is driven by a strong constitutive promoter ($P_{EF-1\alpha}$). The gene in the antisense plasmid expresses the antisense RNA (first anti-controller molecule) under the control of a tTA responsive promoter ($P_{TRE}$). In the open-loop configuration, the TRE promoter was exchanged for a non-responsive promoter. In this setting the controlled species is the tTA protein, which can be perturbed externally by addition of Asunaprevir (ASV), the chemical inducer of the SMASh degradation tag. Fig. 23b shows steady-state levels of the output (mCitrine) under increasing plasmid ratios. The genetic implementation of the closed-loop circuit as shown in panel (a) was transiently transfected at different molar ratios (setpoint := *activator / antisense*). The data was collected 48 hours after transfection and is shown as mean per condition normalized to the lowest setpoint (1/16) $\pm$ s.e. for n = 3 replicates. This shows that increasing the plasmid ratio increases the steady-state output level. Fig. 23c shows steady-state response of the open-loop and closed-loop implementations to induced degradation by ASV. The genetic implementation of the open- and closed-loop circuit as shown in Fig. 23a was transiently transfected at different molar ratios and perturbed with 0.033 $\mu$M of ASV. The data was collected 48 hours after transfection and is shown as mean per condition normalized to the unperturbed conditions for each setpoint separately. This demonstrates the disturbance rejection capability of the closed-loop circuit and shows that the open-loop circuit fails to achieve adaptation.

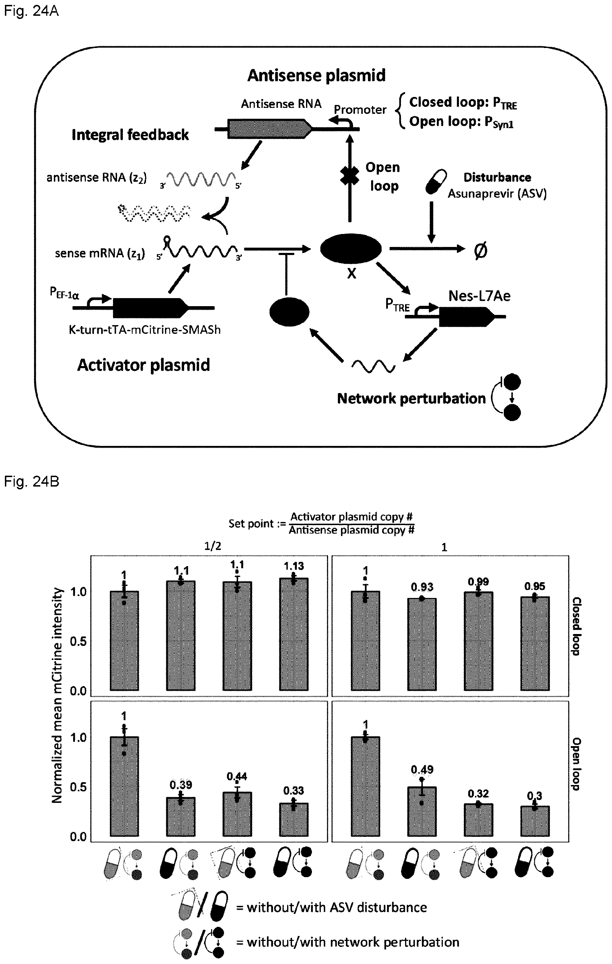

Fig. 24 shows data illustrating responses to a perturbation to the regulated network. Fig. 24a schematically illustrates the extension of the network topology with a negative feedback loop. A negative feedback loop from tTA-mCitrine to its own production was added by expressing the RNA-binding protein L7Ae under the control of a tTA-responsive TRE promoter. This protein binds in the 5' untranslated region of the sense mRNA species to inhibit the translation of tTA. Fig. 24b shows data demonstrating that the closed-loop circuit is impartial to the topology of the regulated network. The closed- and open-loop circuits were perturbed by co-transfecting the network perturbation and by adding 0.033 $\mu$m of ASV. This was done at two setpoints 1/2 and 1 (setpoint := *activator / antisense*). The HEK293T cells were measured using flow cytometry 48 hours after transfection and the data is shown as mean per condition normalized to the unperturbed network and no ASV condition $\pm$ s.e. for n = 3 replicates.

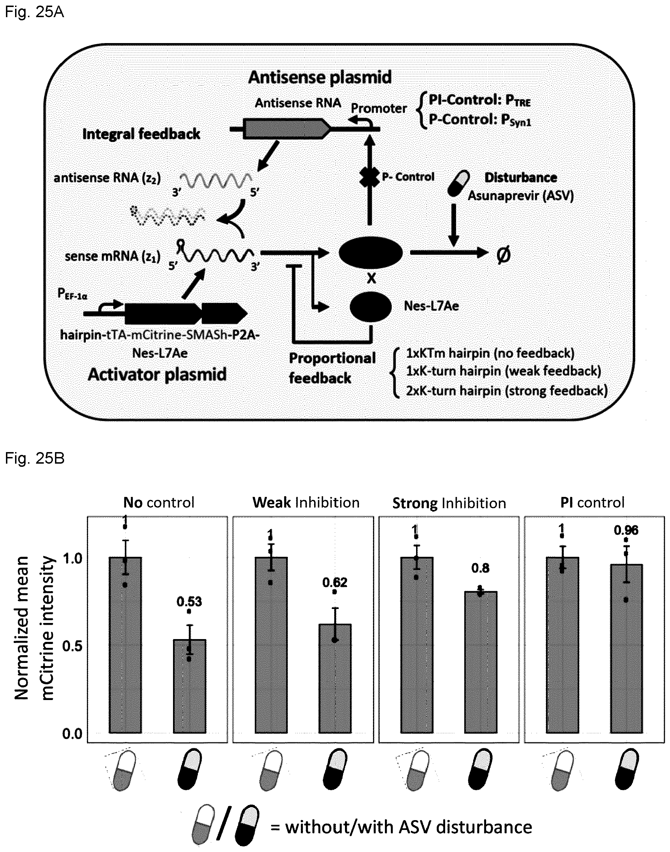

Fig. 25 shows an implementation of a Proportional-Integral Controller according to the invention. Fig. 25a illustrates a genetic implementation of a standalone proportional (P) controller and a Proportional-Integral (PI) controller. A negative feedback loop from the RNA-binding protein L7Ae (which is proxy to tTA-mCitrine since it is simultaneously produced from the same mRNA) is added to the antithetic motif. This protein binds in the 5' untranslated region of the sense mRNA species to inhibit the translation of tTA and itself simultaneously. Stronger proportional feedback is realized by adding additional L7Ae binding hairpins. Fig. 25b shows data demonstrating that a PI controller does not break the adaptation property. The PI and P circuits were perturbed by co-transfecting the network perturbation and by adding 0.033 $\mu$m of ASV. The HEK293T cells were measured using flow cytometry 48 hours after transfection and the data is

EP 3 995 577 A1

shown as mean per condition normalized to the unperturbed (no ASV) condition $\pm$ s.e. for n = 3 replicates. Clearly, controllers without integral feedback fail to meet the adaptation criteria. However, a PI controller ensures adaptation.

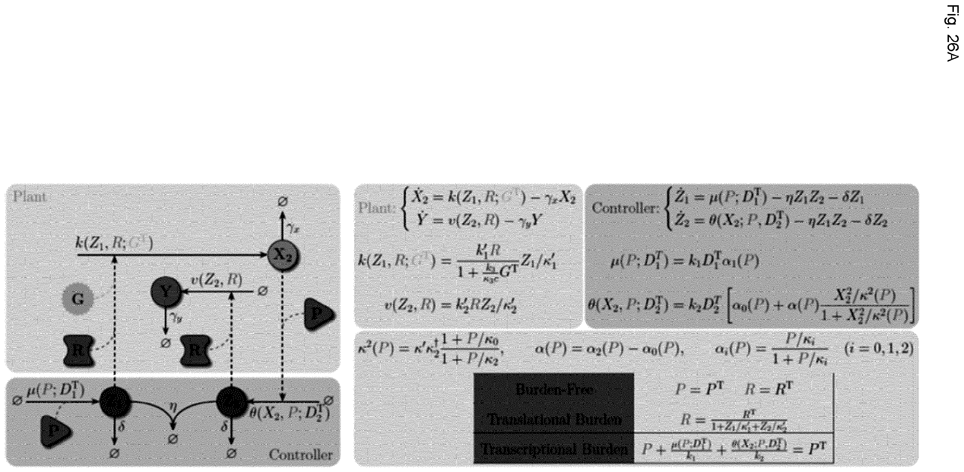

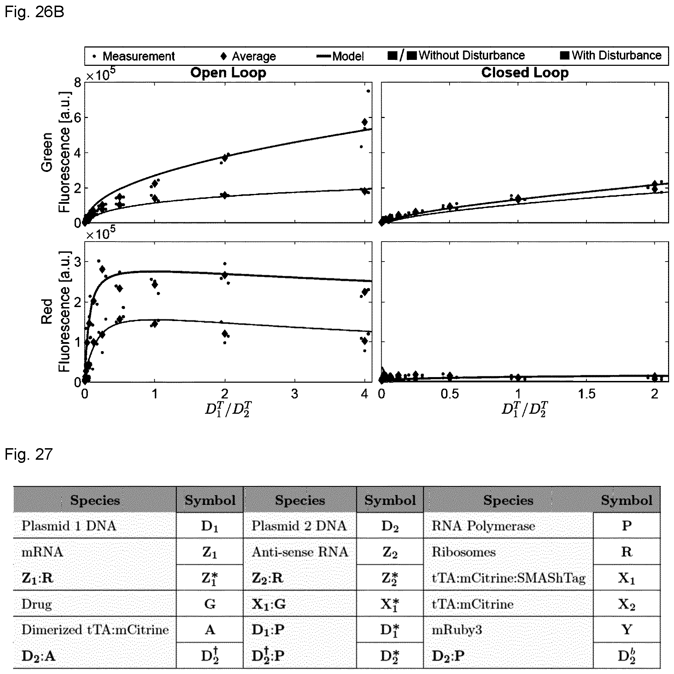

Fig. 26 shows a mathematical model describing closed and open loop integral control and corresponding fitting results. Fig. 26a is a schematic and mathematical description of the reduced model. The sense mRNA, $Z_1$, is constitutively produced at a rate $\mu$ that depends on the total (free and bound) plasmid concentration, $D_1^T$, and the shared transcriptional resources P (e.g. Polymerase). Then, $Z_1$ is translated to a green fluorescent protein, $X_2$, at a rate k that depends on the concentration of $Z_1$, the translational resources R (e.g. Ribosomes), and the total drug concentration $G_T$ which acts as an inhibitor. The protein $X_2$ dimerizes and acts as a transcription factor that activates the transcription of the antisense RNA, $Z_2$. The transcription rate, denoted by $\theta$, is a function of $X_2$, P, and the total plasmid concentration $D_2^T$. The antisense RNA is translated to a red fluorescent protein, Y, at a rate v that depends on $Z_2$ and R. To close the loop, $Z_1$ and $Z_2$ sequester each other at a rate $\eta$. The open loop setting is obtained by setting $\eta = 0$. Transcriptional/translational burden is imposed by the shared resources. Burden can be excluded or included in the model by either making P and R constants, or allowing them to depend on other species as shown in the table. Fig. 26b shows fitting of the model to experimental data. The parameters of the model that considers only translational burden ($P = P^T$) are optimally fit using green and red fluorescence measurements. It is shown that the burden-free scenario cannot fit the data properly, and the full burden scenario does not significantly increase the model fitting accuracy. The fitted model shows a good agreement with the data for the open/closed loop settings, with/without disturbance, and over a wide range of plasmid ratios $D_1^T/D_2^T$. This suggests, mathematically, that the system only exhibits translational burden.

Fig. 27 shows a list of biochemical species used in a mathematical model;

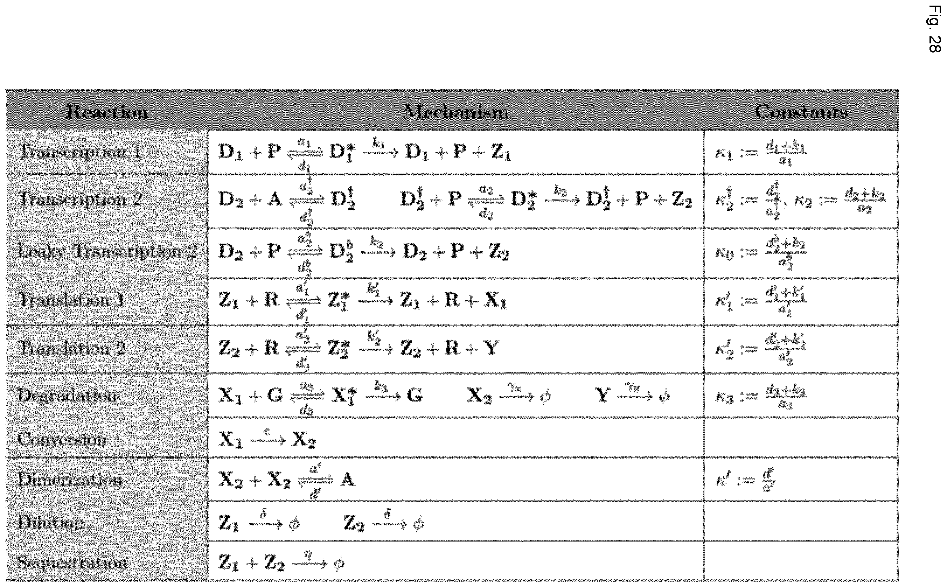

Fig. 28 shows a detailed biochemical reaction network used in a mathematical model describing the controller according to the invention;

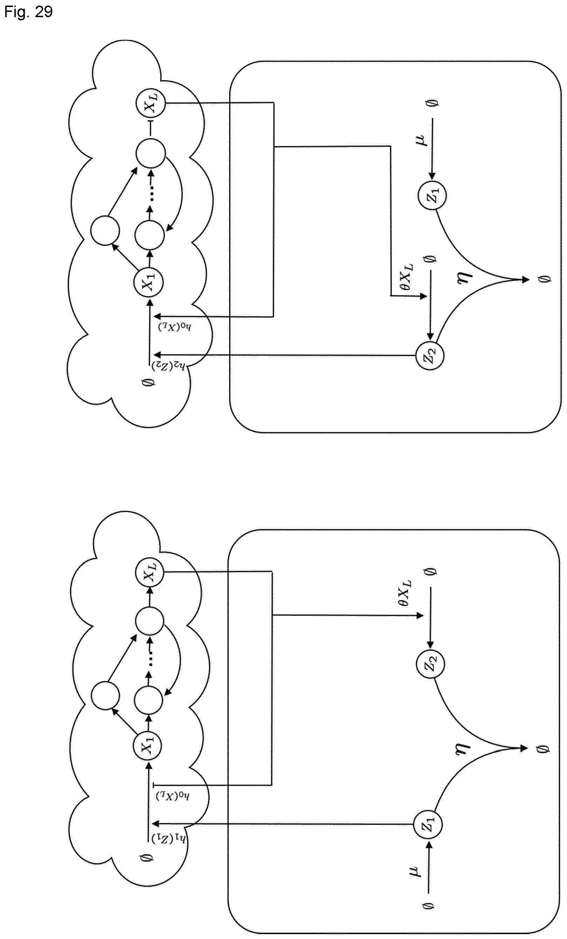

Fig. 29 shows a schematic representation of a mathematical model describing a molecular PI controller based on an antithetic motif with additional feedback control according to the invention for a positive gain process (left, N-type controller) and a negative gain process (right, P-type controller). X1 denotes the actuator molecule, XL denotes the output molecule, Z1 denotes the first controller molecule (left panel) or first anti-controller molecule (right panel), and Z2 denotes the first anti-controller molecule (left panel) or first controller molecule (right panel). $\mu$ is the formation rate of Z1 and $\eta$ is the complex formation / annihilation rate of Z1 and Z2.

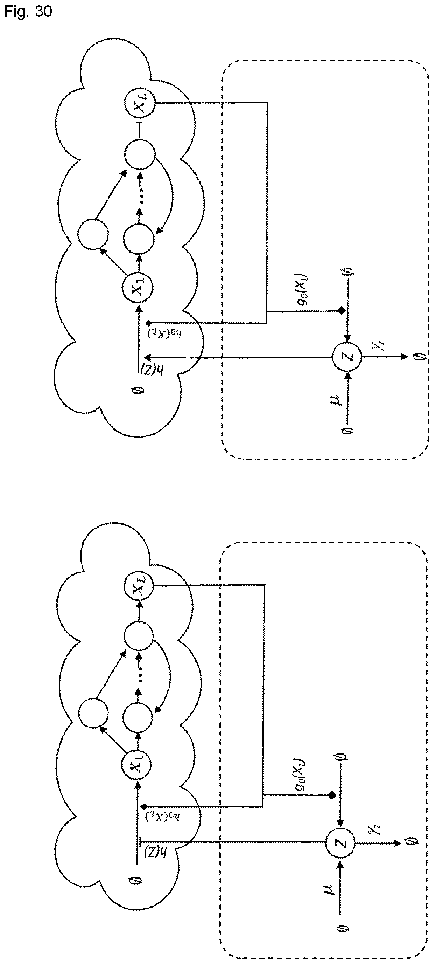

Fig. 30 shows a schematic representation of a mathematical model describing a molecular PD controller according to the invention for a positive gain process (left, N-type controller) and a negative gain process (right, P-type controller). X1 denotes the actuator molecule, XL denotes the output molecule, and Z denotes the first controller molecule. $\mu$ is the formation rate of Z and $\gamma_z$ is the degradation rate of Z.

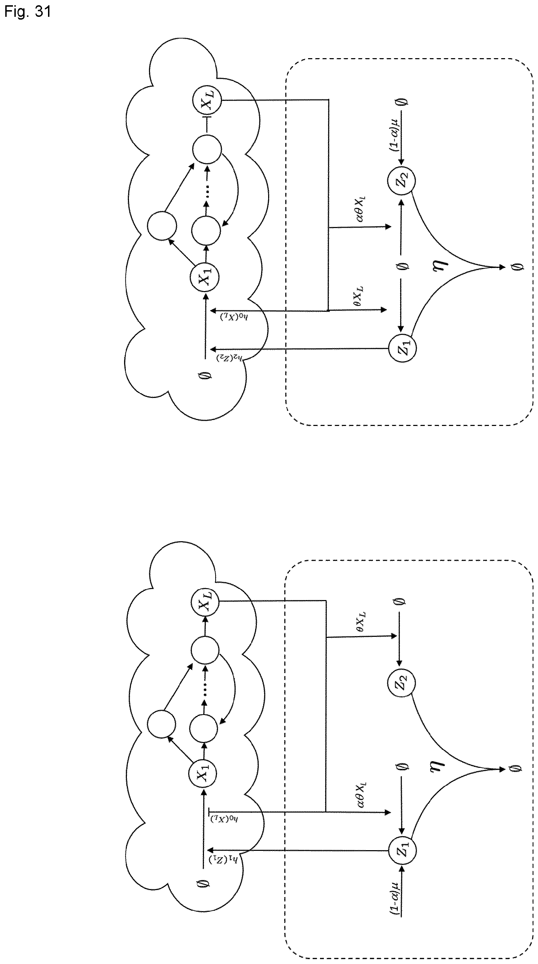

Fig. 31 shows a schematic representation of a mathematical model describing a molecular second order PID controller based on an antithetic motif with additional feedback control according to the invention for a positive gain process (left, N-type controller) and a negative gain process (right, P-type controller). X1 denotes the actuator molecule, XL denotes the output molecule, Z1 denotes the first controller molecule and Z2 denotes the first anti-controller molecule. $\eta$ is the complex formation / annihilation rate of Z1 and Z2.

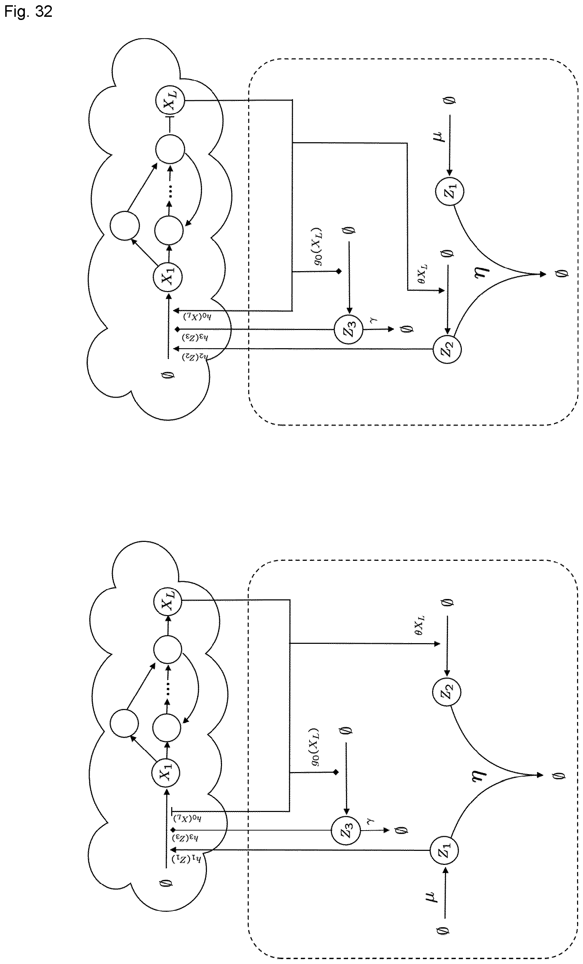

Fig. 32 shows a schematic representation of a mathematical model describing a molecular third order PID controller based on an antithetic motif with additional feedback control according to the invention for a positive gain process (left, N-type controller) and a negative gain process (right, P-type controller). X1 denotes the actuator molecule, XL denotes the output molecule, Z1 denotes the first controller molecule, Z2 denotes the first anti-controller molecule, and Z3 denotes the second controller molecule. $\eta$ is the complex formation / annihilation rate of Z1 and Z2.

Fig. 32 shows a schematic representation of a mathematical model describing a molecular fourth order PID controller based two antithetic motifs with additional feedback control according to the invention for a positive gain process (left, N-type controller) and a negative gain process (right, P-type controller). X1 denotes the actuator molecule, XL denotes the output molecule, Z1 denotes the first controller molecule, Z2 denotes the first anti-controller molecule, Z3 denotes the second controller molecule, and Z4 denotes the second anti-controller molecule. $\eta$ is the complex formation / annihilation rate of Z1 and Z2.

Examples

*Example 1: Antithetic proportional-integral feedback control in mammalian cells*

Here, perfect adaptation is demonstrated in a sense/antisense mRNA implementation of the antithetic integral feedback circuit in mammalian cells and it is shown that the controller is agnostic to the system it is regulating.

*Materials and methods*

*Plasmid construction*

Plasmids for transfection were constructed using a mammalian adaption of the modular cloning (MoClo) yeast toolkit standard (Michael E Lee, William C DeLoache, Bernardo Cervantes, and John E Dueber. A highly characterized yest toolkit for modular, multipart assembly. ACS synthetic biology, 4(9): 975-986, 2015). Custom parts for the toolkit were generated by PCR amplification (Phusion Flash High-Fidelity PCR Master Mix; Thermo Scientific) and assembly into toolkit vectors via golden gate assembly (Carola Engler, Romy Kandzia, and Sylvestre Marillonnet. A one pot, one step, precision cloning method with high throughput capability. PloS one, 3(11), 2008). All enzymes used for applying the MoClo procedure were obtained from New England Biolabs (NEB).

*Cell culture*

HEK293T cells (ATCC, strain number CRL-3216) were cultured in Dulbecco's modified Eagle's medium (DMEM; Gibco) supplemented with 10 % FBS (Sigma-Aldrich), 1x GlutaMAX (Gibco) and 1 mm Sodium Pyruvate (Gibco). The cells were maintained at 37 °C and 5 % $CO_2$. Every 2 to 3 days the cells were passaged into a fresh T25 flask. When required, surplus cells were plated into a 96-well plate at 1e4 cells in 100 $\mu$L per well for transfection.

*Transfection*

Cells used transfection experiments were plated approximately 24 h before treatment with transfection solution. The transfection solution was prepared using Polyethylenimine (PEI) "MAX" (MW40000; Polysciences, Inc.) at a 1:3 ($\mu$g DNA to $\mu$g PEI) ratio with a total of 100 ng plasmid DNA per well. The solution was prepared in Opti-MEM I (Gibco) and incubated for approximately 25 min prior to addition to the cells.

*Flow cytometry*

Approximately 48 h after transfection the cells were collected in 60 $\mu$L Accutase solution (Sigma-Aldrich). The fluorescence was measured on a Beckman Coulter CytoFLEX S flow cytometer using the 488 nm laser with a 525/40+OD1 bandpass filter. For each sample the whole cell suspension was collected. In each measurement additional unstained and single color (mCitrine only) controls were collected for gating and compensation.

*Data analysis*

The acquired data was analyzed using a custom analysis pipeline implemented in the R programming language. The measured events are automatically gated and compensated for further plotting and analysis.

*Results*

A schematic depiction of the sense/antisense RNA implementation of the antithetic integral feedback circuit is shown in Fig. 23A. The basic circuit consist of two genes, which are encoded on separate plasmids. The gene in the *activator plasmid* is the synthetic transcription factor tTA (tetracycline transactivator) fused to the green fluorescent protein mCitrine. The expression of this gene is driven by the strong mammalian EF-1 $\alpha$ promoter. This transcription factor drives the expression of the other gene in the *antisense plasmid.* This gene expresses an antisense RNA that is complementary to the *activator* mRNA. The hybridization of these two species realizes the annihilation reaction and closes the feedback loop. As a control incapable of producing integral feedback, an open-loop analog of the closed-loop circuit was created, in which the tTA-responsive TRE promoter was replaced by a non-responsive promoter. The closed-loop configuration is set up to regulate the expression levels of the activator tTA-mCitrine. To introduce specific perturbations to the activator a Asunaprevir (ASV) inducible degradation tag (SMASh) was additionally fused to tTA-mCitrine.

To show that our genetic implementation of the circuit performs integral feedback constant perturbations were applied with ASV at a concentration of 0.033 $\mu$m to HEK293T cells which were transiently transfected with either the open- or the closed-loop circuit. Additionally, the setpoint was varied by transfecting the two genes at ratios ranging from 1/16 to 1/2. The fluorescence of the cells was measured 48 hours after transfection using flow cytometry. As the setpoint ratio increases, so does the fluorescence of tTAmCitrine, indicating that the circuit permits setpoint control (Fig. 23B). A circuit was considered adapting if its normalized fluorescence intensity stays within 0.1 of the undisturbed control.

Under this criterion, adaptation is achieved for all the setpoints tested in the closed-loop configuration, whereas none of the open-loop configurations managed to meet the criteria for adaptation (Fig. 23C).

Next, it was sought to demonstrate that the implementation of the antithetic integral controller will provide disturbance rejection at different setpoints regardless of the network topology it regulates. Therefore, a negative feedback loop was added from tTA-mCitrine to its own production. This negative feedback was realized by the RNA-binding protein L7Ae, which is expressed under the control of a tTA responsive TRE promoter and binds the 5' untranslated region of the sense mRNA to inhibit translation (Fig. 24A).

The closed- and open-loop circuits were transiently transfected either with or without this negative feedback plasmid to introduce a perturbation to the regulated network. As before, the setpoints 1/2 and 1 were tested by transfecting an appropriate ratio of the activator to antisense plasmids. These different conditions were further perturbed on the molecular level by adding 0.033 $\mu$m ASV to induce degradation of tTA-mCitrine. As shown in Fig. 24B the closed-loop circuit rejects both perturbations in most cases, whereas again the open-loop circuit fails to adapt. However, the closed-loop circuit with a setpoint of 1/2 with both perturbations also fails to meet the adaptation requirement. Nevertheless, it still remains far closer to the desired value as the open-loop circuit in the same conditions.

The capability of the antithetic integral controller to reject topological network perturbations, as demonstrated previously in Fig. 24, allowed to further improve the controller performance by increasing its complexity. In particular, a common control strategy was implemented that is extensively applied in various engineering disciplines and is referred to as a Proportional-Integral (PI) control. This control strategy appends the Integral (I) controller with a Proportional (P) feedback action to enhance the overall performance, such as transient dynamics and variance reduction, while maintaining the adaption property. To implement a proportional feedback control that acts faster than the integral feedback, a proxy protein was used, namely the RNA-binding protein L7Ae, which is produced in parallel with mCitrine-tTA from a single mRNA via the use of P2A self-cleavage peptide (Fig. 25A). Therefore, the expression level of L7Ae is expected to proportionally reflect the level of tTA-mCitrine. The negative feedback is hence realized via the proxy protein that inhibits translation by binding the 5' untranslated region of the sense mRNA. Note that, as opposed to the circuit in Fig. 24A, the production of L7Ae in the PI controller is not regulated by the tTA responsive TRE promoter. In fact, it is directly controlled by the sense mRNA. Furthermore, the proportional feedback realized in the PI controller is expected to act faster than the feedback implemented by the tTA-dependent production of L7Ae (Fig. 24) because it does not require additional transcription and translation steps.

As illustrated in Fig. 25B, controllers without integral feedback fail to meet the adaptation criteria. On the other hand, with a Proportional Integral (PI) controller, the expression of tTA-mCitrine is ensured to be robust to the induced drug disturbance as depicted in Fig. 25. This shows that the additional proportional feedback indeed does not break the adaption property of the antithetic integral controller.

To better understand the mathematical operation of the basic circuit depicted in Fig. 23A, a detailed mechanistic model was derived starting from basic principles of mass-action kinetics. Uppercase letters are used to denote the concentrations of the species represented by their corresponding bold letters.

The detailed model, demonstrated in Fig. 27 and 28, captures the transcription of the two plasmids (denoted by $D_1$ and $D_2$), and the translation of the sense and antisense RNAs (denoted by Z1 and Z2, respectively). The translation of the sense mRNA yields a protein (denoted by $X_1$) that is comprised of tTA, mCitrine and SMAShTag all fused together. The SMAShTag recruits the drug (denoted by G) which in turn degrades the complex $X_1$. The proteins that escape the drug release the SMAShTag, leaving tTA and mCitrine fused together (denoted by $X_2$). When the latter dimerizes, it acts as a transcription factor that activates the production of the antisense RNA. The model also captures the involvement of resources that are shared among different transcription/translation processes. Transcriptional resources (e.g. Polymerases) are denoted by P, and translational resources (e.g. Ribosomes) are denoted by R. Note that an additional translation step - as compared to the circuit of Fig. 23A - is added here, where the antisense RNA is translated to a protein containing mRuby3 (denoted by Y). This allows to obtain an additional set of measurements (red fluorescence) to better mathematically characterize the system.

To obtain a simpler mathematical model, the fully detailed model is reduced based on three mild assumptions (see section "Model Reduction" below). The reduced model is depicted schematically and mathematically in Fig. 26A where $D_1^T$, $D_2^T$, $G^T$, $P^T$ and $R^T$ denote the total concentrations of the plasmids, drug, and resources, respectively, and are assumed to be constants. The reduced model takes the form of a dynamical system which can be divided into a controller module that is connected in feedback with a plant module to be controlled. The open-loop (resp. closed-loop) setting is mathematically realized by setting the sequestration rate $\eta = 0$ (resp. $\eta \gg 0$).

The mathematical complexity of the reduced model depends on the level of modeling detail of the burden imposed by the shared transcriptional and translational resources P and R. Three scenarios of increasing mathematical complexity are considered here. In the simplest scenario, it is assumed that the system is burden-free. That is, the resources P and R are approximately constant and are not affected by the circuit. In the second scenario, it is assumed that the burden originates only from the shared translational resources R. Mathematically, this is realized by making R a hill function of $Z_1$ and $Z_2$ as shown in the table of Fig. 26A. In these two scenarios, the dynamics are described by a set of *Ordinary Differential Equations (ODEs)* in $X_2$; Y, $Z_1$ and $Z_2$ with $P = P^T$. Finally, in the last scenario, transcriptional burden is also considered. This is mathematically realized by adding the algebraic constraint shown in the table of Fig. 26A. This gives an implicit equation for P, and thus resulting with a set of *Differential Algebraic Equations (DAEs)*. The detailed derivations of the reduced model are given in section "Model Reduction" below.

Next, a model fitting was carried out for the three different scenarios. The green fluorescence represents all the molecules involving mCitrine ($X_1 + X_2 +$ dimerized $X_2$), and the red fluorescence represents the molecules involving mRuby3 (Y). It is shown (section "Model Fitting" below) that the burden-free scenario is not enough to properly fitt the available data. However, translational burden is enough to fit the data, and thus Fig. 26B shows an optimal parameter fit of the translational burden scenario. In fact, the model succeeds in fitting the data for the open-loop/closed-loop settings, with/without disturbance, for both green/red fluorescence, and over a wide range of plasmid ratios $D_1^T / D_2^T$. Note that adding transcriptional burden yields only slightly better fitting (due to the additional degrees of freedom) and is thus not considered here.

It can be observed that, in the open-loop setting, the green fluorescence approaches saturation for a high plasmid ratio, and the red fluorescence saturates and starts decreasing for high plasmid ratios. This behavior is a result of burden and cannot be captured with a burden-free model. Furthermore, in the closed-loop setting, it is observed that disturbance rejection is near-perfect for low plasmid ratios, but starts to deteriorate for higher plasmid ratios. This is expected because the circuit exhibits a functional dynamic range which puts a limit on the allowable set-points. This limit is a result of the degradation/dilution of $Z_1$ and $Z_2$ and the burden imposed by the shared resources. Finally, it can be observed that the red fluorescence in the closed-loop setting is very small compared to the open-loop setting. This indicates that the sense-antisense RNA sequestration is highly efficient and, as a result, the circuit exhibits a strong feedback. In fact, the sense mRNA - being constitutively produced - is efficiently sequestering the antisense RNA and keeping it at very low concentrations.

*Discussion*

The presented study demonstrates the first implementation of antithetic integral feedback in mammalian cells. With the proof-of-principle circuit the foundation for robust and predictable control systems engineering in biology is laid.

Based on the antithetic motif (Fig. 21), a proof-of-concept circuit capable of perfect adaptation was designed and built. This was achieved by exploiting the hybridization of mRNA molecules to complementary antisense RNAs. The resulting inhibition of translation realizes the central sequestration mechanism. Specifically, an antisense RNA is expressed through a promoter that is activated by the transcription factor tTA. This antisense RNA is complementary to and binds the mRNA of its tTA to close the negative feedback loop (Fig. 23A). The properties of integral feedback control are highlighted by showing that the circuit permits different setpoints in an approximately 3.5 fold range (Fig. 23B). It is likely, that that fold dynamic range can be improved with further optimization of circuit parameters.

By a disturbance to the regulated species it has been shown that the closed-loop circuit achieves adaptation and is superior to an analogous open-loop circuit (Fig. 23C). Further, it was shown that adaptation is also achieved when the setpoint of the circuit is changed.

Moreover, it was also shown that the realization of the antithetic integral feedback motif is mostly agnostic to the network structure of the regulated species. This was achieved by introducing a perturbation to the network of the controlled species itself (Fig. 24B). Furthermore, it was also demonstrated that the closed-loop circuit still rejects disturbances even in the presence of this extra perturbation to the network. In the open-loop circuit, the disturbance, perturbation and perturbation with disturbance lead to a successively stronger decrease in tTA-mCitrine expression.

Finally, with the goal of enhancing the performance of the antithetic integral controller, a proportional feedback is appended (Fig. 25). It was shown that a standalone proportional controller can reduce the steady-state error of tTA-mCitrine expression, but cannot reduce it enough to meet the adaption criteria. On the other hand, it was shown that a Proportional-Integral (PI) controller does not break the adaptation property of the standalone antithetic motif. It is expected that adding this additional proportional feedback will enhance the performance, such as transient dynamics and variance reduction.

Other than being able to produce integral feedback control, the sense and antisense RNA implementation is very simple to adapt and very generally applicable. Both sense and antisense are fully programmable, with the only requirement that they share sufficient sequence homology to hybridize and inhibit translation. Due to this, mRNAs of endogenous transcription factors may easily be converted into the antithetic motif simply by expressing their antisense RNA from a promoter activated by the transcription factor. However, one should note, that in this case the setpoint to the transcription factor will be lower than without the antisense RNA due to the negative feedback and additionally, if the mRNA of the endogenous transcription factor is not very stable, the integrator is expected to not perform optimally.

It is believed that the ability to precisely and robustly regulate gene expression in mammalian cells will find many applications in industrial biotechnology and biomedicine.

*Full model*

A detailed biochemical reaction network that describes the interactions between the various biochemical species (Fig. 27) is given in Fig. 28.

*Model reduction*

In this section, the full model given in Fig. 28 is mathematically reduced to the model given in Fig. 27 which has been used for the fit shown in Fig. 26b. The model reduction procedure is based on the following assumptions:

**Assumption 1.** *The binding reactions are fast.*
**Assumption 2.** *The SMAShTag is released quickly.*
**Assumption 3.** *The concentration of the complex tTA:mCitrine:SMAShTag is low.*

Assumptions 1 and 2 are based on a time-scale separation principle that exploits the fact that the binding reactions and the only conversion reaction are much faster than the other reactions in the system.

As a result, the Quasi-Steady-State Approximation (QSSA) is applied. It is emphasized that the QSSA gives a reduced model whose dynamics are approximate, but the steady-state behavior is still exact.

Assumption 3 is based on the fact that the complex tTA:mCitrine:SMASHTag ($X_1$) is very unstable, that is, it either quickly loses the SMAShTag (in the conversion reaction) or it quickly binds to the drug which, in turn, rapidly destroys it. More precisely, Assumption 3 is mathematically translated to the following asymptotic inequality: $X_1 \ll \_\kappa_3$. Assumption 3 - unlike Assumptions 1 and 2 - yields an approximate reduced model that is not exact in the steady-state regime.

Now, the mathematical derivation of the reduced model is shown. The conservation laws are given by

$$
\begin{aligned}
D_1 + D_1^* &= D_1^{\mathrm{T}} \\
D_2 + D_2^\dagger + D_2^* + D_2^b &= D_2^{\mathrm{T}} \\
P + D_1^* + D_2^* + D_2^b &= P^{\mathrm{T}} \\
R + Z_1^* + Z_2^* &= R^{\mathrm{T}} \\
G + X_1^* &= G^{\mathrm{T}}.
\end{aligned}
\tag{1}
$$

Since the binding reactions are much faster than the other reactions in the network (Assumption 1), one can invoke the Quasi Steady-State Approximation (QSSA) as follows

$$\dot{D}_1^* \approx 0 \implies a_1 D_1 P - (d_1 + k_1)D_1^* \approx 0 \implies D_1^* \approx \frac{PD_1}{\kappa_1}$$

$$\dot{D}_2^* \approx 0 \implies a_2 D_2^\dagger P - (d_2 + k_2)D_2^* \approx 0 \implies D_2^* \approx \frac{PD_2^\dagger}{\kappa_2}$$

$$\dot{D}_2^\dagger \approx 0 \implies a_2^\dagger D_2 A - d_2^\dagger D_2^\dagger - \left(a_2 D_2^\dagger P - (d_2 + k_2)D_2^*\right) \approx 0 \implies D_2^\dagger \approx \frac{AD_2}{\kappa_2^\dagger}$$

$$\dot{D}_2^b \approx 0 \implies a_2^b D_2 P - (d_2^b + k_2)D_2^b \approx 0 \implies D_2^b \approx \frac{PD_2}{\kappa_0}$$

$$(2)$$

$$\dot{Z}_1^* \approx 0 \implies a_1' Z_1 R - (d_1' + k_1')Z_1^* \approx 0 \implies Z_1^* \approx \frac{Z_1 R}{\kappa_1'}$$

$$\dot{Z}_2^* \approx 0 \implies a_2' Z_2 R - (d_2' + k_2')Z_2^* \approx 0 \implies Z_2^* \approx \frac{Z_2 R}{\kappa_2'}$$

$$\dot{X}_1^* \approx 0 \implies a_3 X_1 G - (d_3 + k_3)X_1^* \approx 0 \implies X_1^* \approx \frac{X_1 G}{\kappa_3}$$

$$\dot{A} \approx 0 \implies a' X_2^2 - d' A \approx 0 \implies A \approx \frac{X_2^2}{\kappa'},$$

where the various dissociation constants ($\kappa_1$, $\kappa_2$, $\kappa_3$, $\kappa_1'$, $\kappa_2'$, $\kappa'$, $\kappa_2^\dagger$, and $\kappa_0$) are all given in Fig. 28.

By substituting the quasi steady-state approximations of $D_1^*$, $D_2^*$, $D_2^\dagger$ and $D_2^b$ in the conservation laws $D_1 + D_1^* = D_1^T$ and $D_2 + D_2^\dagger + D_2^* + D_2^b = D_2^T$, the following expressions are obtained:

$$D_1 \approx D_1^T \frac{1}{1 + \frac{P}{\kappa_1}}, \qquad D_1^* \approx D_1^T \frac{\frac{P}{\kappa_1}}{1 + \frac{P}{\kappa_1}},$$

$$D_2 \approx D_2^T \frac{1}{1 + \frac{P}{\kappa_0} + \frac{A}{\kappa_2^\dagger}\left(1 + \frac{P}{\kappa_2}\right)}, \quad D_2^* \approx D_2^T \frac{\frac{A}{\kappa_2^\dagger}\frac{P}{\kappa_2}}{1 + \frac{P}{\kappa_0} + \frac{A}{\kappa_2^\dagger}\left(1 + \frac{P}{\kappa_2}\right)},$$

$$D_2^\dagger \approx D_2^T \frac{\frac{A}{\kappa_2^\dagger}}{1 + \frac{P}{\kappa_0} + \frac{A}{\kappa_2^\dagger}\left(1 + \frac{P}{\kappa_2}\right)}, \quad D_2^b \approx D_2^T \frac{\frac{P}{\kappa_0}}{1 + \frac{P}{\kappa_0} + \frac{A}{\kappa_2^\dagger}\left(1 + \frac{P}{\kappa_2}\right)}.$$

Similarly, by substituting the quasi steady-state approximations of $Z_1^*$, $Z_2^*$ and $X_1^*$ in the conservation laws $R + Z_1^* + Z_2^* = R^T$ and $G + X_1^* = G^T$, we obtain

$$R \approx R^T \frac{1}{1 + \frac{Z_1}{\kappa_1'} + \frac{Z_2}{\kappa_2'}}, \quad Z_1^* \approx R^T \frac{\frac{Z_1}{\kappa_1'}}{1 + \frac{Z_1}{\kappa_1'} + \frac{Z_2}{\kappa_2'}}, \quad Z_2^* \approx R^T \frac{\frac{Z_2}{\kappa_2'}}{1 + \frac{Z_1}{\kappa_1'} + \frac{Z_2}{\kappa_2'}};$$

$$G \approx G^{\mathrm{T}} \frac{1}{1 + \frac{X_1}{\kappa_3}}, \qquad X_1^* \approx G^{\mathrm{T}} \frac{\frac{X_1}{\kappa_3}}{1 + \frac{X_1}{\kappa_3}}.$$

The only remaining conservation law is that of the RNA Polymerase given by $P + D_1^* + D_2^* + D_2^b = P^{\mathrm{T}}$.

By substituting the quasi steady-state approximations of $D_1^*$, $D_2^*$ and $A$, the following algebraic equation is obtained

$$P + D_1^{\mathrm{T}} \frac{\frac{P}{\kappa_1}}{1 + \frac{P}{\kappa_1}} + D_2^{\mathrm{T}} \frac{\frac{A}{\kappa_2^{\dagger}} \frac{P}{\kappa_2}}{1 + \frac{P}{\kappa_0} + \frac{A}{\kappa_2^{\dagger}}\left(1 + \frac{P}{\kappa_2}\right)} + D_2^{\mathrm{T}} \frac{\frac{P}{\kappa_0}}{1 + \frac{P}{\kappa_0} + \frac{A}{\kappa_2^{\dagger}}\left(1 + \frac{P}{\kappa_2}\right)} = P^{\mathrm{T}}, \qquad (3)$$

,

where $A = \frac{X_2^2}{\kappa'}$.   One would hope to write P as a function of $X_2$. However, since this is a cubic polynomial in P, the closed-form solution is tedious to write down explicitly. Thus, the equation is left implicit in P and $X_2$.

Equipped with the quasi steady-state approximations, a set of *Differential Algebraic Equations (DAEs)* can be written down that describe the evolution of $X_1$, $X_2$, $Z_1$, $Z_2$, P and Y.

$$\dot{X}_1 = k_1' Z_1^* - a_3 X_1 G + d_3 X_1^* - c X_1 \approx k_1' Z_1^* - k_3 X_1^* - c X_1$$

$$\approx k_1' R^{\mathrm{T}} \frac{\frac{Z_1}{\kappa_1^{\dagger}}}{1 + \frac{Z_1}{\kappa_1'} + \frac{Z_2}{\kappa_2'}} - \left( c X_1 + k_3 G^{\mathrm{T}} \frac{\frac{X_1}{\kappa_3}}{1 + \frac{X_1}{\kappa_3}} \right)$$

$$\dot{X}_2 = c X_1 - \gamma_x X_2 - 2\left( a X_2^2 - d A \right) \approx c X_1 - \gamma_x X_2$$

$$\dot{Z}_1 = k_1 D_1^* - \left( a_1' Z_1 R - (d_1' + k_1') Z_1^* \right) - \eta Z_1 Z_2 - \delta Z_1 \approx k_1 D_1^{\mathrm{T}} \frac{\frac{P}{\kappa_1}}{1 + \frac{P}{\kappa_1}} - \eta Z_1 Z_2 - \delta Z_1$$

$$\dot{Z}_2 = k_2 D_2^* + k_2^b D_2^b - \left( a_2' Z_2 R - (d_2' + k_2') Z_2^* \right) - \eta Z_1 Z_2 - \delta Z_2$$

$$\approx k_2 D_2^{\mathrm{T}} \frac{\frac{X_2^2}{\kappa' \kappa_2^{\dagger}} \frac{P}{\kappa_2} + \frac{P}{\kappa_0}}{1 + \frac{P}{\kappa_0} + \frac{X_2^2}{\kappa' \kappa_2^{\dagger}}\left(1 + \frac{P}{\kappa_2}\right)} - \eta Z_1 Z_2 - \delta Z_2$$

$$P + D_1^{\mathrm{T}} \frac{\frac{P}{\kappa_1}}{1 + \frac{P}{\kappa_1}} + D_2^{\mathrm{T}} \frac{\frac{X_2^2}{\kappa' \kappa_2^{\dagger}} \frac{P}{\kappa_2} + \frac{P}{\kappa_0}}{1 + \frac{P}{\kappa_0} + \frac{X_2^2}{\kappa' \kappa_2^{\dagger}}\left(1 + \frac{P}{\kappa_2}\right)} = P^{\mathrm{T}},$$

$$\dot{Y} = k_2' Z_2^* - \gamma_y Y \approx k_2' R^{\mathrm{T}} \frac{\frac{Z_2}{\kappa_2'}}{1 + \frac{Z_1}{\kappa_1'} + \frac{Z_2}{\kappa_2'}} - \gamma_y Y.$$

Equipped with the quasi steady-state approximations, a set of *Differential Algebraic Equations (DAEs)* can be written down that describe the evolution of $X_1$, $X_2$, $Z_1$, $Z_2$, P and Y.

$$\dot{X}_1 = k_1' Z_1^* - a_3 X_1 G + d_3 X_1^* - c X_1 \approx k_1' Z_1^* - k_3 X_1^* - c X_1$$

$$\approx k_1' R^{\mathrm{T}} \frac{\frac{Z_1}{\kappa_1'}}{1 + \frac{Z_1}{\kappa_1'} + \frac{Z_2}{\kappa_2'}} - \left( c X_1 + k_3 G^{\mathrm{T}} \frac{\frac{X_1}{\kappa_3}}{1 + \frac{X_1}{\kappa_3}} \right)$$

$$\dot{X}_2 = c X_1 - \gamma_x X_2 - 2 \left( a X_2^2 - d A \right) \approx c X_1 - \gamma_x X_2$$

$$\dot{Z}_1 = k_1 D_1^* - \left( a_1' Z_1 R - (d_1' + k_1') Z_1^* \right) - \eta Z_1 Z_2 - \delta Z_1 \approx k_1 D_1^{\mathrm{T}} \frac{\frac{P}{\kappa_1}}{1 + \frac{P}{\kappa_1}} - \eta Z_1 Z_2 - \delta Z_1$$

$$\dot{Z}_2 = k_2 D_2^* + k_2^b D_2^b - \left( a_2' Z_2 R - (d_2' + k_2') Z_2^* \right) - \eta Z_1 Z_2 - \delta Z_2$$

$$\approx k_2 D_2^{\mathrm{T}} \frac{\frac{X_2^2}{\kappa' \kappa_2^\dagger} \frac{P}{\kappa_2} + \frac{P}{\kappa_0}}{1 + \frac{P}{\kappa_0} + \frac{X_2^2}{\kappa' \kappa_2^\dagger} \left( 1 + \frac{P}{\kappa_2} \right)} - \eta Z_1 Z_2 - \delta Z_2$$

$$P + D_1^{\mathrm{T}} \frac{\frac{P}{\kappa_1}}{1 + \frac{P}{\kappa_1}} + D_2^{\mathrm{T}} \frac{\frac{X_2^2}{\kappa' \kappa_2^\dagger} \frac{P}{\kappa_2} + \frac{P}{\kappa_0}}{1 + \frac{P}{\kappa_0} + \frac{X_2^2}{\kappa' \kappa_2^\dagger} \left( 1 + \frac{P}{\kappa_2} \right)} = P^{\mathrm{T}},$$

$$\dot{Y} = k_2' Z_2^* - \gamma_y Y \approx k_2' R^{\mathrm{T}} \frac{\frac{Z_2}{\kappa_2'}}{1 + \frac{Z_1}{\kappa_1'} + \frac{Z_2}{\kappa_2'}} - \gamma_y Y.$$

This set of DAEs can be compactly rewritten as

$$\dot{X}_1 = k(Z_1, R) - \left( c X_1 + \gamma(X_1; G^{\mathrm{T}}) \right)$$

$$\dot{X}_2 = c X_1 - \gamma_x X_2$$

$$\dot{Z}_1 = \mu(P; D_1^{\mathrm{T}}) - \eta Z_1 Z_2 - \delta Z_1$$

$$\dot{Z}_2 = \theta(X_2; P, D_2^{\mathrm{T}}) - \eta Z_1 Z_2 - \delta Z_2$$

$$P + \frac{\mu(P; D_1^{\mathrm{T}})}{k_1} + \frac{\theta(X_2; P, D_2^{\mathrm{T}})}{k_2} = P^{\mathrm{T}}$$

$$\dot{Y} = v(Z_2, R) - \gamma_y Y.$$

where

$$\mu(P; D_1^T) = k_1 D_1^{\mathrm{T}} \frac{\frac{P}{\kappa_1}}{1 + \frac{P}{\kappa_1}}, \quad \theta(X_2, P; D_2^T) = k_2 D_2^T \frac{\frac{X_2^2}{\kappa'\kappa_2^\dagger}\frac{P}{\kappa_2} + \frac{P}{\kappa_0}}{1 + \frac{P}{\kappa_0} + \frac{X_2^2}{\kappa'\kappa_2^\dagger}\left(1 + \frac{P}{\kappa_2}\right)},$$

$$k(Z_1, R) = k_1' R \frac{Z_1}{\kappa_1'}, \qquad \gamma(X_1; G^{\mathrm{T}}) = k_3 G^{\mathrm{T}} \frac{\frac{X_1}{\kappa_3}}{1 + \frac{X_1}{\kappa_3}},$$

$$v(Z_2, R) = k_2' R \frac{Z_2}{\kappa_2'}, \qquad R = R^{\mathrm{T}} \frac{1}{1 + \frac{Z_1}{\kappa_1'} + \frac{Z_2}{\kappa_2'}}.$$

One final approximation can also be carried out by invoking Assumptions 2 and 3, that is X1 $<<\_\kappa_3$ and $\dot{X}_1 \approx$ 0. We have

$$\gamma(X_1; G^{\mathrm{T}}) \approx \frac{k_3 G^{\mathrm{T}}}{\kappa_3} X_1 \qquad \text{and} \qquad k(Z_1, R) - \left(c + \frac{k_3 G^{\mathrm{T}}}{\kappa_3}\right) X_1 \approx 0$$

$$cX_1 := \frac{k(Z_1, R)}{1 + \frac{k_3}{\kappa_{3c}} G^{\mathrm{T}}}$$

As a result, we can get rid of                               in the differential equation of $\dot{X}_2$ to obtain the following DAEs

$$\dot{X}_2 = k(Z_1, R; G^{\mathrm{T}}) - \gamma_x X_2$$

$$\dot{Z}_1 = \mu(P; D_1^{\mathrm{T}}) - \eta Z_1 Z_2 - \delta Z_1$$

$$\dot{Z}_2 = \theta(X_2; P, D_2^{\mathrm{T}}) - \eta Z_1 Z_2 - \delta Z_2$$

$$P + \frac{\mu(P; D_1^{\mathrm{T}})}{k_1} + \frac{\theta(X_2; P, D_2^{\mathrm{T}})}{k_2} = P^{\mathrm{T}}$$

$$\dot{Y} = v(Z_2, R) - \gamma_y Y,$$

where, with slight abuse of notation, the definition of the function k is modified to incorporate the drug influence as

$$k(Z_1, R; G^{\mathrm{T}}) := k_1' R \frac{Z_1}{\kappa_1'} \frac{1}{1 + \frac{k_3}{\kappa_{3c}} G^{\mathrm{T}}}$$

Finally, $\theta(X_2, P; D_2^{\mathrm{T}})$ can be rewritten in a more convenient form as

$$\theta(X_2, P; D_2^{\mathrm{T}}) = k_2 D_2^{\mathrm{T}} \left( \alpha_0(P) + \alpha(P) \frac{X_2^n / \kappa^n(P)}{1 + X_2^n / \kappa^n(P)} \right),$$

where:

$$\begin{cases} \alpha_0(P) := \dfrac{P/\kappa_0}{1 + P/\kappa_0}, & \alpha_2(P) := \dfrac{P/\kappa_2}{1 + P/\kappa_2}, \\ \alpha(P) := \alpha_2(P) - \alpha_0(P), & \kappa^n(P) := \kappa' \kappa_2^\dagger \dfrac{1 + P/\kappa_0}{1 + P/\kappa_2}, \end{cases}$$

and n = 2 is the hill coefficient. Note that the dissociation constant corresponding to the basal expression is larger than that corresponding to the expression in the presence of the activator, i.e. $\kappa_0 > \kappa_2$, and thus $\alpha(P) > 0$ for any P > 0.

The reduced model is shown in Fig. 26A.

*Model fitting*

In this section, we show that a burden-free model is not sufficient to fit the data shown in Fig. 26B. The burden-free model in the open-loop setting ($\eta = 0$) is described by the following set of ODEs.

$$\dot{X}_2 = k_0(G^{\mathrm{T}}) Z_1 / \kappa_1' - \gamma_x X_2$$

$$\dot{Z}_1 = k_1 D_1^{\mathrm{T}} \alpha_1 - \delta Z_1$$

$$\dot{Z}_2 = k_2 D_2^{\mathrm{T}} \left[ \alpha_0 + \alpha \frac{(X_2/\kappa)^2}{1 + (X_2/\kappa)^2} \right] - \delta Z_2$$

$$\dot{Y} = k_2' R^{\mathrm{T}} Z_2 / \kappa_2' - \gamma_y Y \tag{4}$$

$$k_0(G^{\mathrm{T}}) := \frac{k_1' R^{\mathrm{T}}}{1 + \frac{k_3}{\kappa_{3c}} G^{\mathrm{T}}},$$

where: $\alpha_0(P^T)$, $\alpha_1 := \alpha_1(P^T)$, , $\alpha := \alpha(P^T)$, $\kappa := \kappa(P^T)$.

The fixed point $(\overline{X}_2, \overline{Z}_1, \overline{Z}_2, \overline{Y})$ of the open-loop dynamics is calculated by setting the time derivatives to zero to obtain

$$\bar{X}_2 = \frac{k_0(G^{\mathrm{T}})k_1\alpha_1}{\gamma_x\delta\kappa'_1}D_1^{\mathrm{T}}$$

$$\bar{Z}_1 = \frac{k_1 D_1^{\mathrm{T}}\alpha_1}{\delta}$$

$$\bar{Z}_2 = \frac{k_2 D_2^{\mathrm{T}}}{\delta}\left[\alpha_0 + \alpha\frac{(\bar{X}_2/\kappa)^2}{1+(\bar{X}_2/\kappa)^2}\right] \tag{5}$$

$$\bar{Y} = \frac{k'_2 k_2 R^{\mathrm{T}} D_2^{\mathrm{T}}}{\gamma_y\delta\kappa'_2}\left[\alpha_0 + \alpha\frac{(\bar{X}_2/\kappa)^2}{1+(\bar{X}_2/\kappa)^2}\right].$$

The green and red fluorescence measured in the experiments, denoted by $M_G$ and $M_R$ respectively, are given by

$$M_G := c_G(\bar{X}_2 + \bar{A}) = c_G\left(\bar{X}_2 + \frac{\bar{X}_2^2}{\kappa'}\right)$$

$$M_R := c_R\bar{Y},$$

where $c_G$ and $c_R$ are proportionality constants that map concentrations to green and red fluorescence, respectively. Note that A represents the dimerized version of $X_2$ that acts as a transcription factor and is also green fluorescent. It is shown that its concentration at steady state is given by $\overline{A = \bar{X}_2^2/\kappa'}$ (refer to section "Reduced model" for a detailed explanation). Observe that $M_G$ is quadratically increasing in $D_1^T$ (since $\bar{X}_2$ is linearly increasing in $D_1^T$ ). Furthermore, observe that $M_R$ is a monotonically increasing hill function of $\bar{X}_2$ and thus $D_1^T$. These two observations present a contradiction with the data shown in Fig. 26B, since the green fluorescence saturates for high $D_1^T$, and the red fluorescence starts decreasing at high $D_1^T$. As a result, a burden-free model cannot capture these two behaviors.

*Example 2: Mathematical description of PI, PD and PID Molecular Controllers*

The process we wish to control has L dynamically interacting species whose concentrations are given by: $X_1$, ..., $X_L$. Here $X_1$ is assumed to be the concentration of the actuated species (process input), and $X_L$ the concentration of regulated species (process output). The molecular controller is assumed to have n species whose concentrations are given by $Z_1$, ... , $Z_n$. The way we control the process is through influencing $X_1$ (see Fig. above). In particular

$$\dot{X}_1 = f_1(X_1,\ldots,X_L) + \underbrace{U(Z_1,\ldots,Z_n,X_1,X_L)}_{control}$$

The function $U$ can depend on $X_L$ to allow feedback, and can depend on the actuated species, to allow creation or elimination of the actuated species in a way that depends on its concentration.
The variables participating in the control are indicated through arrows or T-lines. In Fig. 29-33, for example, an arrow indicates an increase in the rate of creation of $X_1$ as a function of the variable associated with the arrow. This could be achieved through various means, e.g. increasing its expression or activation, decreasing its degradation or inhibition of $X_1$, etc. On the other hand a line that ends with a T indicates a decrease in the rate of creation of $X_1$ as a function of the variable associated with the T-line, which could be achieved through opposite processes, e.g. decreased expression, decreased activation, increased inhibition, increased degradation, etc. In the example shown,

$$U = U(Z_1, Z_2, X_1, X_L),$$

and near the operating point $U$ is an increasing function of $Z_1$ and $Z_2$ and a decreasing function of $X_L$. For linear analysis, and without loss of generality, one could simply assume a $U$ of the following form:

$$U = h_0(X_L; X_1) + h_1(Z_1; X_1) + h_2(Z_2; X_1)$$

where $h_0$ and $h_1$ are monotonically increasing functions of their arguments (consistent with arrows) and $h_2$ is monotonically decreasing (consistent with the T-line). Indeed, at a given fixed point, the linearization of both expressions of $U$ above have the same form. For the analysis we carry out next, the dependence of $U$ on $X_1$ will be suppressed to simplify the exposition. In other words, we will take

$$U = h_0(X_L) + h_1(Z_1) + h_2(Z_2)$$

No loss of generality is incurred by suppressing the possible dependence on $X_1$, and the analysis can be easily carried out similarly whenever our $U$ implementation (e.g. activation/inhibition/expression/degradation of the actuating species) depends on the actuation species concentration, $X_1$.

*1. PI controllers*

There are two implementation types to be considered: N-type and P-type. N-type controllers are suitable for positive processes, which P-type controllers are suitable for negative processes. This ensures the overall control loop implements negative feedback.

*1.1 Second Order Implementations of PI Controllers*

*1.1.1 Processes with negative gain*

These processes require P-type controllers for stability. The process is described as follows

$$\begin{aligned}
\dot{X}_1 &= f_1(X_1, \ldots, X_L) + U \\
\dot{X}_2 &= f_2(X_1, \ldots, X_L) \\
&\vdots \\
\dot{X}_L &= f_L(X_1, \ldots, X_L)
\end{aligned}$$

Given a desired setpoint $X_L = X_L^*$, we assume there exists a corresponding nonzero fixed point ( $X_1^*$, ..., $X_L^*$, $U^*$).

The P-type PI controller dynamics are as follows (see Fig. 29, right panel):

$$\dot{Z}_1 = \mu - \eta Z_1 Z_2$$

$$\dot{Z}_2 = \theta X_L - \eta Z_1 Z_2$$

$$U = h_2(Z_2) + h_0(X_L)$$

We will take $h_0$ and $h_2$ to be monotonically increasing.

**Lemma:** A necessary and sufficient condition for the closed-loop to have a non-negative fixed point $(Z_1^*, Z_2^*, X_1^*, \ldots, X_L^*)$ is

$$h_2(0) < U^* - h_0(\mu/\theta) < h_2(\infty)$$

Linearizing the dynamics at this fixed point we have:

$$\dot{z}_1 \;=\; -\eta Z_2^* z_1 - \eta Z_1^* z_2$$

$$\dot{z}_2 \;=\; \theta x_L - \eta Z_2^* z_1 - \eta Z_1^* z_2$$

$$\dot{x}_1 \;=\; \underbrace{h_2'(*)z_2 + h_0'(*)x_L}_{u} + \nabla^T f_1(*)x$$

where $h_0'(*)$ and $h_2'(*)$ are the derivatives of $h_0$ and $h_2$, respectively, evaluated at the fixed point.

Let $u := h_2'(*)z_2 + h_0'(*)x_L.$ The transfer function from $x_L$ to u is given by

$$\hat{u}(s)/\hat{x}_L(s) \;=\; \begin{bmatrix} 0 & h_2'(*) \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} 0 \\ \theta \end{bmatrix} + h_0'(*)$$

$$=\; \theta h_2'(*) \cdot \frac{s + \eta Z_2^*}{s(s + \eta(Z_1^* + Z_2^*))} + h_0'(*)$$

$$\approx\; \frac{\theta h_2'(*)}{s} + h_0'(*) \qquad (\eta \gg 1)$$

$$=\; \left( K_P + \frac{K_I}{s} \right)$$

*1.1.2 Processes with positive gain*

These processes require N-type controllers for stability. The process is described as follows

$$\dot{X}_1 \;=\; f_1(X_1, \ldots, X_L) + U$$
$$\dot{X}_2 \;=\; f_2(X_1, \ldots, X_L)$$
$$\vdots$$
$$\dot{X}_L \;=\; f_L(X_1, \ldots, X_L)$$

Given a desired setpoint $X_L = X_L^*$, we assume there exists a corresponding nonzero fixed point ( $X_1^*$, ..., $X_L^*$, $U^*$).

The N-type PI controller dynamics are as follows (see Fig. 29, left panel):

$$\dot{Z}_1 = \mu - \eta Z_1 Z_2$$

$$\dot{Z}_2 = \theta X_L - \eta Z_1 Z_2$$

$$U = h_1(Z_1) + h_0(X_L)$$

We will take $h_0$ to be monotonically decreasing and $h_1$ to be monotonically increasing.

**Lemma:** A necessary and sufficient condition for the closed-loop to have a non-negative fixed point $(Z_1^*, Z_2^*, X_1^*, \ldots, X_L^*)$ is

$$h_1(0) < U^* - h_0(\mu/\theta) < h_1(\infty)$$

Linearizing the dynamics at this fixed point we have:

$$\dot{z}_1 = -\eta Z_2^* z_1 - \eta Z_1^* z_2$$

$$\dot{z}_2 = \theta x_L - \eta Z_2^* z_1 - \eta Z_1^* z_2$$

$$\dot{x}_1 = \underbrace{h_1'(*)z_1 + h_0'(*)x_L}_{u} + \nabla^T f_1(*) x$$

where $h_0'(*)$ and $h_1'(*)$ are the derivatives of $h_0$ and $h_1$, respectively, evaluated at the fixed point. Let $u := h_2'(*)z_2 + h_0'(*)x_L$. The transfer function from $x_L$ to $u$ is given by

$$\hat{u}(s)/\hat{x}_L(s) = \begin{bmatrix} h'_1(*) & 0 \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} 0 \\ \theta \end{bmatrix} + h'_0(*)$$

$$= \theta h'_1(*) . \frac{-\eta Z_1^*}{s(s+\eta(Z_1^*+Z_2^*))} + h'_0(*)$$

$$\approx \frac{-\theta h'_2(*)}{s} \frac{\eta Z_1^*}{s+\eta Z_1^*} + h'_0(*) \qquad (\eta \gg 1)$$

$$\approx \left( K_P + \frac{K_I}{s} \right) \qquad (\eta Z_1^* \gg 1)$$

Note: This controller is a pure proportional with a *filtered* integral. However, the filter cutoff-frequency is high for large $\eta Z_1^*$, so the filter can be neglected in this case.

*2. PD controllers*

*2.1 Negative gain processes*

These processes are described as follows (Fig. 30, right panel):

$$\dot{X}_1 = f_1(X_1, \dots, X_L) + U$$
$$\dot{X}_2 = f_2(X_1, \dots, X_L)$$
$$\vdots$$
$$\dot{X}_L = f_L(X_1, \dots, X_L)$$

We assume there exists a nonzero fixed point ( $X_1^*$, ... , $X_L^*$, U*).
The P-type PD controller dynamics are as follows (see Fig. 30, right panel):

$$\dot{Z} = \mu + g_0(X_L) - \gamma_z Z$$

$$U = h(Z) + h_0(X_L)$$

We assume $g_0$ is monotonically decreasing or increasing (depending on the desired PD parameters) while $h_0$ and *h* are monotonically increasing.
The linearized dynamics

$$\dot{z} = g'_0(*)x_L - \gamma_z z$$

$$\dot{u} = h'(*)z + h'_0(*)x_L$$

It follows that

$$\hat{u}(s)/\hat{x}_L(s) = \frac{h'(*)g_0'(*)}{s+\gamma_z} + h_0'(*)$$

$$= \frac{h_0'(*)s + h_0'(*)\gamma_z + h'(*)g_0'(*)}{s+\gamma_z}$$

$$= \frac{K_D s + K_p}{s+\gamma_z}$$

*2.2 Positive gain processes*

These processes are described as follows (Fig. 30, left panel):

$$\dot{X}_1 = f_1(X_1, \ldots, X_L) + U$$
$$\dot{X}_2 = f_2(X_1, \ldots, X_L)$$
$$\vdots$$
$$\dot{X}_L = f_L(X_1, \ldots, X_L)$$

We assume there exists a nonzero fixed point ( $X_1^*$, ..., $X_L^*$, $U^*$).

The N-type PD controller dynamics are as follows (see Fig. 30, left panel):

$$\dot{Z} = \mu + g_0(X_L) - \gamma_z Z$$

$$U = h(Z) + h_0(X_L)$$

We assume $g_0$ is monotonically decreasing or increasing (depending on the desired PD parameters) while $h_0$ and $h$ are monotonically decreasing.

The linearized dynamics are as follows:

$$\hat{u}(s)/\hat{x}_L(s) = \frac{h'(*)g_0'(*)}{s+\gamma_z} + h_0'(*)$$

$$= \frac{h_0'(*)s + h_0'(*)\gamma_z + h'(*)g_0'(*)}{s+\gamma_z}$$

$$= -\frac{K_D s + K_p}{s+\gamma_z}$$

*3. PID controllers*

We present three implementations, one is second order requiring two species, another is a 3rd order implementation requiring three species, and the last is a 4th order implementation requiring 4 species. The second order controller implementation is simpler, but it covers only a subset of all PID controllers, while the third order implementation for all practical purposes covers all possible PID controller parameters with filtered PD components. The 4th order implementation is the most general, and covers all PID controllers with a filtered D component. It is the one most closely matches PID industrial controllers.

*3.1 Second-order PID implementations*

*3.1.1 Processes with negative gain*

Negative gain process are those with a decreasing dose response. These processes require P-type controllers for stability. We assume the process is described as

$$
\begin{aligned}
\dot{X}_1 &= f_1(X_1, \ldots, X_L) + U \\
\dot{X}_2 &= f_2(X_1, \ldots, X_L) \\
&\vdots \\
\dot{X}_L &= f_L(X_1, \ldots, X_L)
\end{aligned}
$$

Given a desired setpoint $X_L = X_L^*$, we assume there exists a corresponding nonzero fixed point ($X_1^*$, ..., $X_L^*$, $U^*$).

The P-type PID controller dynamics are as follows (see Fig. 31, right panel):

$$
\dot{Z}_1 = (1 - \alpha)\mu + \alpha\theta X_L - \eta Z_1 Z_2
$$

$$
\dot{Z}_2 = \theta X_L - \eta Z_1 Z_2
$$

$$
U = h_0(X_L) + h_2(Z_2)
$$

We will take $h_0$ and $h_2$ to be monotonically increasing.

Lemma: A necessary and sufficient condition for the closed-loop to have a non-negative fixed point ($Z_1^*$, $Z_2^*$, $X_1^*$, ..., $X_L^*$) is

$$
h_2(0) < U^* - h_0(\mu/\theta) < h_2(\infty)
$$

Linearizing the dynamics at this fixed point we have:

$$
\dot{z}_1 = \alpha\theta x_L - \eta Z_2^* z_1 - \eta Z_1^* z_2
$$

$$
\dot{z}_2 = \theta x_L - \eta Z_2^* z_1 - \eta Z_1^* z_2
$$

$$
\dot{x}_1 = \underbrace{h_2'(*)z_2 + h_0'(*)x_L}_{u} + \nabla^T f_1(*)x
$$

where $h'_0(*)$ and $h'_2(*)$ are the derivatives of $h_0$ and $h_2$, respectively, evaluated at the fixed point. Let

$u := h'_2(*)z_2 + h'_0(*)x_L.$ The transfer function from $x_L$ to $u$ is given by

$$
\begin{aligned}
\hat{u}(s)/\hat{x}_L(s) &= \begin{bmatrix} 0 & h'_2(*) \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} \alpha\theta \\ \theta \end{bmatrix} + h'_0(*) \\
&= \theta h'_2(*).\frac{s+(1-\alpha)\eta Z_2^*}{s(s+\eta(Z_1^*+Z_2^*))} + h'_0(*) \\
&= \frac{h'_0(*)s^2 + (\theta h'_2(*)+h'_0(*)\eta(Z_1^*+Z_2^*))s + \theta h'_2(*)(1-\alpha)\eta Z_2^*}{s(s+\eta(Z_1^*+Z_2^*))} \\
&= \left(K_D s + K_P + \frac{K_I}{s}\right)\frac{1}{(s+\eta(Z_1^*+Z_2^*))}
\end{aligned}
$$

*3.1.2 Processes with positive gain*

Positive gain processes are those with increasing dose response. These processes require N-type controllers for stability. We assume the process is described as

$$
\begin{aligned}
\dot{X}_1 &= f_1(X_1,\ldots,X_L)+U \\
\dot{X}_2 &= f_2(X_1,\ldots,X_L) \\
&\vdots \\
\dot{X}_L &= f_L(X_1,\ldots,X_L)
\end{aligned}
$$

Given a desired setpoint $X_L = X_L^*$, we assume there exists a corresponding nonzero fixed point ($X_1^*$, ... , $X_L^*$, $U^*$).

The N-type PID controller dynamics are as follows (see Fig. 31, left panel):

$$
\dot{Z}_1 = (1-\alpha)\mu + \alpha\theta X_L - \eta Z_1 Z_2
$$

$$
\dot{Z}_2 = \theta X_L - \eta Z_1 Z_2
$$

$$
U = h_0(X_L) + h_1(Z_1)
$$

We will take $h_0$ to be monotonically decreasing and $h_1$ to be monotonically increasing.

**Lemma:** A necessary and sufficient condition for the closed-loop to have a non-negative fixed point ($Z_1^*$, $Z_2^*$, $X_1^*$, ... , $X_L^*$) is

$$h_1(0) < U^* - h_0(\mu/\theta) < h_1(\infty)$$

Linearizing the dynamics at this fixed point we have:

$$\dot{z}_1 = \alpha\theta x_L - \eta Z_2^* z_1 - \eta Z_1^* z_2$$

$$\dot{z}_2 = \theta x_L - \eta Z_2^* z_1 - \eta Z_1^* z_2$$

$$\dot{x}_1 = \underbrace{h_1'(*)z_1 + h_0'(*)x_L}_{u} + \nabla^T f_1(*)x$$

where $h_0'(*)$ and $h_1'(*)$ are the derivatives of $h_0$ and $h_1$, respectively, evaluated at the fixed point.

Let $u := h_1'(*)z_1 + h_0'(*)x_L.$ The transfer function from $x_L$ to $u$ is given by

$$
\begin{aligned}
\hat{u}(s)/\hat{x}_L(s) &= \begin{bmatrix} h_1'(*) & 0 \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} \alpha\theta \\ \theta \end{bmatrix} + h_0'(*) \\
&= \theta h_1'(*).\frac{\alpha s - (1-\alpha)\eta Z_2^*}{s(s+\eta(Z_1^*+Z_2^*))} + h_0'(*) \\
&= \frac{h_0'(*)s^2 + (\alpha\theta h_1'(*) + h_0'(*)\eta(Z_1^*+Z_2^*))s - \theta h_1'(*)(1-\alpha)\eta Z_2^*}{s(s+\eta(Z_1^*+Z_2^*))} \\
&= -\left(K_D s + K_P + \frac{K_I}{s}\right)\frac{1}{(s+\eta(Z_1^*+Z_2^*))}
\end{aligned}
$$

where $K_D = -h_0'(*) > 0, K_P = -\alpha\theta h_1'(*) - h_0'(*)\eta(Z_1^*+Z_2^*) > 0$ (when $\alpha$ is chosen to be sufficiently small),
and $K_I = \theta h_1'(*)(1-\alpha)\eta Z_2^* > 0.$

*3.2 Third-order PID implementations*

*3.2.1 Processes with negative gain*

These processes usually require P-type controllers for stability. We assume the process is described as

$$
\begin{aligned}
\dot{X}_1 &= f_1(X_1,\ldots,X_L) + U \\
\dot{X}_2 &= f_2(X_1,\ldots,X_L) \\
&\vdots \\
\dot{X}_L &= f_L(X_1,\ldots,X_L)
\end{aligned}
$$

Given a desired setpoint $X_L = X_L^*$, we assume there exists a corresponding nonzero fixed point ( $X_1^*$, ... , $X_L^*$; $U^*$). The p-type PID controller dynamics are as follows (see Fig. 32, right panel):

$$\dot{Z}_1 = \mu - \eta Z_1 Z_2$$

$$\dot{Z}_2 = \theta X_L - \eta Z_1 Z_2$$

$$\dot{Z}_3 = g_0(X_L) - \gamma Z_3$$

$$U = h_0(X_L) + h_2(Z_2) + h_3(Z_3)$$

We will take $h_2$ to be monotonically increasing.

Lemma: A necessary and sufficient condition for the closed-loop to have a non-negative fixed point ( $Z_1^*$, $Z_2^*$, $Z_3^*$, $X_1^*$, ... , $X_L^*$ ). is

$$h_2(0) < \left[ U^* - h_0(\mu/\theta) - h_3\left(\frac{g_0(\mu/\theta)}{\gamma}\right) \right] < h_2(\infty)$$

Linearizing the dynamics at this fixed point we have:

$$\dot{z}_1 = -\eta Z_2^* z_1 - \eta Z_1^* z_2$$

$$\dot{z}_2 = \theta x_L - \eta Z_2^* z_1 - \eta Z_1^* z_2$$

$$\dot{z}_3 = -\gamma z_3 + g_0'(*) x_L$$

$$\dot{x}_1 = \underbrace{h_2'(*) z_2 + h_3'(*) z_3 + h_0'(*) x_L}_{u} + \nabla^T f_1(*) x$$

where $g_0'(*)$, $h_0'(*)$, $h_1'(*)$, and $h_3'(*)$ are the derivatives of $g_0, h_0, h_1,$ and $h_3$ evaluated at the fixed point.

Let $u := h_2'(*) z_2 + h_3'(*) z_3 + h_0'(*) x_L$ +The transfer function from $x_L$ to $u$ is given by

$$\hat{u}(s)/\hat{x}_L(s) = \begin{bmatrix} 0 & h'_2(*) & h'_3(*) \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} & 0 \\ \frac{-\eta Z_2^*}{s(s+\eta(Z_1^*+Z_2^*))} & \frac{s+\eta Z_2^*}{s(s+\eta(Z_1^*+Z_2^*))} & 0 \\ 0 & 0 & \frac{1}{s+\gamma_z} \end{bmatrix} \begin{bmatrix} 0 \\ \theta \\ g'_0(*) \end{bmatrix} + h'_0(*)$$

$$= \theta h'_2(*).\frac{s+\eta Z_2^*}{s(s+\eta(Z_1^*+Z_2^*))} + h'_3(*)g'_0(*)\frac{1}{s+\gamma} + h'_0(*)$$

$$\approx \frac{\theta h'_2(*)}{s} + \frac{h'_0(*)s+\gamma h'_0(*)+h'_3(*)g'_0(*)}{s+\gamma} \qquad (\eta \gg 1)$$

$$= \frac{K_I}{s} + \frac{K_D s + K_P}{s+\gamma}$$

where $h_0$, $h_2$, $h_3$, and $g_0$ were chosen so that and $K_I = \theta h'_2(*), K_D = h'_0(*)$, and $K_P = \gamma h'_0(*) + h'_3(*)g'_0(*)$. There is some flexibility in picking these functions to satisfy these conditions plus the fixed-point existence conditions in the lemma. For example, $h_2(Z_2) = k_2 Z_2$, $h_3(Z_3) =$

$k_3 Z_3$, $h_0(X_L) = \frac{\alpha_h(X_L/\beta_h)^n}{1+(X_L/\beta_h)^n}$, $g_0(X_L) = \frac{\alpha_g(X_L/\beta_g)^m}{1+(X_L/\beta_g)^m}$ (or $g_0(X_L) = \frac{\alpha_g}{1+(X_L/\beta_g)^m}$).

*3.2.2 Processes with positive gain*

These processes usually require P-type controllers for stability. We assume the process is described as

$$\begin{aligned} \dot{X}_1 &= f_1(X_1,\ldots,X_L)+U \\ \dot{X}_2 &= f_2(X_1,\ldots,X_L) \\ &\vdots \\ \dot{X}_L &= f_L(X_1,\ldots,X_L) \end{aligned}$$

Given a desired setpoint $X_L = X_L^*$, we assume there exists a corresponding nonzero fixed point ( $X_1^*$, ... , $X_L^*$; $U^*$).

The n-type PID controller dynamics are as follows (see Fig. 32, left panel):

$$\dot{Z}_1 = \mu - \eta Z_1 Z_2$$

$$\dot{Z}_2 = \theta X_L - \eta Z_1 Z_2$$

$$\dot{Z}_3 = g_0(X_L) - \gamma Z_3$$

$$U = h_0(X_L) + h_1(Z_1) + h_3(Z_3)$$

We will take $h_0$ and $h_3$ to be monotonically decreasing, and $h_1$ to be monotonically increasing.

A necessary and sufficient condition for the closed-loop to have the non-negative fixed point ( $Z_1^*$, $Z_2^*$, $Z_3^*$,

..., $X_L^{*'}$) is that $\mu/\theta = X_L^*$ and

$$h_1(0) < \left[ U^* - h_0(\mu/\theta) - h_3\left(\frac{g_0(\mu/\theta)}{\gamma}\right) \right] < h_1(\infty)$$

Linearizing the dynamics at this fixed-point we have:

$$\dot{z}_1 = -\eta Z_2^* z_1 - \eta Z_1^* z_2$$

$$\dot{z}_2 = \theta x_L - \eta Z_2^* z_1 - \eta Z_1^* z_2$$

$$\dot{z}_3 = -\gamma z_3 + g_0'(*) x_L$$

$$\dot{x}_1 = \underbrace{h_1'(*) z_1 + h_3'(*) z_3 + h_0'(*) x_L}_{u} + \nabla^T f_1(*) x$$

Letting $u := h_1'(*) z_1 + h_3'(*) z_3 + h_0'(*) x_L,$ the transfer function from $x_L$ to $u$ is given by

$$\hat{u}(s)/\hat{x}_L(s) = \begin{bmatrix} h_1'(*) & 0 & h_3'(*) \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} & 0 \\ \frac{-\eta Z_2^*}{s(s+\eta(Z_1^*+Z_2^*))} & \frac{s+\eta Z_2^*}{s(s+\eta(Z_1^*+Z_2^*))} & 0 \\ 0 & 0 & \frac{1}{s+\gamma_z} \end{bmatrix} \begin{bmatrix} 0 \\ \theta \\ g_0'(*) \end{bmatrix} + h_0'(*)$$

$$= \theta h_1'(*) \cdot \frac{-\eta Z_1^*}{s(s+\eta(Z_1^*+Z_2^*))} + h_3'(*) g_0'(*) \frac{1}{s+\gamma} + h_0'(*)$$

$$\approx -\frac{\theta h_1'(*)}{s} + \frac{h_0'(*)s + \gamma h_0'(*) + h_3'(*) g_0'(*)}{s+\gamma} \qquad (\eta \gg 1)$$

$$= -\frac{K_I}{s} - \frac{K_D s + K_P}{s+\gamma}$$

where $h_0$, $h_2$, $h_3$, and $g_0$ were chosen so that $K_I = \theta h_1'(*), K_D = -h_0'(*)$, and $K_P = -\gamma h_0'(*) - h_3'(*) g_0'(*)$.

### 3.3 Fourth-order PID controllers

We present a fourth-order PID controller based on two antithetic motifs. The implementation is that of a PI plus filtered D controller. As the derivative must always be filtered, this is the most general and least restrictive architecture, and it admits all possible PID controller parameters and filter cut-off parameter. This is the most general PID architecture.

*3.3.1 Processes with negative gain*

These processes usually require p-type controllers for stability. We assume the process is described as

$$
\begin{aligned}
\dot{X}_1 &= f_1(X_1, \ldots, X_L) + U \\
\dot{X}_2 &= f_2(X_1, \ldots, X_L) \\
&\vdots \\
\dot{X}_L &= f_L(X_1, \ldots, X_L)
\end{aligned}
$$

Given a desired setpoint $X_L = X_L^*$, we assume there exists a corresponding nonzero fixed point ( $X_1^*$, ... , $X_L^*$, $U^*$).

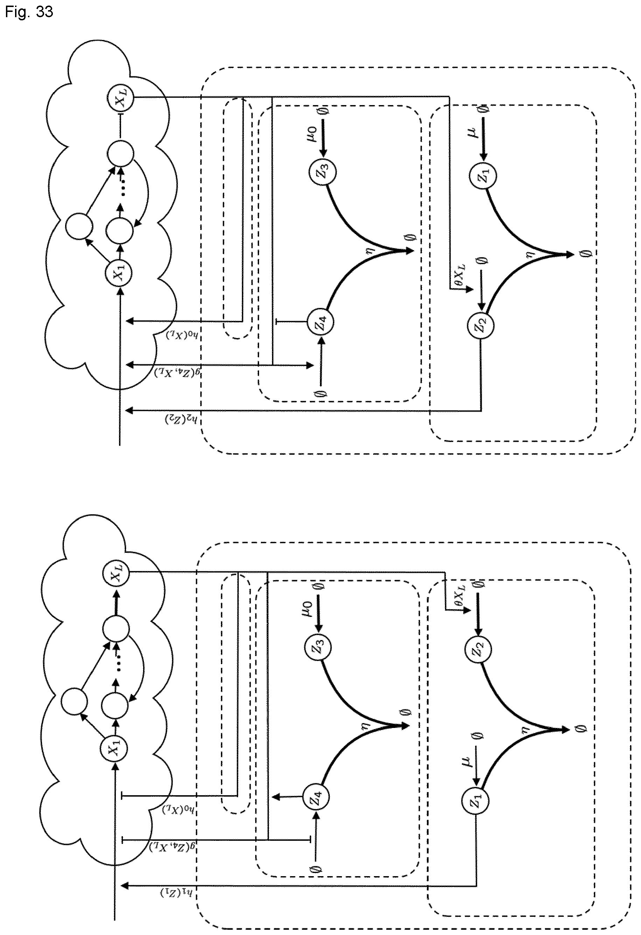

The p-type PID controller dynamics are as follows (see Fig. 33, right panel):

$$
\dot{Z}_1 = \mu - \eta Z_1 Z_2
$$

$$
\dot{Z}_2 = \theta X_L - \eta Z_1 Z_2
$$

$$
\dot{Z}_3 = \mu_0 - \eta Z_3 Z_4
$$

$$
\dot{Z}_4 = g(Z_4, X_L) - \eta Z_3 Z_4
$$

$$
U = \underbrace{h_0(X_L)}_{U_P} + \underbrace{h_2(Z_2)}_{U_I} + \underbrace{g(Z_4, X_L)}_{U_D}
$$

We will take $h_0$ and $h_2$ to be strictly monotonically increasing, and $g(Z_4;X_L)$ to be strictly monotonically increasing in $X_L$ and strictly monotonically decreasing in $Z_4$. For example $g(Z_4, X_L) = \frac{X_L/\beta_1}{1 + Z_4/\beta_2}$, or $g(Z_4, X_L) = \alpha \frac{X_L/\beta_1}{1 + Z_4/\beta_1} \cdot \frac{1}{1 + Z_4/\beta_2}$, or $g(Z_4, X_L) = \frac{\alpha_1 X_L/\beta_1}{1 + X_L/\beta_1} + \frac{\alpha_2}{1 + Z_4/\beta_2}$, etc.

**Lemma 1:** Necessary and sufficient conditions for the closed-loop to have a non-negative fixed point ( $Z_1^*$, ... , $Z_4^*$, $X_1^*$, ... , $X_L^*$ ) are

$$
g(\infty, \mu/\theta) < \mu_0 < g(0, \mu/\theta)
$$

and

$$
h_2(0) < [U^* - h_0(\mu/\theta) - \mu_0] < h_2(\infty)
$$

**Lemma 2:** $Z_2^*$, $Z_4^*$ are independent of $\eta$ and are both positive. $Z_1^*, Z_3^* \to 0$ as $\eta \to \infty$.

Linearizing the dynamics at this fixed point we have:

$$\dot{z}_1 \;=\; -\eta Z_2^* z_1 - \eta Z_1^* z_2$$

$$\dot{z}_2 \;=\; \theta x_L - \eta Z_2^* z_1 - \eta Z_1^* z_2$$

$$\dot{z}_3 \;=\; -\eta Z_4^* z_3 - \eta Z_3^* z_4$$

$$\dot{z}_4 \;=\; \partial_z g(*)z_4 + \partial_x g(*)x_L - \eta Z_4^* z_3 - \eta Z_3^* z_4$$

$$\dot{x}_1 \;=\; \underbrace{h_0'(*)x_L}_{u_P} + \underbrace{h_2'(*)z_2}_{u_I} + \underbrace{\partial_z g(*)z_4 + \partial_x g(*)x_L}_{u_D} + \nabla^T f_1(*)x$$

where $h_0'(*)$, $h_2'(*)$ are the derivatives of $h_0$, $h_2$ evaluated at the fixed point; $\partial_z g$ and $\partial_x g$ are the partial derivatives of g with respect to $Z_4$ and $X_L$, respectively, evaluated at the fixed point.

We next compute the transfer functions from $x_L$ to $u := u_P + u_I + u_D$. The transfer function from $x_L$ to $u_P$ is given by $\hat{u}_P(s)/\hat{x}_L(s) = K_P$, where $K_P = h_0'(*)$. The transfer function from $x_L$ to $u_I$ is given by

$$
\begin{aligned}
\hat{u}_I(s)/\hat{x}_L(s) &= \begin{bmatrix} 0 & h_2'(*) \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} 0 \\ \theta \end{bmatrix} \\[2ex]
&= \theta h_2'(*) . \frac{s+\eta Z_2^*}{s(s+\eta(Z_1^*+Z_2^*))} \\[2ex]
&\approx \frac{\theta h_2'(*)}{s} \qquad (\eta \gg 1) \\[2ex]
&= \frac{K_I}{s}
\end{aligned}
$$

To compute the transfer function from $x_L$ to $u_D$, we first compute the transfer function from $u_D$ to $z_4$.

$$\hat{z}_4(s)/\hat{u}_D(s) \;=\; \begin{bmatrix} 0 & 1 \end{bmatrix} \begin{bmatrix} \dfrac{s+\eta Z_3^*}{s(s+\eta(Z_3^*+Z_4^*))} & \dfrac{-\eta Z_3^*}{s(s+\eta(Z_3^*+Z_4^*))} \\[2mm] \dfrac{-\eta Z_4^*}{s(s+\eta(Z_3^*+Z_4^*))} & \dfrac{s+\eta Z_4^*}{s(s+\eta(Z_3^*+Z_4^*))} \end{bmatrix} \begin{bmatrix} 0 \\ 1 \end{bmatrix}$$

$$= \; \frac{s+\eta Z_4^*}{s(s+\eta(Z_3^*+Z_4^*))}$$

$$\approx \; \frac{1}{s} \qquad (\eta \gg 1)$$

Combining this with the fact that $\hat{u}_D = \partial_z g(*)\hat{z}_4 + \partial_x g(*)\hat{x}_L$, we immediately get the transfer function from $x_L$ to $u_D$

$$\frac{\hat{u}_D(s)}{\hat{x}_L(s)} = \frac{K_D s}{s+\gamma},$$

where $K_D = \partial_x(*)$ and $y = -\partial_z g(*)$. Note that $\gamma > 0$.

It follows that

$$\frac{\hat{u}}{\hat{x}_L} = K_P + \frac{K_I}{s} + \frac{K_D s}{s+\gamma}$$

*3.3.2 Processes with positive gain*

These processes usually require n-type controllers for stability. We assume the process is described as

$$\begin{aligned} \dot{X}_1 &= f_1(X_1, \ldots, X_L) + U \\ \dot{X}_2 &= f_2(X_1, \ldots, X_L) \\ &\;\;\vdots \\ \dot{X}_L &= f_L(X_1, \ldots, X_L) \end{aligned}$$

Given a desired setpoint $X_L = X_L^*$, we assume there exists a corresponding nonzero fixed point ( $X_1^*$, ..., $X_L^*$, $U^*$).

The n-type PID controller dynamics are as follows (see Fig. 33, left panel):

$$\dot{Z}_1 \;=\; \mu - \eta Z_1 Z_2$$

$$\dot{Z}_2 \;=\; \theta X_L - \eta Z_1 Z_2$$

$$\dot{Z}_3 \;=\; \mu_0 - \eta Z_3 Z_4$$

$$\dot{Z}_4 \;=\; g(Z_4, X_L) - \eta Z_3 Z_4$$

$$U = \underbrace{h_0(X_L)}_{U_P} + \underbrace{h_1(Z_1)}_{U_I} + \underbrace{g(Z_4, X_L)}_{U_D}$$

We will take $h_0$ to be strictly monotonically decreasing, $h_2$ to be strictly monotonically increasing, and $g(Z_4, X_L)$ to be strictly monotonically decreasing in $X_L$ and in $Z_4$. For example

$$g(Z_4, X_L) = \alpha \frac{1}{1 + X_L/\beta_1} \cdot \frac{1}{1 + Z_4/\beta_2}, \text{ or } g(Z_4, X_L) = \frac{\alpha_1}{1 + X_L/\beta_1} + \frac{\alpha_2}{1 + Z_4/\beta_2}, \text{ etc.}$$

**Lemma 1:** Necessary and sufficient conditions for the closed-loop to have a non-negative fixed point ( $Z_1^*$, ... , $Z_4^*$, $X_1^*$, ... , $X_L^*$ ). are

$$g(\infty, \mu/\theta) < \mu_0 < g(0, \mu/\theta)$$

and

$$h_2(0) < [U^* - h_0(\mu/\theta) - \mu_0] < h_2(\infty)$$

**Lemma 2:** $Z_1^*$, $Z_4^*$ are independent of $\eta$ and are both positive. $Z_2^*, Z_3^* \to 0$ as $\eta \to 0$.

Linearizing the dynamics at this fixed point we have:

$$\dot{z}_1 = -\eta Z_2^* z_1 - \eta Z_1^* z_2$$

$$\dot{z}_2 = \theta x_L - \eta Z_2^* z_1 - \eta Z_1^* z_2$$

$$\dot{z}_3 = -\eta Z_4^* z_3 - \eta Z_3^* z_4$$

$$\dot{z}_4 = \partial_z g(*) z_4 + \partial_x g(*) x_L - \eta Z_4^* z_3 - \eta Z_3^* z_4$$

$$\dot{x}_1 = \underbrace{h_0'(*) x_L}_{u_P} + \underbrace{h_1'(*) z_1}_{u_I} + \underbrace{\partial_z g(*) z_4 + \partial_x g(*) x_L}_{u_D} + \nabla^T f_1(*) x$$

where $h_0'(*)$, $h_2'(*)$ are the derivatives of $h_0$, $h_1$ evaluated at the fixed point; $\partial_z g$ and $\partial_x g$ are the partial derivatives of g with respect to $Z_4$ and $X_L$, respectively, evaluated at the fixed point.

We next compute the transfer functions from $x_L$ to $u := u_p + u_I + u_D$. The transfer function from $x_L$ to $u_P$ is given by $\hat{u}_P(s)/\hat{x}_L(s) = -K_P$, where $K_P = -h_0'(*) > 0.$ The transfer function from $x_L$ to $u_I$ is given by

$$\hat{u}_I(s)/\hat{x}_L(s) = \begin{bmatrix} h'_1(*) & 0 \end{bmatrix} \begin{bmatrix} \frac{s+\eta Z_1^*}{s(s+\eta(Z_1^*+Z_2^*))} & \frac{-\eta Z_1^*}{s(s+\eta(Z_1^*+Z_2^*))} \\ \frac{-\eta Z_2^*}{s(s+\eta(Z_1^*+Z_2^*))} & \frac{s+\eta Z_2^*}{s(s+\eta(Z_1^*+Z_2^*))} \end{bmatrix} \begin{bmatrix} 0 \\ \theta \end{bmatrix}$$

$$= \theta h'_1(*) . \frac{-\eta Z_1^*}{s(s+\eta(Z_1^*+Z_2^*))}$$

$$\approx \frac{-\theta h'_1(*)}{s} \qquad (\eta \gg 1)$$

$$= \frac{-K_I}{s}$$

To compute the transfer function from $x_L$ to $u_D$, we first compute the transfer function from $u_D$ to $z_4$.

$$\hat{z}_4(s)/\hat{u}_D(s) = \begin{bmatrix} 0 & 1 \end{bmatrix} \begin{bmatrix} \frac{s+\eta Z_3^*}{s(s+\eta(Z_3^*+Z_4^*))} & \frac{-\eta Z_3^*}{s(s+\eta(Z_3^*+Z_4^*))} \\ \frac{-\eta Z_4^*}{s(s+\eta(Z_3^*+Z_4^*))} & \frac{s+\eta Z_4^*}{s(s+\eta(Z_3^*+Z_4^*))} \end{bmatrix} \begin{bmatrix} 0 \\ 1 \end{bmatrix}$$

$$= \frac{s+\eta Z_4^*}{s(s+\eta(Z_3^*+Z_4^*))}$$

$$\approx \frac{1}{s} \qquad (\eta \gg 1)$$

Combining this with the fact that $\hat{u}_D = \partial_z g(*)\hat{z}_4 + \partial_x g(*)\hat{x}_L$, we immediately get the transfer function from $x_L$ to $d_u$

$$\frac{\hat{u}_D(s)}{\hat{x}_L(s)} = \frac{-K_D s}{s+\gamma},$$

Where $K_D = -\partial_x g(*)$ and $\gamma = -\partial_z g(*)$. Note that $K_D, \gamma > 0$.

It follows that

$$\frac{\hat{u}}{\hat{x}_L} = -\left( K_P + \frac{K_I}{s} + \frac{K_D s}{s+\gamma} \right)$$

.

## Claims

1. An expression system for controlling a network in a cell, wherein the network comprises an actuator molecule and an output molecule, wherein the output molecule is positively or negatively regulated by the actuator molecule, wherein the expression system comprises a recombinant gene encoding a first controller molecule, wherein the first controller molecule positively or negatively regulates the actuator molecule.

2. The expression system according to claim 1, wherein the expression system further comprises a recombinant gene encoding a feedback molecule, wherein the feedback molecule is positively regulated by said output molecule, and wherein

   a. in case the actuator molecule positively regulates the output molecule, the feedback molecule negatively regulates the actuator molecule, and
   b. in case the actuator molecule negatively regulates the output molecule, the feedback molecule positively regulates the actuator molecule.

3. The expression system according to claim 2, wherein

    a. in case the actuator molecule positively regulates the output molecule, the feedback molecule is

        i. a microRNA which negatively regulates production of the actuator molecule, or
        ii. an RNA binding protein which negatively regulates production of the actuator molecule, or

    b. in case the actuator molecule negatively regulates the output molecule, the feedback molecule is an additional mRNA encoding the actuator molecule.

4. The expression system according to any one of the preceding claims, wherein the first controller molecule positively regulates the actuator molecule, and wherein the expression system further comprises a recombinant gene encoding a first anti-controller molecule, wherein the first anti- controller molecule negatively regulates, particularly inactivates, sequesters and/or annihilates, the first controller molecule, and wherein the first controller molecule negatively regulates, particularly inactivates, sequesters and/or annihilates, the first anti-controller molecule, wherein

    a. in case the actuator molecule positively regulates the output molecule, the first anti-controller molecule is positively regulated by the output molecule, and
    b. in case the actuator molecule negatively regulates the output molecule, the first controller molecule is positively regulated by the output molecule.

5. The expression system according to claim 4, wherein

    a. the first controller molecule is a sense mRNA encoding the actuator molecule or a sense mRNA coding for an activator which positively regulates the actuator molecule, and wherein the second controller molecule comprises an anti-sense RNA comprising a sequence which is complementary to a sequence of the sense mRNA, or,
    b. the first controller molecule is an activator protein which positively regulates production of the actuator molecule activating translation of an mRNA encoding the actuator molecule or inhibiting degradation of an mRNA encoding the actuator molecule or inhibiting degradation of the actuator molecule or by negatively regulating an inhibitor of the function of the actuator molecule, and wherein the first anti-controller molecule is an anti-activator protein, wherein the activator protein and the anti-activator protein form a complex, wherein the positive regulation of the actuator molecule by the activator protein is inhibited by formation of the complex.

6. The expression system according to claim 4 or 5, wherein

    a. the actuator molecule positively regulates the output molecule, and wherein the first controller molecule is positively regulated by the output molecule,
    b. the actuator molecule negatively regulates the output molecule, and wherein the first anti-controller molecule is positively regulated by the output molecule,

7. The expression system according to any one of the claims 4 to 6, wherein the expression system further comprises a recombinant gene encoding a second controller molecule,

    a. in case the actuator molecule positively regulates the output molecule,

        i. the second controller molecule is positively or negatively regulated by the output molecule and the second controller molecule negatively regulates the actuator molecule, or
        ii. the second controller molecule is negatively regulated by the output molecule and the second controller molecule positively or negatively regulates the actuator molecule,

    and,
    b. in case the actuator molecule negatively regulates the output molecule,

        i. the second controller molecule is positively or negatively regulated by the output molecule and the second controller molecule positively regulates the actuator molecule, or

ii. the second controller molecule is positively regulated by the output molecule and the second controller molecule positively or negatively regulates the actuator molecule.

8. The expression system according to claim 7, wherein the expression system further comprises a recombinant gene encoding a second anti-controller molecule, wherein the second anti- controller molecule negatively regulates, particularly inactivates, sequesters and/or annihilates, the second controller molecule, wherein the second controller molecule negatively regulates, particularly inactivates, sequesters and/or annihilates, the second anti-controller molecule, and wherein the second controller molecule negatively regulates itself, and wherein

    a. in case the actuator molecule negatively regulates the output molecule, the second controller molecule is positively regulated by the output molecule, and
    b. in case the actuator molecule positively regulates the output molecule, the second controller molecule is negatively regulated by the output molecule.

9. The expression system according to claim 8, wherein

    a. the second controller molecule is a sense mRNA encoding a regulator protein which regulates expression of the actuator molecule, wherein the second anti-controller molecule is an antisense RNA comprising a complementary sequence to a sequence of the sense mRNA encoding the regulator protein, wherein particularly in case the feedback molecule is an additional mRNA encoding the actuator molecule, the regulator protein regulates the expression of the additional mRNA encoding the actuator molecule, or
    b. the second controller molecule is an RNA binding protein binding to an untranslated region of an mRNA encoding the actuator molecule, thereby negatively or positively regulating the actuator molecule, and wherein the second anti-controller molecule is an anti-RNA-binding protein, wherein the RNA binding protein and the anti-RNA-binding protein form a complex, wherein the negative or positive regulation of the actuator molecule by the RNA binding protein is inhibited by formation of the complex.

10. The expression system according to claim 2, wherein

    a. in case the actuator molecule positively regulates the output molecule,

        i. the first controller molecule is positively or negatively regulated by the output molecule and the first controller molecule negatively regulates the actuator molecule, or
        ii. the first controller molecule is negatively regulated by the output molecule and the first controller molecule positively or negatively regulates the actuator molecule,

    and,
    b. in case the actuator molecule negatively regulates the output molecule,

        i. the first controller molecule is positively or negatively regulated by the output molecule and the first controller molecule positively regulates the actuator molecule, or
        ii. the first controller molecule is positively regulated by the output molecule and the first controller molecule positively or negatively regulates the actuator molecule.

11. A cell comprising the expression system according to any one of the claims 1 to 10.

12. The cell according to claim 11, wherein the cell is a mammalian cell, particularly a human cell.

13. The cell according to claim 11 or 12, wherein the cell is a T cell, particularly expressing a chimeric antigen receptor, CAR, particularly wherein a concentration of the output molecule in the cell is indicative of a concentration of at least one inflammatory cytokine in the cell, and wherein the actuator molecule positively regulates production or release of at least one immunosuppressive agent in the cell.

14. The cell according to any one of the claims 11 to 13 for use as a medicament, particularly for use in a method for the treatment of an immunological condition, particularly cytokine release syndrome or rheumatoid arthritis, or for use in a method for the treatment of a metabolic or endocrine condition, particularly diabetes.

15. A method for controlling a network in a cell, wherein the method comprises expressing the at least one recombinant gene of the expression system according to any one of the claims 1 to 10 in the cell.

I implementation (N-type)

Fig. 2

PI implementation (N-type)

Second-order PID implementation (N-type)

Fig. 4

Third-order PID implementation (N-type)

1 — Output-sensing promoter — Anti-sense RNA (z2)

2 — Constitutive promoter — Activator sense mRNA (z1)

3 — Activator-sensing promoter — Actuator mRNA (X1)

4 — Output-sensing promoter — microRNA

5 — Output-sensing promoter — Regulator mRNA (z3)

6 — Reg-sensing promoter — Actuator mRNA (X1)

Act   Activator protein

Reg   Regulator protein (Activator or Repressor)

miR   microRNA

z1   Activator sense mRNA
z2   Anti-sense mRNA
z3   Regulator mRNA

Fig.5

Fourth-order PID implementation (N-type)

1 — Output-sensing promoter — Anti-sense RNA (z2)
2 — Constitutive promoter — Activator sense mRNA (z1)
3 — Activator-sensing promoter — Actuator mRNA (X1)
4 — Output-sensing promoter — microRNA
5 — Output-sensing promoter — Repressor sense mRNA (z5)
6 — Rep-sensing promoter — Actuator mRNA (X1)
7 — Rep-sensing promoter — Repressor sense mRNA (z4)
8 — Constitutive promoter — Anti-sense RNA (z3)

Act  Activator protein
Rep  Repressor protein
miR  microRNA

z1  Activator sense mRNA
z2  Anti-sense mRNA
z3  Anti-sense mRNA
z4  Repressor sense mRNA
z5  Repressor sense mRNA

Fig.6

Fig.7

PI implementation (P-type)

Fig. 8

Second-order PID implementation (P-type)

z1  Anti-sense mRNA
z2  Activator sense mRNA

Act  Activator protein

m1  mRNA
m2  mRNA

1  Output-sensing promoter
   Activator sense mRNA (z2)

2  Constitutive promoter
   Anti-sense RNA (z1)

3  Activator-sensing promoter
   mRNA (m1)

4  Output-sensing promoter
   mRNA (m2)

5  Output-sensing promoter
   Anti-sense RNA (z1)

EP 3 995 577 A1

57

Fig. 9

Fig. 10

Fourth-order PID implementation (P-type)

Output-sensing promoter — Activator sense mRNA (z2) — 1
Constitutive promoter — Anti-sense RNA (z1) — 2
Activator-sensing promoter — mRNA (m1) — 3
Output-sensing promoter — mRNA (m2) — 4
Rep/Output-sensing promoter — Repressor mRNA (z4) — 5
Rep/Output-sensing promoter — mRNA (m2) — 6
Constitutive promoter — Anti-sense RNA (z3) — 7

Actuator (X1)
Output (XL)

Act  Activator protein
Rep  Repressor protein
m1  mRNA
m2  mRNA

z1  Anti-sense mRNA
z2  Activator sense mRNA
z3  Anti-sense mRNA
z4  Repressor sense mRNA

Fig. 11

Fig. 12

Fig. 13

Second-order PID implementation (N-type)

| | Output-sensing promoter |
|1| Anti-activator mRNA (z2) |
|2| Constitutive promoter — Activator mRNA (z1) |
|3| Activator-sensing promoter — Actuator mRNA (m1) |
|4| Output-sensing promoter — RBP mRNA (z3) |
|5| Output-sensing promoter — Activator mRNA (z1) |

z1  Activator mRNA        Z1  Activator protein        RBP  RNA binding protein
z2  Anti-activator mRNA   Z2  Anti-activator protein   m1   Actuator mRNA
z3  RBP mRNA

Fig. 14

Third-order PID implementation (N-type)

| 1 | Output-sensing promoter — Anti-activator mRNA (z2) |
| 2 | Constitutive promoter — Activator mRNA (z1) |
| 3 | Activator-sensing promoter — Actuator mRNA (m1) |
| 4 | Output-sensing promoter — RBP mRNA (z3) |
| 5 | Output-sensing promoter — Regulator mRNA (z4) |

RBP   RNA binding protein
Reg   Regulator protein (Translational repression or activation)
m1    Actuator mRNA

z1   Activator mRNA
z2   Anti-activator mRNA
z3   RBP mRNA
z4   Regulator mRNA

Z1   Activator protein
Z2   Anti-activator protein

Fig. 15

Fourth-order PID implementation (N-type)

Fig. 16

Fig. 17

EP 3 995 577 A1

PI implementation (P-type)

Output-sensing promoter

**1** Activator mRNA (z2)

Constitutive promoter

**2** Anti-activator mRNA (z1)

Activator-sensing promoter

**3** Actuator mRNA (m1)

Output-sensing promoter

**4** Actuator mRNA (m2)

Ø

Ø → m1

**3**

Ø ← m2

Ø

Actuator (X1)

Output (XL)

**4**

**1**

Ø

Ø

z1 → **2**

Ø

Z1

Ø

Z2 (Act)

Ø

z2 ← Ø

Ø

z1 Anti-activator mRNA   Z1 Anti-activator protein   m1 Actuator mRNA

z2 Activator mRNA   Z2 Activator protein   m2 Actuator mRNA

Fig. 18

Fig. 19

Fig. 20

Fig. 21

Fig. 22

**A**

Output
Disturbance

Setpoint

*perfect adaptation*

Setpoint

*no adaptation*

Amplitude

Time

**B**

**Perfect Adaptation Property of the Antithetic Integral Controller**

Control system dynamics

$$\dot{Z}_1 = \mu - \eta Z_1 Z_2$$

$$\dot{Z}_2 = \theta X_L - \eta Z_1 Z_2$$

Integral feedback representation

$$(Z_1 - Z_2)(t) = \int_0^t (\mu - \theta X_L(\tau))$$

Output of interest

$$X_L \to \frac{\mu}{\theta} \quad \text{(steady state)}$$

Fig. 23A

**Antisense plasmid**

Antisense RNA    Promoter $\left\{ \begin{array}{l} \textbf{Closed loop: P}_{TRE} \\ \textbf{Open loop: P}_{Syn1} \end{array} \right.$

**Integral feedback**

antisense RNA $_{3'}\bigwedge\bigwedge_{5'}$ ($z_2$)

**Open loop**

**Disturbance** Asunaprevir (ASV)

sense mRNA $_{5'}\bigwedge\bigwedge_{3'}$ ($z_1$)

X

$\emptyset$

P$_{EF-1\alpha}$

tTA-mCitrine-SMASh

$$\text{Set point} := \frac{\text{Activator plasmid copy \#}}{\text{Antisense plasmid copy \#}}$$

**Activator plasmid**

Fig. 23B

Fig. 23C

= without/with ASV disturbance

Fig. 24A

Fig. 24B

$$\text{Set point} := \frac{\text{Activator plasmid copy \#}}{\text{Antisense plasmid copy \#}}$$

Fig. 25A

Fig. 25B

Fig. 26A

Fig. 26B

Fig. 27

| Species | Symbol | Species | Symbol | Species | Symbol |
|---|---|---|---|---|---|
| Plasmid 1 DNA | $D_1$ | Plasmid 2 DNA | $D_2$ | RNA Polymerase | $P$ |
| mRNA | $Z_1$ | Anti-sense RNA | $Z_2$ | Ribosomes | $R$ |
| $Z_1{:}R$ | $Z_1^*$ | $Z_2{:}R$ | $Z_2^*$ | tTA:mCitrine:SMAShTag | $X_1$ |
| Drug | $G$ | $X_1{:}G$ | $X_1^*$ | tTA:mCitrine | $X_2$ |
| Dimerized tTA:mCitrine | $A$ | $D_1{:}P$ | $D_1^*$ | mRuby3 | $Y$ |
| $D_2{:}A$ | $D_2^\dagger$ | $D_2^\dagger{:}P$ | $D_2^*$ | $D_2{:}P$ | $D_2^b$ |

Fig. 28

EP 3 995 577 A1

| Reaction | Mechanism | Constants |
|---|---|---|
| Transcription 1 | $\mathbf{D_1 + P} \underset{d_1}{\overset{a_1}{\rightleftharpoons}} \mathbf{D_1^*} \overset{k_1}{\longrightarrow} \mathbf{D_1 + P + Z_1}$ | $\kappa_1 := \frac{d_1 + k_1}{a_1}$ |
| Transcription 2 | $\mathbf{D_2 + A} \underset{d_2^\dagger}{\overset{a_2^\dagger}{\rightleftharpoons}} \mathbf{D_2^\dagger} \qquad \mathbf{D_2^\dagger + P} \underset{d_2}{\overset{a_2}{\rightleftharpoons}} \mathbf{D_2^*} \overset{k_2}{\longrightarrow} \mathbf{D_2^\dagger + P + Z_2}$ | $\kappa_2^\dagger := \frac{d_2^\dagger}{a_2^\dagger}, \ \kappa_2 := \frac{d_2 + k_2}{a_2}$ |
| Leaky Transcription 2 | $\mathbf{D_2 + P} \underset{d_2^b}{\overset{a_2^b}{\rightleftharpoons}} \mathbf{D_2^b} \overset{k_2}{\longrightarrow} \mathbf{D_2 + P + Z_2}$ | $\kappa_0 := \frac{d_2^b + k_2}{a_2^b}$ |
| Translation 1 | $\mathbf{Z_1 + R} \underset{d_1'}{\overset{a_1'}{\rightleftharpoons}} \mathbf{Z_1^*} \overset{k_1'}{\longrightarrow} \mathbf{Z_1 + R + X_1}$ | $\kappa_1' := \frac{d_1' + k_1'}{a_1'}$ |
| Translation 2 | $\mathbf{Z_2 + R} \underset{d_2'}{\overset{a_2'}{\rightleftharpoons}} \mathbf{Z_2^*} \overset{k_2'}{\longrightarrow} \mathbf{Z_2 + R + Y}$ | $\kappa_2' := \frac{d_2' + k_2'}{a_2'}$ |
| Degradation | $\mathbf{X_1 + G} \underset{d_3}{\overset{a_3}{\rightleftharpoons}} \mathbf{X_1^*} \overset{k_3}{\longrightarrow} \mathbf{G} \qquad \mathbf{X_2} \overset{\gamma_x}{\longrightarrow} \phi \qquad \mathbf{Y} \overset{\gamma_y}{\longrightarrow} \phi$ | $\kappa_3 := \frac{d_3 + k_3}{a_3}$ |
| Conversion | $\mathbf{X_1} \overset{c}{\longrightarrow} \mathbf{X_2}$ | |
| Dimerization | $\mathbf{X_2 + X_2} \underset{d'}{\overset{a'}{\rightleftharpoons}} \mathbf{A}$ | $\kappa' := \frac{d'}{a'}$ |
| Dilution | $\mathbf{Z_1} \overset{\delta}{\longrightarrow} \phi \qquad \mathbf{Z_2} \overset{\delta}{\longrightarrow} \phi$ | |
| Sequestration | $\mathbf{Z_1 + Z_2} \overset{\eta}{\longrightarrow} \phi$ | |

Fig. 29

Fig. 30

Fig. 31

Fig. 32

Fig. 33

Europäisches Patentamt

European Patent Office

Office européen des brevets

## EUROPEAN SEARCH REPORT

Application Number

EP 20 20 6417

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/027414 A1 (MASSACHUSETTS INST TECHNOLOGY [US]) 7 February 2019 (2019-02-07) * page 13 - page 17; claims 1-20,30-37; figures 2,3,11-22 * * page 52 - page 54 * | 1-15 | INV. C12N15/11 C12N15/63 |
| X | WO 2013/155439 A1 (MASSACHUSETTS INST TECHNOLOGY [US]) 17 October 2013 (2013-10-17) * claims 2-6, 18; figures 1,2 * | 1-15 | |
| X | WO 2012/012739 A2 (HARVARD COLLEGE [US]; MASSACHUSETTS INST TECHNOLOGY [US] ET AL.) 26 January 2012 (2012-01-26) * claims 1-10; figures 2,4 * | 1,11-15 | |
| X | WO 2011/066541 A2 (UNIV BOSTON [US]; MASSACHUSETTS INST TECHNOLOGY [US] ET AL.) 3 June 2011 (2011-06-03) * claims 1-3; figures 1,5 * | 1,11-15 | TECHNICAL FIELDS SEARCHED (IPC) |
| X | EP 3 156 493 A1 (UNIV TSINGHUA [CN]) 19 April 2017 (2017-04-19) * figures 16,20,21 * | 1,11-15 | C12N C40B |
| X | SIMON AUSLÄNDER ET AL: "From gene switches to mammalian designer cells: present and future prospects", TRENDS IN BIOTECHNOLOGY, vol. 31, no. 3, 13 December 2012 (2012-12-13), pages 155-168, XP55117371, ISSN: 0167-7799, DOI: 10.1016/j.tibtech.2012.11.006 * figure 3 * | 1,11-15 | |

-/--

The present search report has been drawn up for all claims

| Place of search | Date of completion of the search | Examiner |
|---|---|---|
| The Hague | 20 April 2021 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 20 6417

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAOJUN WANG ET AL: "Customizing cell signaling using engineered genetic logic circuits", TRENDS IN MICROBIOLOGY, vol. 20, no. 8, 6 June 2012 (2012-06-06), pages 376-384, XP028430531, ISSN: 0966-842X, DOI: 10.1016/J.TIM.2012.05.001 [retrieved on 2012-05-08] * figures 2,3 * | 1,11-15 | |
| X | GABRIELE LILLACCI ET AL: "Synthetic control systems for high performance gene expression in mammalian cells", NUCLEIC ACIDS RESEARCH, vol. 46, no. 18, 7 September 2018 (2018-09-07), pages 9855-9863, XP055753584, ISSN: 0305-1048, DOI: 10.1093/nar/gky795 * figure 1 * | 1,2, 11-15 | |
| X | AOKI STEPHANIE K ET AL: "A universal biomolecular integral feedback controller for robust perfect adaptation", NATURE, MACMILLAN JOURNALS LTD., ETC, LONDON, vol. 570, no. 7762, 19 June 2019 (2019-06-19), pages 533-537, XP036817374, ISSN: 0028-0836, DOI: 10.1038/S41586-019-1321-1 [retrieved on 2019-06-19] * figure 3 * | 1,2, 11-15 | TECHNICAL FIELDS SEARCHED (IPC) |

-/--

The present search report has been drawn up for all claims

| Place of search | Date of completion of the search | Examiner |
|---|---|---|
| The Hague | 20 April 2021 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 20 6417

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WIELAND MARKUS ET AL: "Engineering Molecular Circuits Using Synthetic Biology in Mammalian Cells", ANNUAL REVIEW OF CHEMICAL AND BIOMOLECULAR ENGINEERING, vol. 3, no. 1, 29 March 2012 (2012-03-29), pages 209-234, XP55792389, US ISSN: 1947-5438, DOI: 10.1146/annurev-chembioeng-061010-114145 Retrieved from the Internet: URL:https://www.annualreviews.org/doi/pdf/10.1146/annurev-chembioeng-061010-114145> * figures 2-5 * | 1-15 | |
| A | WO 2016/095934 A2 (EL ABD HISHAM MOHAMED MAGDY [EG]) 23 June 2016 (2016-06-23) * claims 9,10,15; figures 14,18,19,34,35 * | 1-15 | |

TECHNICAL FIELDS
SEARCHED      (IPC)

The present search report has been drawn up for all claims

| Place of search | Date of completion of the search | Examiner |
|---|---|---|
| The Hague | 20 April 2021 | Bucka, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 20 6417

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2021

| Patent document cited in search report | | Publication date | Patent family member(s) | | Publication date |
|---|---|---|---|---|---|
| WO 2019027414 | A1 | 07-02-2019 | NONE | | |
| WO 2013155439 | A1 | 17-10-2013 | US | 2015087055 A1 | 26-03-2015 |
| | | | WO | 2013155439 A1 | 17-10-2013 |
| WO 2012012739 | A2 | 26-01-2012 | AU | 2011280958 A1 | 24-01-2013 |
| | | | CA | 2805915 A1 | 26-01-2012 |
| | | | EP | 2596128 A2 | 29-05-2013 |
| | | | NZ | 605438 A | 27-02-2015 |
| | | | US | 2013202532 A1 | 08-08-2013 |
| | | | WO | 2012012739 A2 | 26-01-2012 |
| WO 2011066541 | A2 | 03-06-2011 | US | 2013034907 A1 | 07-02-2013 |
| | | | WO | 2011066541 A2 | 03-06-2011 |
| EP 3156493 | A1 | 19-04-2017 | EP | 3156493 A1 | 19-04-2017 |
| | | | EP | 3636765 A1 | 15-04-2020 |
| | | | US | 2017145426 A1 | 25-05-2017 |
| | | | US | 2020071711 A1 | 05-03-2020 |
| | | | WO | 2015165275 A1 | 05-11-2015 |
| WO 2016095934 | A2 | 23-06-2016 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MICHAEL E LEE ; WILLIAM C DELOACHE ; BER-NARDO CERVANTES ; JOHN E DUEBER.** A highly characterized yest toolkit for modular, multipart assembly. *ACS synthetic biology,* 2015, vol. 4 (9), 975-986

- **CAROLA ENGLER ; ROMY KANDZIA ; SYLVES-TRE MARILLONNET.** A one pot, one step, precision cloning method with high throughput capability. *PloS one,* 2008, vol. 3 (11